# EUROPEAN PATENT APPLICATION

(11) **EP 4 461 736 A1**
(43) Date of publication of application: **13.11.2024**
(21) Application number: 23737172.9
(22) Date of filing: 06.01.2023
(51) Int. Cl.: C07J 43/00, A61P 5/26, A61K 31/56

(54) **STEROID COMPOUND, AND PREPARATION METHOD THEREFOR AND USE THEREOF**

(30) Priority: 07.01.2022 CN 202210015953
(71) Applicant: Shanghai Institute of Materia Medica, Chinese Academy of Sciences, Pudong, Shanghai 201203 (CN)
(72) Inventor: YANG, Yushe, Shanghai 201203 (CN); WANG, Ao, Shanghai 201203 (CN); LUO, Xianggang, Shanghai 201203 (CN); WANG, Yawan, Shanghai 201203 (CN)
(74) Representative: dompatent von Kreisler Selting Werner - Partnerschaft von Patent- und Rechtsanwälten mbB
(86) International application number: PCT/CN2023/071074
(87) International publication number: WO 2023/131310

(57) **Abstract**

Disclosed in the present invention are a steroid compound, and a preparation method therefor and the use thereof. The structure of the steroid compound is as shown in formula I, and the definition of each substituent in the formula are as described in the description and claims. The steroid compound of the present invention is a steroid compound having the dual functions of excellent AR antagonistic activity and AR degradative activity, has the characteristics of good druggability and safety, and can be used for treating diseases related to an AR signaling pathway.

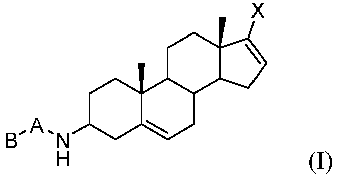

## Description

### Technical Field

The present invention belongs to the field of pharmacology, relating to pharmaceutical synthesis and pharmacology. More specifically, it relates to steroidal compounds with dual mechanisms of androgen receptor degradation and antagonism, preparation methods thereof, and their applications in the treatment of diseases related to androgen receptor (AR) signaling pathways, including prostate cancer, castration-resistant prostate cancer, SARS-CoV-2 infectious diseases, and advanced breast cancer.

### Background

Prostate cancer (PCa) is a common malignant tumor of the male genitourinary system and an androgen-dependent malignancy. Its occurrence, development, metastasis, and deterioration are closely related to androgens and their receptor signaling pathways. Therefore, androgen deprivation therapy (ADT) has become the main treatment for early PCa. However, after 18-24 months of ADT treatment, patients adapt to low levels of androgens in the body and develop into lethal castration-resistant prostate cancer (CRPC). In the past two decades, there has been significant progress in the treatment of CRPC patients. Among them, the CYP17A1 enzyme (androgen synthesis enzyme) inhibitor abiraterone acetate and second-generation AR antagonists enzalutamide, apalutamide, and darolutamide have been approved for first-line treatment of metastatic castration-resistant prostate cancer (mCRPC) and non-metastatic castration-resistant prostate cancer (nmCRPC) patients, achieving good therapeutic effects.

However, after 9-15 months of clinical treatment with abiraterone and second-generation AR antagonists, acquired drug resistance still occurs, which can be divided into AR-related resistance and AR bypass activation resistance. AR-related resistance includes: 1) AR gene amplification and overexpression. About 80% of CRPC patients exhibit higher AR expression levels, allowing tumors to adapt to low levels of androgens and continue to grow and proliferate; 2) Point mutations in the AR ligand-binding domain and expression of splice variants. In CRPC patients who have received enzalutamide and abiraterone treatment, about 12-48% develop AR mutations. Among them, the F876L mutation causes enzalutamide and apalutamide to change from AR antagonist activity to AR agonist activity, promoting tumor growth instead. The L701H and T877A mutations lead to resistance and ineffectiveness of abiraterone. In patients who have received enzalutamide or abiraterone treatment, about 50% express splice variants. AR-V567 and AR-V7 are the two most common splice variants, which partially or completely lack the ligand-binding domain of AR, making all current AR antagonists unable to bind to AR and exert their effects, leading to resistance and inactivation. AR bypass-driven resistance refers to the increased expression of glucocorticoid receptor (GR) in CRPC patients, which can competitively bind to response elements with AR, causing patients to promote the transcription of downstream tumor-related genes without relying on the AR signaling pathway, leading to disease deterioration.

Due to the five-year survival rate of less than 30% for CRPC patients, current treatment options for CRPC patients cannot effectively control the disease. Therefore, there is an urgent clinical need to find new treatment strategies for CRPC patients and drug-resistant CRPC.

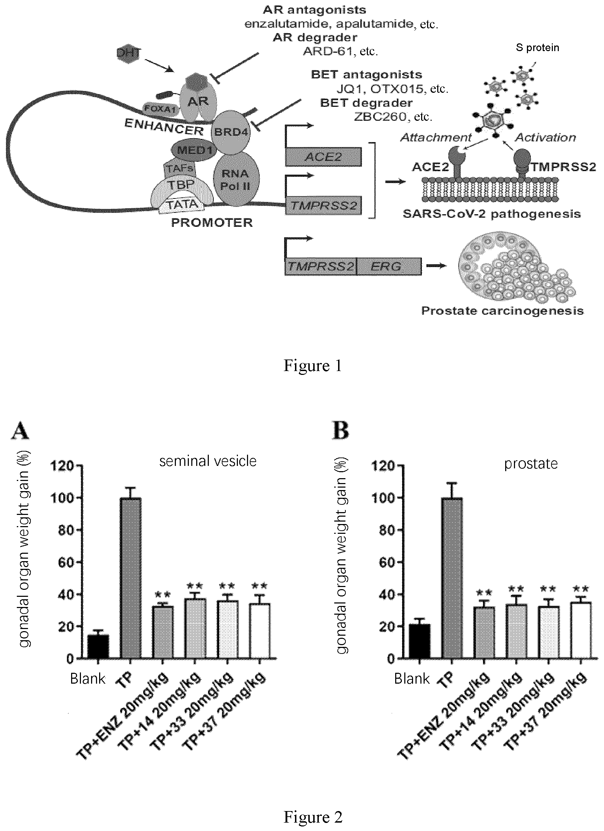

SARS-CoV-2 (COVID-19) invasion (infection) of host cells mainly relies on two proteases, namely transmembrane serine protease 2 (TMPRSS2) and angiotensin-converting enzyme 2 (ACE2). SARS-CoV-2 attaches to the cell membrane through ACE2, and TMPRSS2 completes the proteolytic cleavage of the spike protein at the S1/S2 site of SARS-CoV-2 to initiate the fusion and entry of SARS-CoV-2 and host cells, completing the invasion/infection of host cells. TMPRSS2 and ACE2 proteins are positively regulated by the human androgen receptor signaling pathway. Therefore, AR antagonists or AR degraders can inhibitAR function, downregulate the expression of TMPRSS2 and ACE2 at the transcriptional level, and block SARS-CoV-2 invasion of host cells, thereby blocking COVID-19 infection at the source, as shown in Figure 1.

Clinical studies have also shown that treatment strategies targeting the AR signaling pathway can effectively treat COVID-19 patients and significantly reduce mortality rates. Kintor Pharmaceutical's AR antagonist proxalutamide has completed a phase II proof-of-concept study for the treatment of COVID-19 patients with AR antagonists and achieved surprising clinical anti-COVID-19 effects. Phase III clinical studies are currently being conducted in multiple countries. The drug received emergency use authorization in Paraguay in July 2021.

In summary, the AR signaling pathway plays an important role in various diseases, including prostate cancer and COVID-19 pneumonia. The development of AR antagonists or AR degraders is expected to be better used in the treatment of diseases related to the AR signaling pathway.

### Summary of the Invention

The purpose of the present invention is to provide a class of steroidal compounds with dual functions of excellent AR antagonism and AR degradation activity. Compared with existing antagonists, these compounds can more thoroughly block the AR signaling pathway and can be better used in the treatment of diseases related to the AR signaling pathway.

The first aspect of the present invention provides a compound of general formula (I), or an enantiomer, diastereomer, stereoisomer, prodrug, deuterated compound, hydrate, solvate, racemate or pharmaceutically acceptable salt thereof, 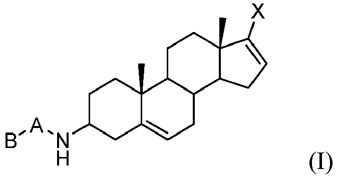
wherein A is 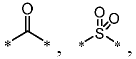 -SO- or C1-C3 alkylene;
B is hydrogen, substituted or unsubstituted C₁₋₆ alkyl, substituted or unsubstituted C₃₋₈ cycloalkyl, substituted or unsubstituted 4-8 membered heterocycloalkyl, substituted or unsubstituted C₆₋₁₀ aryl, substituted or unsubstituted 5-10 membered heteroaryl;
X is substituted or unsubstituted 5-10 membered heteroaryl, or substituted or unsubstituted 4-8 membered heterocycloalkyl;
each said substitution independently refers to substitution by one or more groups selected from the group consisting of deuterium, hydroxyl, carboxyl, amino, mercapto, C₁₋₆ alkyl, C₁₋₆ alkoxy, sulfonyl, halogen, cyano, NO₂, C₁₋₆ haloalkyl, -SO-C₁₋₆ alkyl, -SO₂-C₁₋₆ alkyl, -CONH-C₁₋₆ alkyl, -NHCO-C₁₋₆ alkyl, and C₃₋₆ cycloalkyl;
the heteroatom of said heterocycloalkyl and heteroaryl is selected from: O, N or S, and the number of heteroatoms is 1, 2, 3 or 4.

In another preferred example, the compound of formula I has the following formula:

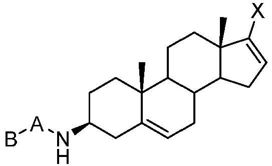

In another preferred embodiment, A is 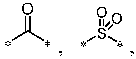 -SO-, -CH₂-, -CH₂CH₂- or -CH₂CH₂CH₂-.

In another preferred embodiment, B is hydrogen, substituted or unsubstituted C₁₋₄ alkyl, substituted or unsubstituted C₃₋₆ cycloalkyl, substituted or unsubstituted 5-6 membered heterocycloalkyl, substituted or unsubstituted phenyl, substituted or unsubstituted 5-7 membered heteroaryl;
wherein the substitution refers to substitution by 1, 2 or 3 groups selected from: deuterium, hydroxyl, carboxyl, amino, mercapto, C₁₋₄ alkyl, C₁₋₄ alkoxy, sulfonyl, F, Cl, Br, CN, NO₂, C₁₋₄ haloalkyl, -SO-C₁₋₄ alkyl, -SO₂-C₁₋₄ alkyl, -CONH-C₁₋₄ alkyl, -NHCO-C₁₋₄ alkyl, C₃₋₆ cycloalkyl;
wherein the heteroatom of the heterocycloalkyl and heteroaryl is selected from: O, N or S, and the number of heteroatoms is 1, 2 or 3.

In another preferred embodiment, B is 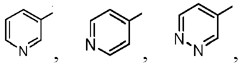 cyclopropyl, morpholinyl 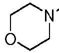 or phenyl, wherein the above groups are optionally substituted by 1, 2 or 3 groups selected from: deuterium, methyl, ethyl, n-propyl, isopropyl, hydroxyl, amino, F, Cl, Br, cyano, NO₂, -SOCH₃, -SOCH₂CH₃, -SOCH(CH₃)₂, - SO₂CH₃, -SO₂CH₂CH₃, -SO₂CH(CH₃)₂.

In another preferred embodiment, X is selected from: substituted or unsubstituted 5-9 membered heteroaryl, substituted or unsubstituted 4-6 membered heterocycloalkyl;
wherein the substitution refers to substitution by 1, 2 or 3 groups selected from: deuterium, hydroxyl, carboxyl, amino, mercapto, C₁₋₄ alkyl, C₁₋₄ alkoxy, sulfonyl, F, Cl, Br, CN, NO₂, C₁₋₄ haloalkyl, -SO-C₁₋₄ alkyl, -SO₂-C₁₋₄ alkyl, -CONH-C₁₋₄ alkyl, -NHCO-C₁₋₄ alkyl, C₃₋₆ cycloalkyl;
wherein the heteroatom of the heterocycloalkyl and heteroaryl is selected from: O, N or S, and the number of heteroatoms is 1, 2, 3 or 4.

In another preferred embodiment, X is a nitrogen-containing heteroaryl selected from: pyridinyl, imidazolyl, benzimidazolyl, triazolyl, tetrazolyl, pyrimidinyl, pyridazinyl; wherein the above groups are optionally substituted by 1, 2 or 3 groups selected from: deuterium, hydroxyl, carboxyl, amino, mercapto, C₁₋₄ alkyl, C₁₋₄ alkoxy, sulfonyl, F, Cl, Br, CN, NO₂, C₁₋₄ haloalkyl, -SO-C₁₋₄ alkyl, -SO₂-C₁₋₄ alkyl, -CONH-C₁₋₄ alkyl, -NHCO-C₁₋₄ alkyl, C₃₋₆ cycloalkyl.

In another preferred embodiment, X is selected from 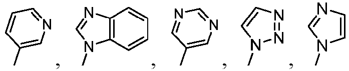 ; wherein the above groups are optionally substituted by 1 or 2 groups selected from: methyl, ethyl, n-propyl, isopropyl, cyclopropyl, n-butyl, isobutyl, tert-butyl, methoxy, ethoxy, trifluoromethyl, difluoromethyl, cyano, nitro, carboxyl, fluoro, chloro, bromo, -CONHCH₃, -NHCOCH₃.

In another preferred embodiment, the pharmaceutically acceptable salt is formed by the compound of formula (I) with an organic acid or inorganic acid, organic base or inorganic base. The pharmaceutically acceptable salts non-limitingly include inorganic acid salts, such as hydrochloride, hydrobromide, nitrate, sulfate, phosphate, etc.; organic acid salts, such as formate, acetate, propionate, maleate, fumarate, succinate, tartrate, citrate, acidic amino acid salts, alkylsulfonates (such as methanesulfonate, ethanesulfonate, etc.), arylsulfonates (such as benzenesulfonate, p-toluenesulfonate, etc.), etc. The pharmaceutically acceptable salts non-limitingly include inorganic base salts, such as sodium salt, potassium salt, magnesium salt, calcium salt; organic base salts, including ethylenediamine salt, glucamine salt, trometamol salt, etc.

The compound of formula (I) of the present invention, optical isomers or pharmaceutically acceptable salts thereof may also exist in the form of solvates, such as hydrates, alcoholates, etc., which are also included in the scope of the present invention.

In another preferred embodiment, the compound or pharmaceutically acceptable salt thereof is selected from the group consisting of 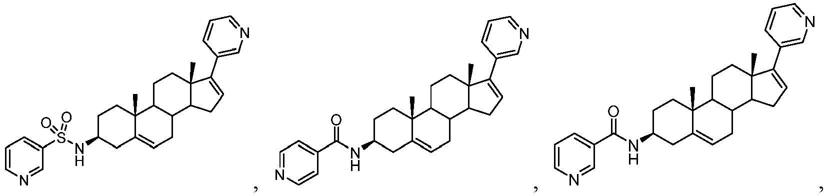 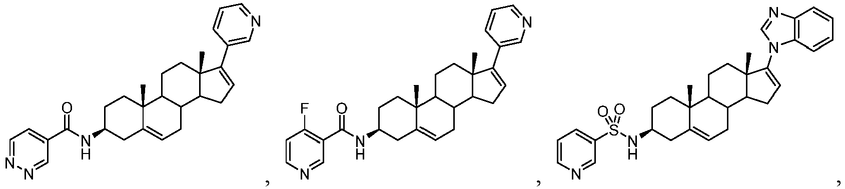 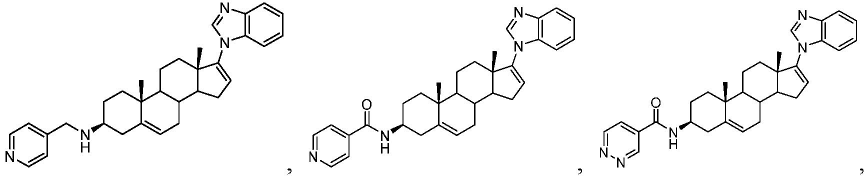 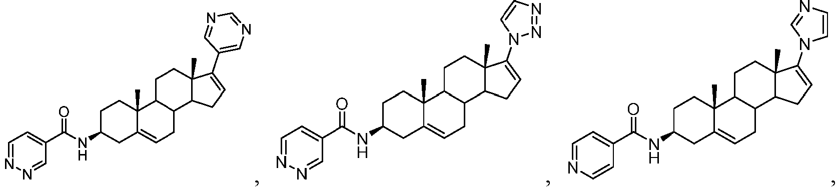 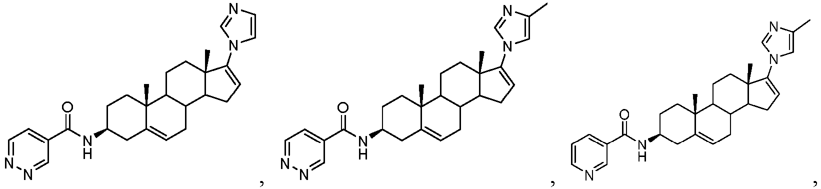 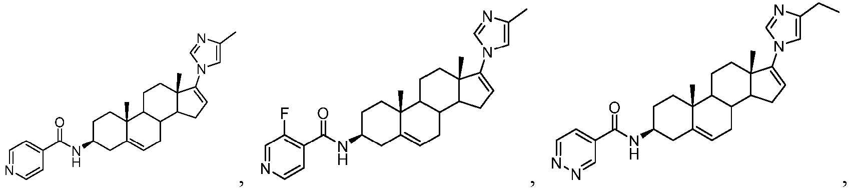 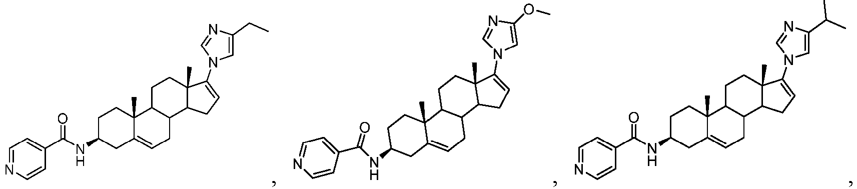 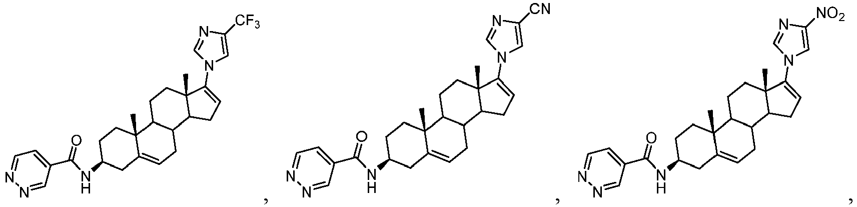 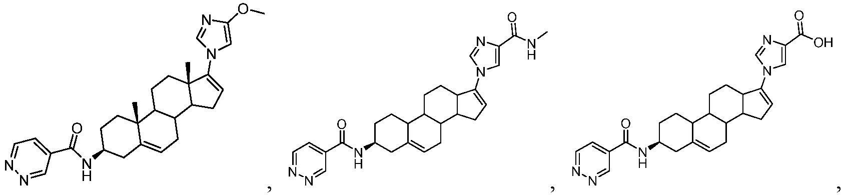 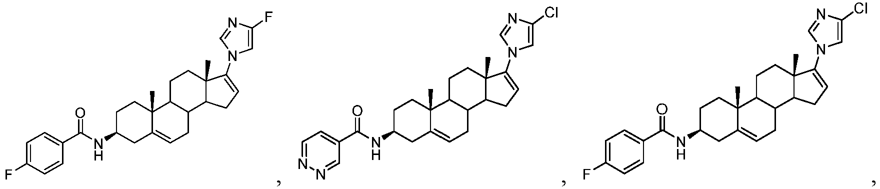 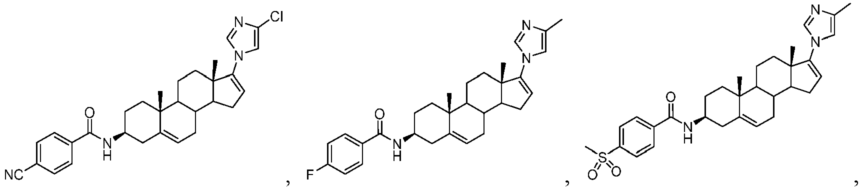 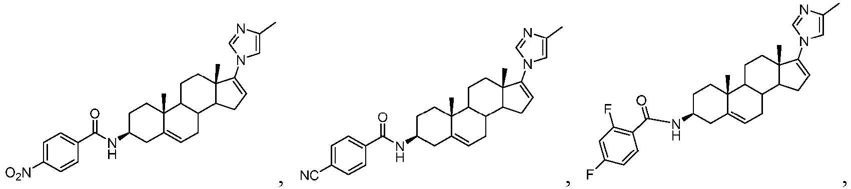 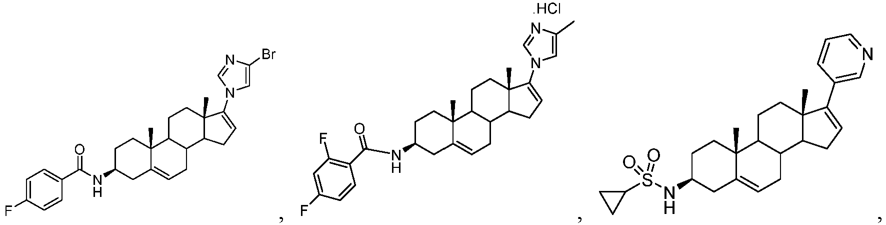 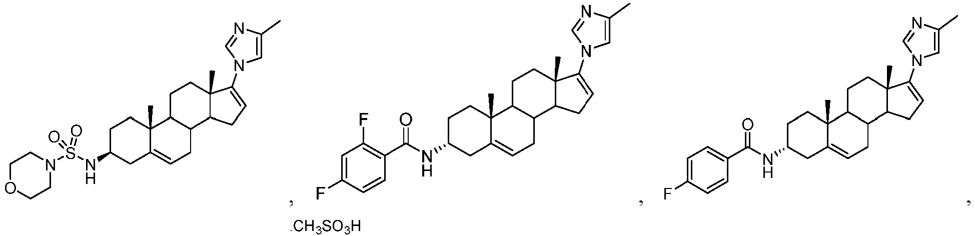 and

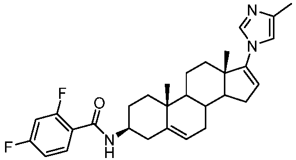

The second aspect of the present invention provides a method for preparing the compound of the first aspect, comprising the following steps: 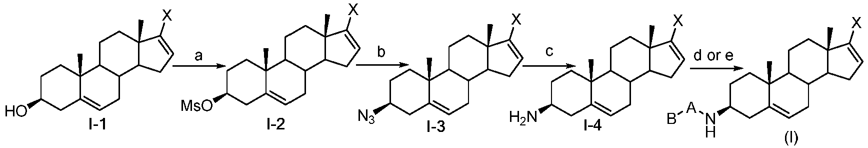
a) reacting Intermediate I-1 with methanesulfonyl chloride to obtain intermediate I-2;
b) reacting Intermediate I-2 with azidotrimethylsilane and boron trifluoride etherate to obtain intermediate I-3;
c) reacting Intermediate I-3 with lithium aluminum hydride to obtain intermediate I-4;
d) reacting Intermediate I-4 with acyl chloride to obtain the target compound I; or
e) subjecting Intermediate I-4 to a condensation reaction with carboxylic acid to obtain the target compound I,
wherein X, A and B are as defined above.

In another preferred embodiment, in step a), intermediate I-1 is dissolved in a polar aprotic solvent in the presence of an organic base, then reacted with methanesulfonyl chloride at room temperature for 2-6 h under the catalysis of 4-dimethylaminopyridine to obtain intermediate I-2. The organic base can be triethylamine, N,N-diisopropylethylamine; the polar aprotic solvent can be: 1,4-dioxane, toluene, tetrahydrofuran, dichloromethane; the optimal solvent is dichloromethane.

In another preferred embodiment, in step b), intermediate I-2 is dissolved in anhydrous dichloromethane solution, followed by addition of 3-6 equivalents of azidotrimethylsilane and 4-8 equivalents of boron trifluoride etherate, and reacted at room temperature for 12-24 h to obtain intermediate I-3 with retained stereochemistry. The optimal equivalent ratio of azidotrimethylsilane is 3 equivalents; the optimal equivalent ratio of boron trifluoride etherate is 4 equivalents.

In another preferred embodiment, in step c), intermediate I-3 is dissolved in a polar aprotic solvent, 1.5 equivalents of lithium aluminum hydride is added and reacted at room temperature for 2-6 h to obtain intermediate I-4. The polar aprotic solvent can be: 1,4-dioxane, toluene, tetrahydrofuran, dichloromethane; the optimal solvent is tetrahydrofuran.

In another preferred embodiment, in step d, intermediate I-4 is dissolved in a polar aprotic solvent in the presence of an organic base, and then reacted with various acid chlorides (isonicoyl chloride hydrochloride, nicotinoyl chloride hydrochloride, pyridine-3-sulfonyl chloride hydrochloride, etc.) at room temperature for 6-12 h under the catalysis of 4-dimethylaminopyridine to obtain the target compound I. The organic base can be triethylamine or N,N-diisopropylethylamine; the polar aprotic solvent can be 1,4-dioxane, toluene, tetrahydrofuran, or dichloromethane; the optimal solvent is dichloromethane.

In another preferred embodiment, in step e, intermediate I-4 is dissolved in N,N-dimethylformamide, and reacted with various carboxylic acids (4-pyrazinecarboxylic acid, 4-fluoropyridine-3-carboxylic acid, etc.) at room temperature for 8-12 h in the presence of the condensing agent 2-(7-azabenzotriazol-1-yl)-N,N,N',N'-tetramethyluronium hexafluorophosphate (HATU) to obtain the target compound I. The reaction temperature is 0-50°C, with the optimal temperature being room temperature.

In another preferred embodiment, compound II-1 undergoes a Suzuki coupling reaction with boronic acid or boronic acid ester to obtain the intermediate I-1, 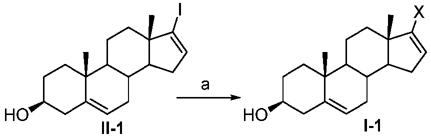 wherein X is as defined above.

In another preferred embodiment, compound II-1 is dissolved in a mixed solution of 1,4-dioxane and water, and then undergoes a Suzuki coupling reaction with various boronic acids or boronic acid esters (5-pyrimidinylboronic acid, 5-methylpyridin-3-ylboronic acid, 5-methoxy-3-pyrimidinylboronic acid pinacol ester, etc.) at 100 °C under the catalysis of bis(triphenylphosphine)palladium(II) dichloride to obtain the corresponding compound 1-1. The volume ratio of 1,4-dioxane to water is 5:1-2:1, with the optimal volume ratio being 3:1; the reaction temperature is 80-110 °C, with the optimal reaction temperature being 100 °C.

In another preferred embodiment, the preparation method of the intermediate I-1 comprises the following steps: 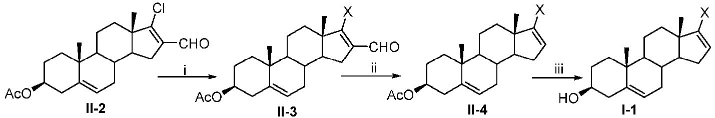
i) Compound II-2 undergoes an addition-elimination reaction to obtain compound II-3;
ii) Compound II-3 undergoes aldehyde removal to obtain compound II-4;
iii) Compound II-4 undergoes hydrolysis to obtain intermediate I-1,
wherein X is as defined above.

In another preferred embodiment, in step i, compound II-2 is dissolved in a polar solvent, and then reacted with various aromatic heterocyclic compounds (1,2,3-triazole, imidazole, 4-methylimidazole, 4-ethylimidazole, etc.) at 80°C in the presence of an inorganic base to undergo an addition-elimination reaction to obtain the corresponding compound II-3. The polar solvent can be acetonitrile, N,N-dimethylformamide, or N,N-dimethylacetamide; the inorganic base can be sodium carbonate, potassium carbonate, or cesium carbonate, with the optimal inorganic base being potassium carbonate.

In another preferred embodiment, in step ii, compound II-3 is dissolved in dry N,N-dimethylformamide, and reacted with a stoichiometric amount of palladium on carbon for 18-72 h to remove the aldehyde group and obtain compound II-4. The reaction temperature can be 140-170 °C, with the optimal reaction temperature being 160 °C.

In another preferred embodiment, in step iii, compound II-4 is dissolved in a polar protic solvent, and then potassium hydroxide is added to hydrolyze the acetate ester at room temperature to obtain the corresponding compound I-1. The polar protic solvent can be methanol or ethanol.

It should be understood that for the compounds of formula (I) described in this invention, those skilled in the art can prepare them using various combinations of methods known in the field of organic synthesis or medicinal chemistry under the guidance of the above methods. The methods described above can be combined with known synthetic methods or variations thereof understood by those skilled in the art to synthesize the compounds of this invention, and are not limited to the above methods.

The third aspect of the present invention provides a pharmaceutical composition comprising:
the compound of formula (I) as described in the first aspect, or the enantiomer, diastereomer, stereoisomer, prodrug, deuterated compound, hydrate, solvate, racemate or pharmaceutically acceptable salt thereof; and
a pharmaceutically acceptable carrier.

The fourth aspect of the present invention provides the use of the compound of formula (I) as described in the first aspect or the pharmaceutical composition as described in the third aspect for preparing a medicament for preventing and/or treating a disease related to the AR signaling pathway.

In another preferred embodiment, the disease related to the AR signaling pathway is selected from: prostate cancer, castration-resistant prostate cancer, breast cancer, SARS-CoV-2 infectious diseases, osteoporosis, and digestive system diseases.

It should be understood that within the scope of this invention, the above technical features of this invention and the technical features specifically described below (such as in the examples) can be combined with each other to form new or preferred technical solutions. The features disclosed in the specification can be replaced by any alternative features that provide the same, equivalent, or similar purpose. Due to space limitations, they will not be repeated here one by one.

### BRIEF DESCRIPTION OF THE DRAWINGS

Figure 1 shows the AR-ACE2/TMPRSS2 signaling pathway mediating SARS-CoV-2 virus infection of host cells.
Figure 2 shows the effect of compounds on the gonadal organ weight gain in vivo, wherein (A) is the weight of seminal vesicle; (B) is the weight of prostate.

### DETAILED DESCRIPTION

After extensive and in-depth research, the inventors designed and synthesized a series of steroid compounds with the general structure of formula (I). Through in vitro tests of AR antagonistic activity, AR degradation activity, mutant AR antagonistic activity and cell proliferation inhibitory activity, as well as systematic biological activity tests such as in vivo rat gonadal weight gain and mouse in vivo xenograft tumor tests, the results showed that the compounds of the present invention have excellent anti-tumor activity and are safer and more effective AR degraders/antagonists. At the same time, the in vivo pharmacokinetic experiments of the compounds of the present invention in rats showed that these compounds have high plasma exposure, exhibiting excellent pharmacokinetic properties and druggability. All these results prove that the compounds of the present invention can be used for the treatment of diseases related to the AR signaling pathway, including prostate cancer, COVID-19 patients, etc. On this basis, the present invention was completed.

### TERM

In the present invention, unless otherwise specified, the terms used have the general meanings well known to those skilled in the art.

In the present invention, the halogen refers to F, Cl, Br or I.

In the present invention, the term "C₁₋₆" means having 1, 2, 3, 4, 5 or 6 carbon atoms, "C₁₋₄" means having 1, 2, 3 or 4 carbon atoms, and so on.

In the present invention, the term "alkyl" refers to a saturated linear or branched hydrocarbon moiety consisting only of carbon atoms and hydrogen atoms. For example, the term "C₁₋₆ alkyl" refers to a straight or branched alkyl group having 1 to 6 carbon atoms, including, but not limited to, methyl, ethyl, n-propyl, isopropyl, n-butyl, isobutyl, sec-butyl, tert-butyl, pentyl, and hexyl; preferably ethyl, n-propyl, isopropyl, n-butyl, isobutyl, sec-butyl, and tert-butyl.

In the present invention, the term "alkylene" refers to a straight or branched saturated aliphatic group having a specified number of carbon atoms and connecting at least two other groups, i.e., a divalent hydrocarbon group. The two groups connected to the alkylene may be connected to the same or different atoms of the alkylene. For example, a straight-chain alkylene may be a divalent group of -(CH₂)n-, where n is 1, 2, 3, 4, 5, or 6. Representative alkylenes include, but are not limited to, methylene, ethylene, propylene, isopropylene, butylene, isobutylene, sec-butylene, pentylene, and hexylene.

In the present invention, the term "alkoxy" refers to an -O-(C₁₋₆ alkyl) group. For example, the term "C₁₋₆ alkoxy" refers to a straight or branched alkoxy group having 1 to 6 carbon atoms, including, but not limited to, methoxy, ethoxy, propoxy, isopropoxy, and butoxy.

In the present invention, the term "cycloalkyl" refers to a stable non-aromatic monocyclic or polycyclic hydrocarbon group consisting only of carbon atoms and hydrogen atoms, which may include fused ring systems, bridged ring systems, or spiro ring systems, typically having 3 to 15 carbon atoms. For example, the term "C₃₋₈ cycloalkyl" refers to a cyclic alkyl group having 3 to 8 carbon atoms in the ring, including, but not limited to, cyclopropyl, cyclobutyl, cyclopentyl, cyclohexyl, cycloheptyl, and cyclooctyl. The term "C₃₋₆ cycloalkyl" has a similar meaning.

In the present invention, the term "heterocycloalkyl" refers to a saturated or unsaturated non-aromatic cyclic group containing at least one (such as 1, 2, 3, or 4) ring heteroatom (e.g., N, O, or S), such as tetrahydropyridinyl, pyrrolinyl, dihydropyridinyl, dihydrofuranyl, dihydrothienyl, and morpholinyl.

In the present invention, the term "4-8 membered" refers to the number of atoms forming the closed ring skeleton itself in the defined closed ring system group (such as aryl, heteroaryl, heterocycloalkyl, etc.) being 4, 5, 6, 7, or 8, which may vary depending on the number of rings, degree of saturation, and nature of the atoms constituting the ring in the closed ring system group. From this, the meanings of other terms described in a similar manner can be inferred, such as "3-6 membered", "4-6 membered", etc.

In the present invention, the term "aryl" refers to a hydrocarbon moiety containing one or more aromatic rings. For example, the term "C₆₋₁₀ aryl" refers to an aromatic ring group having 6 to 10 carbon atoms in the ring without heteroatoms, such as phenyl and naphthyl.

In the present invention, the term "heteroaryl" refers to a conjugated aromatic ring system group composed of carbon atoms and heteroatoms selected from nitrogen, oxygen, and sulfur, which may be monocyclic, bicyclic, tricyclic, or more ring systems, and can be connected to the rest of the molecule through a single bond via an atom on the aromatic ring. Typically, examples of heteroaryl include, but are not limited to, pyrrolyl, furanyl, thienyl, imidazolyl, pyrazolyl, thiazolyl, oxazolyl, oxadiazolyl, isoxazolyl, triazolyl, tetrazolyl, pyridinyl, pyrimidinyl, pyrazinyl, thiadiazinyl, indolyl, isoindolyl, indazolyl, benzimidazolyl, benzotriazolyl, quinolinyl, isoquinolinyl, benzothiazolyl, benzothiadiazinyl, quinazolinyl, and quinoxalinyl.

Unless otherwise specified, the alkyl, cycloalkyl, heterocycloalkyl, aryl, and heteroaryl groups mentioned herein are substituted or unsubstituted groups. Possible substituents include, but are not limited to: hydroxyl, amino, nitro, cyano, halogen, C1-C6 alkyl, C2-C10 alkenyl, C2-C10 alkynyl, C3-C20 cycloalkyl, C3-C20 cycloalkenyl, C1-C20 heterocycloalkyl, C1-C20 heterocycloalkenyl, C1-C6 alkoxy, aryl, heteroaryl, heteroaryloxy, C1-C10 alkylamino, C1-C20 dialkylamino, arylamino, diarylamino, C1-C10 alkylaminosulfonyl, arylaminosulfonyl, C1-C10 alkylimino, C1-C10 alkylsulfonylimino, arylsulfonylimino, mercapto, C1-C10 alkylthio, C1-C10 alkylsulfonyl, arylsulfonyl, acylamino, aminocarbonyl, aminothiocarbonyl, guanidino, ureido, cyano, acyl, thioacyl, acyloxy, carboxyl, and carboxylic ester groups. On the other hand, cycloalkyl, heterocycloalkyl, heterocycloalkenyl, aryl, and heteroaryl groups may also be fused with each other.

In the present invention, the said substitution may be mono-substitution or multi-substitution, where multi-substitution refers to di-substitution, tri-substitution, tetra-substitution, or penta-substitution. Di-substitution means having two substituents, and so on.

### Compounds

The present invention provides a class of steroidal compounds with dual mechanisms of AR degradation and antagonism, which can promote AR degradation while antagonizing AR, achieving complete blockade of the androgen receptor signaling pathway. These compounds have better efficacy than existing single-function AR antagonists, single-function AR degraders, or single-function androgen synthesis enzyme inhibitors for the treatment of CRPC and even prostate cancer patients at various stages, addressing the increasingly serious drug resistance problem in PCa patients. They can also be used for the treatment of diseases such as COVID-19 pneumonia, achieving better treatment for AR signaling pathway-related diseases.

The structure of the compound of the present invention is as follows: 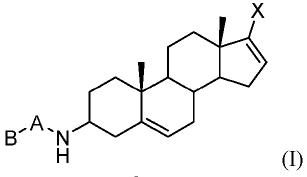 wherein the definitions of the substituents are the same as described above.

### Pharmaceutical Composition

The present invention also provides a pharmaceutical composition comprising a safe and effective amount of an active ingredient and a pharmaceutically acceptable carrier.

The "active ingredient" referred to in the present invention means the compound of formula (I) described in the present invention.

The "active ingredient" and pharmaceutical composition of the present invention are used for preparing a medicament for treating AR signaling pathway-related diseases. The "active ingredient" and pharmaceutical composition of the present invention can be used as AR degraders or antagonists. The AR signaling pathway-related diseases are selected from: prostate cancer, SARS-CoV-2 infectious diseases, castration-resistant prostate cancer, breast cancer, osteoporosis, and digestive system diseases.

"Safe and effective amount" refers to: an amount of the active ingredient sufficient to significantly improve the condition without causing serious side effects.

"Pharmaceutically acceptable carrier" refers to one or more compatible solid or liquid fillers or gel substances that are suitable for human use and must have sufficient purity and sufficiently low toxicity. "Compatible" herein means that the components in the composition can be mixed with the active ingredient of the present invention and with each other without significantly reducing the efficacy of the active ingredient. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerin, mannitol, sorbitol, etc.), emulsifiers (such as Tween^{®}), wetting agents (such as sodium dodecyl sulfate), colorants, flavoring agents, stabilizers, antioxidants, preservatives, pyrogen-free water, etc.

There are no particular limitations on the administration methods for the active ingredient or pharmaceutical composition of the present invention. Representative administration methods include, but are not limited to: oral, intratumoral, rectal, parenteral (intravenous, intramuscular or subcutaneous), etc.

The compounds of the present invention can be administered alone or in combination with other therapeutic drugs (such as antitumor drugs).

When using the pharmaceutical composition, a safe and effective amount of the compound of the present invention is administered to a mammal (such as a human) in need of treatment, wherein the dose administered is an effective dose considered pharmaceutically. Of course, the specific dose should also take into account factors such as the route of administration and the patient's health status, which are within the skill of a skilled physician.

Unless otherwise defined, all technical and scientific terms used herein have the same meaning as commonly understood by one of ordinary skill in the art. In addition, any methods and materials similar or equivalent to those described herein can be used in the practice of the present invention. The preferred embodiments and materials described herein are only for illustrative purposes.

It should be understood that within the scope of the present invention, the above-mentioned technical features and the technical features specifically described below (such as in the examples) can be combined with each other to form new or preferred technical solutions. The features disclosed in the specification can be replaced by any alternative features that provide the same, equivalent or similar purpose. These improvements to the present invention using techniques known to those skilled in the art, such as prodrugs or precursor drugs (hydroxyl, carboxyl, NH, etc. in the structure of the compounds of the present invention can be prepared into prodrugs by conventional methods), deuteration, salt formation, solvates, etc., made without inventive effort are all within the scope of patent protection of the present invention.

The following specific examples are intended to enable those skilled in the art to more clearly understand and implement the present invention. They should not be considered as limiting the scope of the invention, but only as illustrative and typical representations of the invention. Those skilled in the art should understand that there are other synthetic routes for forming the compounds of the present invention, and the following provides non-limiting examples.

All reactions were monitored by thin layer chromatography (TLC) on silica gel F-254 TLC plates. Column chromatography was performed using silica gel (200-300 mesh). The structures of the compounds were determined by nuclear magnetic resonance (NMR). The determination solvent was deuterated dimethyl sulfoxide (DMSO-d₆) or deuterated chloroform (CDCl₃). ¹H and ¹³C NMR were determined using Bruker 400, Bruker 500 or Bruker 600 NMR spectrometers. Chemical shifts (δ) are expressed in parts per million (ppm), and coupling constants (J) are expressed in Hertz (Hz). Mass spectra were obtained using a Finnigan MAT95 mass spectrometer for electron ionization (EI) mass spectra, a Krats MS 80 mass spectrometer for electrospray ionization (ESI) mass spectra, and a 1290-6545 UHPLC-QTOF high-resolution mass spectrometer for high-resolution mass spectra. All final compounds were purified to > 95% purity by analytical high-performance liquid chromatography (PLATISIL ODS 250 mm×4.6 mm, particle size 5 µm) using acetonitrile/water, acetonitrile/buffer (0.1% trifluoroacetic acid/water) or methanol/buffer (0.1% trifluoroacetic acid/water) as the mobile phase.

### Example 1:

### N-((3S,10R,13S)-10,13-dimethyl-17-(pyridin-3-yl)-2,3,4,7,8,9,10,11,12,13,14,15-dodecahydro-1H-cyclopenta[a]phenanthren-3-yl)pyridine-3-sulfonamide (Compound 1)

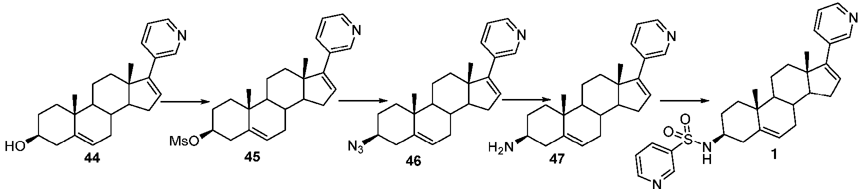

### (3S,10R,13S)-10,13-dimethyl-17-(pyridin-3-yl)-2,3,4,7,8,9,10,11,12,13,14,15-dodecahydro-1H-cyclopenta[a]phenanthren-3-yl methanesulfonate (45)

Abiraterone (44) (1 g, 2.86 mmol) and 4-dimethylaminopyridine (35 mg, 0.286 mmol) were placed in a flask, anhydrous dichloromethane was added as solvent and stirred in an ice bath, then triethylamine (1.19 mL, 8.58 mmol) was added and stirred for a period of time, finally methanesulfonyl chloride (0.5 mL, 5.72 mmol) was slowly added. After the addition was complete, the mixture was stirred at room temperature for 5 h, and the reaction was monitored by TLC until completion. Ice water was then added for quenching, followed by extraction with ethyl acetate. The organic phase was washed twice with saturated brine, dried over anhydrous sodium sulfate, filtered, and silica gel was added to the organic phase, concentrated and purified by automated column chromatography (20-50% ethyl acetate/petroleum ether) to give the desired product. Finally, 705 mg of light yellow solid 45 was obtained in 57.6% yield. ¹H NMR (400 MHz, CDCl₃) δ 8.63 (s, 1H), 8.48 (d, J = 4.7 Hz, 1H), 7.66 (d, J = 7.7 Hz, 1H), 7.24 (dd, J = 7.6, 4.9 Hz, 1H), 6.01 (s, 1H), 5.49 (d, J = 4.5 Hz, 1H), 4.56 (dq, J = 16.2, 5.3 Hz, 1H), 3.04 (s, 3H), 1.10 (s, 3H), 1.06 (s, 3H).

### 3-((3S,10R,13S)-3-azido-10,13-dimethyl-2,3,4,7,8,9,10,11,12,13,14,15-dodecahydro-1H-cyclopenta[a]phenanthren-17-yl)pyridine (46)

Compound 45 (470 mg, 1.1 mmol) was dissolved in anhydrous dichloromethane and stirred at room temperature. Trimethylsilyl azide (0.22 mL, 1.65 mmol) and boron trifluoride etherate (0.27 mL, 2.2 mmol) were added sequentially via plastic syringe. The mixture was stirred at room temperature for 16 h and monitored by TLC until completion. Saturated sodium bicarbonate solution was added to quench the reaction. The mixture was extracted with dichloromethane, and the organic layer was washed twice with brine, dried over anhydrous sodium sulfate, filtered, and concentrated. The residue was purified by automated flash chromatography (5-10% ethyl acetate/petroleum ether) to afford the desired product. Compound 46 was obtained as a white solid (120 mg, 29.1% yield). ¹H NMR (400 MHz, CDCl₃) δ 8.64 (d, J = 1.8 Hz, 1H), 8.48 (dd, J = 4.8, 1.4 Hz, 1H), 7.67 (dt, J = 7.9, 1.9 Hz, 1H), 7.24 (dd, J = 7.9, 4.8 Hz, 1H), 6.02 (dd, J = 3.1, 1.7 Hz, 1H), 5.45 (d, J = 5.1 Hz, 1H), 3.24 (ddd, J = 10.5, 9.2, 4.1 Hz, 1H), 1.09 (s, 3H), 1.07 (s, 3H).

### (3S,10R,13S)-10,13-dimethyl-17-(pyridin-3-yl)-2,3,4,7,8,9,10,11,12,13,14,15-dodecahydro-1H-cyclopenta[a]phenanthren-3-amine (47)

Compound 46 (253 mg, 0.68 mmol) was dissolved in anhydrous tetrahydrofuran and stirred in an ice bath. A solution of lithium aluminum hydride in tetrahydrofuran (1 M, 1 mL, 1 mmol) was slowly added via plastic syringe. The mixture was stirred at room temperature for 1.5 h and monitored by TLC until completion. The reaction was quenched with ice water in an ice bath. The mixture was extracted with ethyl acetate, and the organic layer was washed twice with brine, dried over anhydrous sodium sulfate, filtered, and concentrated. The residue was purified by automated flash chromatography (5-10% methanol/dichloromethane) to afford the desired product. Compound 47 was obtained as a white solid (113 mg, 48.1% yield). ¹H NMR (400 MHz, CDCl₃) δ 8.63 (d, J = 1.7 Hz, 1H), 8.47 (dd, J = 4.7, 1.4 Hz, 1H), 7.66 (d, J = 7.9 Hz, 1H), 7.24 (dd, J = 7.8, 4.8 Hz, 1H), 6.01 (s, 1H), 5.39 (d, J = 5.1 Hz, 1H), 1.07 (s, 6H).

### N-((3S,10R,13S)-10,13-dimethyl-17-(pyridin-3-yl)-2,3,4,7,8,9,10,11,12,13,14,15-dodecahydro-1H-cyclopenta[a]phenanthren-3-yl)pyridine-3-sulfonamide (Compound 1)

Compound 47 (50 mg, 0.143 mmol) and 4-dimethylaminopyridine (2 mg, 0.015 mmol) were placed in a flask. Anhydrous dichloromethane was added as the solvent, and the mixture was stirred in an ice bath. Triethylamine (60 µL, 0.42 mmol) was added and stirred for a period of time. Pyridine-3-sulfonyl chloride hydrochloride (40 mg, 0.17 mmol) was then added, and the mixture was stirred at room temperature for 4 h. The reaction was monitored by TLC until completion. The mixture was extracted with dichloromethane and water. The organic layer was collected, washed twice with brine, dried over anhydrous sodium sulfate, filtered, and concentrated. The residue was purified by automated flash chromatography (30-40% ethyl acetate/petroleum ether) to afford the desired product. Compound 1 was obtained as a white solid (45 mg, 64.1% yield). ¹H NMR (400 MHz, DMSO) δ 8.97 (d, J = 2.1 Hz, 1H), 8.82 (dd, J = 4.8, 1.5 Hz, 1H), 8.58 (s, 1H), 8.43 (d, J = 3.5 Hz, 1H), 8.23 - 8.17 (m, 1H), 8.02 (d, J = 7.4 Hz, 1H), 7.78 - 7.71 (m, 1H), 7.65 (dd, J = 8.0, 4.8 Hz, 1H), 7.33 (dd, J = 7.9, 4.8 Hz, 1H), 6.11 (s, 1H), 5.21 (d, J = 4.8 Hz, 1H), 2.90 (dd, J = 11.6, 4.7 Hz, 1H), 0.99 (s, 3H), 0.95 (s, 3H). 13C NMR (126 MHz, DMSO) δ 153.36, 148.26, 147.61, 147.27, 140.99, 138.94, 134.70, 133.75, 132.57, 129.44, 124.76, 123.82, 121.51, 57.43, 53.99, 50.04, 47.07, 37.64, 36.55, 34.97, 31.72, 31.26, 30.27, 29.51, 20.73, 19.27, 16.68. HRMS (ESI) (M+H)+ m/z calcd for C29H36N3O₂S 490.2523, found: 490.2513.

### Example 2:

### N-((3S,10R,13S)-10,13-dimethyl-17-(pyridin-3-yl)-2,3,4,7,8,9,10,11,12,13,14,15-dodecahydro-1H-cyclopenta[a]phenanthren-3-yl)isonicotinamide (Compound 2)

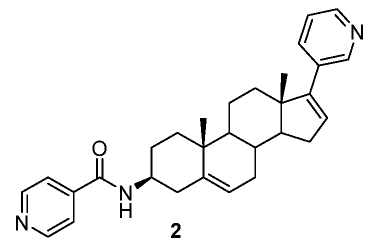

Compound 47 (50 mg, 0.143 mmol) and 4-dimethylaminopyridine (2 mg, 0.015 mmol) were added to a flask. Anhydrous dichloromethane was added as solvent and the mixture was stirred in an ice bath. Triethylamine (60 µL, 0.42 mmol) was then added and stirred for a period of time. Finally, isonicotinoyl chloride hydrochloride (30 mg, 0.17 mmol) was added and the mixture was stirred at room temperature for 6 h. The reaction was monitored by TLC until completion. After the reaction was complete, silica gel was added directly to the system, concentrated, and purified by automated column chromatography (1-3% methanol/dichloromethane) to obtain the desired product. Finally, 34 mg of white solid compound 2 was obtained in 52.3% yield. ¹H NMR (600 MHz, CDCl₃) δ 8.69 (d, J = 5.8 Hz, 2H), 8.59 (s, 1H), 8.43 (d, J = 4.3 Hz, 1H), 7.65 (d, J = 7.9 Hz, 1H), 7.59 (d, J = 5.8 Hz, 2H), 7.22 (dd, J = 7.8, 4.9 Hz, 1H), 6.28 (d, J = 7.9 Hz, 1H), 5.99 (s, 1H), 5.43 (d, J = 5.1 Hz, 1H), 3.94 - 3.86 (m, 1H), 1.05 (s, 3H), 1.02 (s, 3H). 13C NMR (151 MHz, CDCl₃) δ 164.77, 151.48, 150.52, 147.43, 141.95, 140.29, 134.23, 133.07, 129.49, 123.21, 122.05, 120.90, 57.45, 50.44, 50.29, 47.32, 39.14, 37.75, 36.78, 35.09, 31.81, 31.47, 30.40, 29.01, 20.77, 19.30, 16.58. HRMS (ESI) (M-H)- m/z calculated for C30H34N3O 452.2707, found: 452.2703.

### Example 3:

### N-((3S,10R,13S)-10,13-dimethyl-17-(pyridin-3-yl)-2,3,4,7,8,9,10,11,12,13,14,15-dodecahydro-1H-cyclopenta[a]phenanthren-3-yl)nicotinamide (Compound 3)

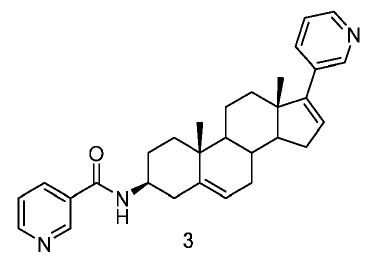

Compound 47 (50 mg, 0.143 mmol) and 4-dimethylaminopyndine (2 mg, 0.015 mmol) were added to a flask. Anhydrous dichloromethane was added as solvent and the mixture was stirred in an ice bath. Triethylamine (60 µL, 0.42 mmol) was then added and stirred for a period of time. Finally, nicotinoyl chloride hydrochloride (30 mg, 0.17 mmol) was added and the mixture was stirred at room temperature for 3 h. The reaction was monitored by TLC until completion. After the reaction was complete, silica gel was added directly to the system, concentrated, and purified by automated column chromatography (1-3% methanol/dichloromethane) to obtain the desired product. Finally, 44 mg of white solid compound 3 was obtained in 67.7% yield. ¹H NMR (500 MHz, CDCl₃) δ 8.98 (d, J = 1.6 Hz, 1H), 8.72 (dd, J = 4.8, 1.6 Hz, 1H), 8.64 (d, J = 1.7 Hz, 1H), 8.47 (dd, J = 4.8, 1.4 Hz, 1H), 8.14 - 8.10 (m, 1H), 7.67 (dt, J = 7.9, 1.8 Hz, 1H), 7.39 (dd, J = 7.8, 4.9 Hz, 1H), 7.24 (dd, J = 7.9, 4.8 Hz, 1H), 6.24 (d, J = 7.8 Hz, 1H), 6.02 (dd, J = 3.1, 1.7 Hz, 1H), 5.48 - 5.45 (m, 1H), 3.95 (tdd, J = 12.1, 8.1, 4.3 Hz, 1H), 1.11 (s, 3H), 1.07 (s, 3H). 13C NMR (126 MHz, CDCl₃) δ 151.60, 151.12, 147.30, 147.27, 147.22, 139.85, 134.61, 133.35, 128.85, 122.97, 122.59, 121.47, 56.98, 49.87, 49.82, 46.84, 38.76, 37.29, 36.30, 34.72, 31.30, 30.99, 29.92, 28.61, 20.28, 18.81, 16.02. HRMS (ESI) (M+H)+m/z calculated for C30H36N3O 454.2853, found: 454.2849.

### Example 4:

### N-((3S,10R,13S)-10,13-dimethyl-17-(pyridin-3-yl)-2,3,4,7,8,9,10,11,12,13,14,15-dodecahydro-1H-cyclopenta[a]phenanthren-3-yl)pyrazine-4-carboxamide (Compound 4)

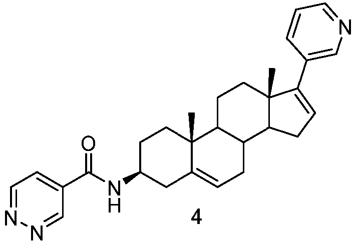

Compound 47 (50 mg, 0.143 mmol) and pyrazine-4-carboxylic acid (20 mg, 0.161 mmol) were added to a flask. Anhydrous N,N-dimethylformamide was added as solvent and the mixture was stirred at room temperature. Then, 2-(7-azabenzotriazol-1-yl)-N,N,N',N'-tetramethyluronium hexafluorophosphate (74 mg, 0.195 mmol) was added and stirred for a period of time. Finally, N,N-diisopropylethylamine (43 µL, 0.29 mmol) was added and the mixture was stirred at room temperature for 12 h. The reaction was monitored by TLC until completion. After the reaction was complete, the mixture was extracted with ethyl acetate and water. The organic phase was collected, washed twice with saturated brine, dried over anhydrous sodium sulfate, filtered, and then silica gel was added to the system, concentrated, and purified by automated column chromatography (30-60% ethyl acetate/petroleum ether) to obtain the desired product. Finally, 29 mg of light yellow solid compound 4 was obtained in 44.6% yield. ¹H NMR (600 MHz, DMSO) δ 9.50 (dd, J = 2.1, 1.3 Hz, 1H), 9.39 (dd, J = 5.3, 1.1 Hz, 1H), 8.82 (d, J = 7.9 Hz, 1H), 8.77 (d, J = 1.6 Hz, 1H), 8.63 (d, J = 4.4 Hz, 1H), 8.24 (d, J = 8.2 Hz, 1H), 7.96 (dd, J = 5.3, 2.3 Hz, 1H), 7.72 (dd, J = 8.0, 5.3 Hz, 1H), 6.33 (d, J = 1.0 Hz, 1H), 5.36 (d, J = 5.0 Hz, 1H), 3.73 - 3.65 (m, 1H), 1.03 (s, 3H), 1.01 (s, 3H). 13C NMR (151 MHz, DMSO) δ 162.66, 152.40, 149.28, 142.67, 141.30, 139.28, 132.89, 132.01, 125.99, 124.67, 121.33, 117.45, 57.47, 50.30, 50.05, 47.07, 38.54, 37.88, 36.86, 34.55, 32.02, 31.26, 30.26, 28.23, 20.83, 19.46, 16.53. HRMS (ESI) (M-H)- m/z calculated for C29H33N4O 453.266, found: 453.2654.

### Example 5:

### N-((3S,10R,13S)-10,13-dimethyl-17-(pyridin-3-yl)-2,3,4,7,8,9,10,11,12,13,14,15-dodecahydro-1H-cyclopenta[a]phenanthren-3-yl)-4-fluoronicotinamide (Compound 5)

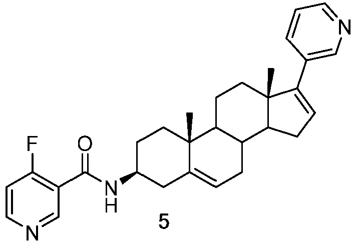

To a flask containing compound 47 (50 mg, 0.143 mmol) and 4-fluoropyridine-3-carboxylic acid (22 mg, 0.143 mmol), anhydrous N,N-dimethylformamide was added as solvent and the mixture was stirred at room temperature. Then 2-(7-azabenzotriazol-1-yl)-N,N,N',N'-tetramethyluronium hexafluorophosphate (74 mg, 0.195 mmol) was added and stirred for a period of time. Finally, N,N-diisopropylethylamine (43 µL, 0.29 mmol) was added and the mixture was stirred at room temperature for 10 h, with the reaction monitored by TLC until completion. After the reaction was complete, the mixture was extracted with ethyl acetate and water. The organic phase was collected, washed twice with saturated brine, dried over anhydrous sodium sulfate, filtered, concentrated with silica gel added, and purified by automated column chromatography (1-2% methanol/dichloromethane) to obtain the desired product. Finally, 36 mg of white solid compound 5 was obtained with a yield of 53.2%. ¹H NMR (600 MHz, CDCl₃) δ 8.88 (s, 1H), 8.69 (d, J = 4.8 Hz, 1H), 8.61 (d, J = 2.0 Hz, 1H), 8.59 (d, J = 4.8 Hz, 1H), 8.28 (d, J = 8.1 Hz, 1H), 7.99 - 7.95 (m, 1H), 7.78 (dd, J = 7.8, 5.6 Hz, 1H), 6.65 - 6.59 (m, 1H), 6.33 (s, 1H), 5.48 (d, J = 4.8 Hz, 1H), 3.96 (d, J = 5.8 Hz, 1H), 1.12 (s, 6H). 13C NMR (151 MHz, CDCl₃) δ 160.23, 148.55, 146.44, 140.98, 140.24, 139.80, 139.02, 136.70, 134.88, 126.03, 124.91, 121.83, 57.41, 50.61, 50.09, 47.44, 39.00, 37.68, 36.78, 34.89, 32.18, 31.32, 30.25, 28.87, 20.67, 19.31, 16.61. HRMS (ESI) (M+H)+ m/z calculated for C30H35FN3O 472.2759, found: 472.2763.

### Example 6:

### N-((3S,10R,13S)-17-(1H-benzo [d]imidazol-1-yl)-10,13-dimethyl-2,3,4,7,8,9,10,11,12,13,14,15-dodecahydro-1H-cyclopenta[a]phenanthren-3-yl)pyridine-3-sulfonamide (Compound 6)

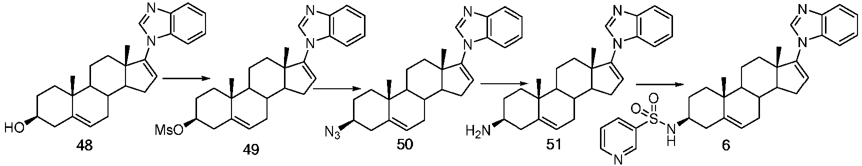

### (3S,10R,13S)-10,13-dimethyl-17-(1H-benzo[d]imidazol-1-yl)-2,3,4,7,8,9,10,11,12,13,14,15-dodecahydro-1H-cyclopenta[a]phenanthren-3-yl methanesulfonate (49)

To a flask containing Galeterone (48) (1.645 g, 4.24 mmol) and 4-dimethylaminopyridine (52 mg, 0.424 mmol), anhydrous dichloromethane was added as solvent and the mixture was stirred in an ice bath. Then triethylamine (1.8 mL, 12.72 mmol) was added and stirred for a period of time. Finally, methanesulfonyl chloride (0.7 mL, 8.4 mmol) was slowly added. After the addition was complete, the mixture was stirred at room temperature for 8 h, with the reaction monitored by TLC until completion. Ice water was then added to quench the reaction, followed by extraction with ethyl acetate. The organic phase was washed twice with saturated brine, dried over anhydrous sodium sulfate, filtered, concentrated with silica gel added, and purified by automated column chromatography (30-50% ethyl acetate/petroleum ether) to obtain the desired product. Finally, 1.64 g of light yellow solid compound 49 was obtained with a yield of 83.2%. ¹H NMR (400 MHz, CDCl₃) δ 7.97 (s, 1H), 7.86 - 7.82 (m, 1H), 7.52 - 7.49 (m, 1H), 7.35 - 7.30 (m, 2H), 6.00 (dd, J = 3.1, 1.7 Hz, 1H), 5.50 (d, J = 5.2 Hz, 1H), 4.57 (tdd, J = 11.4, 7.1, 4.7 Hz, 1H), 3.04 (s, 3H), 1.09 (s, 3H), 1.03 (s, 3H).

### 3-((3S,10R,13S)-3-azido-10,13-dimethyl-2,3,4,7,8,9,10,11,12,13,14,15-dodecahydro-1H-cyclopenta[a]phenanthren-17-yl)-1H-benzo[d]imidazole (50)

Compound 49 (979 mg, 2.1 mmol) was dissolved in anhydrous dichloromethane and stirred at room temperature. Then azidotrimethylsilane (0.41 mL, 3.15 mmol) and boron trifluoride etherate (0.52 mL, 4.2 mmol) were added sequentially using a plastic syringe. After the addition was complete, the mixture was stirred at room temperature overnight, with the reaction monitored by TLC until completion. Saturated sodium bicarbonate solution was then added to quench the reaction, followed by extraction with dichloromethane. The organic phase was washed twice with saturated brine, dried over anhydrous sodium sulfate, filtered, concentrated with silica gel added, and purified by automated column chromatography (5-10% ethyl acetate/petroleum ether) to obtain the desired product. Finally, 266 mg of white solid compound 50 was obtained with a yield of 30.7%. ¹H NMR (400 MHz, CDCl₃) δ 8.45 (s, 1H), 8.11 (d, J = 7.6 Hz, 1H), 7.60 - 7.52 (m, 3H), 6.24 (dd, J = 3.0, 1.6 Hz, 1H), 5.47 (d, J = 5.0 Hz, 1H), 3.25 (dt, J = 11.5, 6.3 Hz, 1H), 1.08 (s, 3H), 1.05 (s, 3H).

### (3S,10R,13S)-17-(1H-benzo[d]imidazol-1-yl)-10,13-dimethyl-2,3,4,7,8,9,10,11,12,13,14,15-dodecahydro-1H-cyclopenta[a]phenanthren-3-amine (51)

Compound 50 (266 mg, 0.643 mmol) was dissolved in anhydrous tetrahydrofuran and stirred in an ice bath. A solution of 1 M lithium aluminum hydride in tetrahydrofuran (1 mL, 1 mmol) was slowly added dropwise using a plastic syringe. After completion of addition, the mixture was stirred at room temperature for 2 h, and the reaction was monitored by TLC until completion. The reaction mixture was then placed in an ice bath and quenched with ice water. The product was extracted with ethyl acetate, and the organic phase was washed twice with saturated brine, dried over anhydrous sodium sulfate, filtered, and concentrated onto silica gel. Purification was performed by automated column chromatography (5-10% methanol/dichloromethane) to afford the desired product. Finally, 107 mg of compound 51 was obtained as a white solid in 43.3% yield. ¹H NMR (400 MHz, DMSO) δ 8.27 (s, 1H), 7.71 (d, J = 7.4 Hz, 1H), 7.57 (d, J = 7.4 Hz, 1H), 7.28 (ddd, J = 15.0, 13.7, 6.6 Hz, 2H), 6.06 (s, 1H), 5.32 (d, J = 4.3 Hz, 1H), 0.99 (s, 3H), 0.97 (s, 3H).

### N-((3S,10R,13S)-17-(1H-benzo[d]imidazol-1-yl)-10,13-dimethyl-2,3,4,7,8,9,10,11,12,13,14,15-dodecahydro-1H-cyclopenta[a]phenanthren-3-yl)pyridine-3-sulfonamide (Compound 6)

Compound 51 (50 mg, 0.129 mmol) and 4-dimethylaminopyridine (2 mg, 0.013 mmol) were placed in a flask. Anhydrous dichloromethane was added as solvent and the mixture was stirred in an ice bath. Triethylamine (54 µL, 0.39 mmol) was added and stirred for a period of time. Pyridine-3-sulfonyl chloride (20 µL, 0.155 mmol) was then slowly added. After completion of addition, the mixture was stirred at room temperature for 6 h, and the reaction was monitored by TLC until completion. Upon completion, silica gel was directly added to the reaction mixture, concentrated, and purified by automated column chromatography (30-60% ethyl acetate/petroleum ether) to afford the desired product. Finally, 35 mg of compound 6 was obtained as a white solid in 51.3% yield. ¹H NMR (600 MHz, CDCl₃) δ 9.09 (d, J = 1.9 Hz, 1H), 8.78 (dd, J = 4.8, 1.6 Hz, 1H), 8.16 (ddd, J = 8.0, 2.2, 1.7 Hz, 1H), 7.99 (s, 1H), 7.81 - 7.78 (m, 1H), 7.48 - 7.42 (m, 2H), 7.31 - 7.26 (m, 2H), 5.97 (dd, J = 3.1, 1.6 Hz, 1H), 5.31 (d, J = 7.0 Hz, 2H), 3.16 - 3.08 (m, 1H), 0.97 (s, 6H). 13C NMR (151 MHz, CDCl₃) δ 153.03, 147.92, 146.97, 141.52, 140.13, 138.18, 134.59, 134.42, 124.42, 123.74, 123.63, 122.76, 121.93, 119.94, 111.24, 55.75, 54.21, 50.36, 47.21, 40.28, 37.65, 36.59, 34.73, 30.96, 30.28, 30.23, 20.50, 19.15, 15.98. HRMS (ESI) (M+H)+ m/z calcd for C31H37N4O₂S 529.2632, found: 529.2635.

### Example 7:

### (3S,10R,13S)-17-(1H-benzo[d]imidazol-1-yl)-10,13-dimethyl-N-(pyridin-4-ylmethyl)-2,3,4,7,8,9,10,11,12,13,14,15-dodecahydro-1H-cyclopenta[a]phenanthren-3-amine (Compound 7)

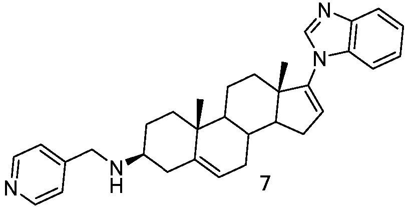

Compound 51 (50 mg, 0.129 mmol) was placed in a flask. Anhydrous N,N-dimethylformamide was added as solvent and the mixture was stirred at room temperature. Potassium carbonate (36 mg, 0.261 mmol) was added and stirred for a period of time. 4-(Bromomethyl)pyridine hydrochloride (33 mg, 0.130 mmol) was then added. The reaction mixture was slowly heated to 50°C and stirred overnight. The reaction was monitored by TLC until completion. Upon completion, the product was extracted with ethyl acetate and water. The organic phase was collected, washed twice with saturated brine, dried over anhydrous sodium sulfate, filtered, and concentrated onto silica gel. Purification was performed by automated column chromatography (2-5% methanol/dichloromethane) to afford the desired product. Finally, 32 mg of compound 7 was obtained as a white solid in 51.8% yield. ¹H NMR (600 MHz, CDCl₃) δ 8.53 (dd, J = 4.6, 1.3 Hz, 2H), 7.94 (s, 1H), 7.81 - 7.77 (m, 1H), 7.48 - 7.45 (m, 1H), 7.30 (d, J = 5.6 Hz, 2H), 7.29 - 7.26 (m, 2H), 5.96 (dd, J = 2.9, 1.6 Hz, 1H), 5.38 - 5.35 (m, 1H), 3.87 (s, 2H), 1.03 (s, 3H), 0.99 (s, 3H). 13C NMR (151 MHz, CDCl₃) δ 149.92, 147.17, 143.22, 141.64, 134.55, 124.11, 123.41, 123.25, 122.50, 120.81, 120.17, 111.15, 57.65, 55.87, 50.56, 49.31, 47.23, 37.68, 37.24, 34.86, 31.09, 30.33, 30.29, 20.59, 19.33, 16.02. HRMS (ESI) (M+H)+ m/z calcd for C32H39N4 479.3169, found: 479.3168.

### Example 8:

### N-((3S,10R,13S)-17-(1H-benzo[d]imidazol-1-yl)-10,13-dimethyl-2,3,4,7,8,9,10,11,12,13,14,15-dodecahydro-1H-cyclopenta[a]phenanthren-3-yl)isonicotinamide (Compound 8)

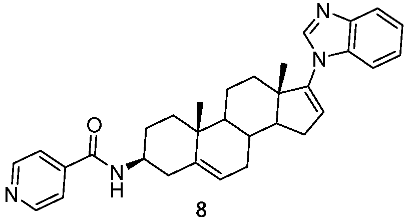

Compound 8 was synthesized using the same method as for compound 2. Starting from compound 51 (50 mg, 0.129 mmol), 24 mg of white solid compound 8 was obtained with a yield of 37.8%. ¹H NMR (500 MHz, DMSO) δ 8.74 (d, J = 4.4 Hz, 2H), 8.65 (s, 1H), 8.62 (d, J = 8.0 Hz, 1H), 7.80 (d, J = 5.9 Hz, 2H), 7.75 (d, J = 7.6 Hz, 1H), 7.65 (d, J = 7.7 Hz, 1H), 7.41 - 7.32 (m, 2H), 6.15 (s, 1H), 5.39 (d, J = 4.6 Hz, 1H), 3.77 - 3.67 (m, 1H), 1.03 (s, 3H), 0.97 (s, 3H). 13C NMR (126 MHz, DMSO) δ 163.98, 149.94, 146.22, 141.55, 126.38, 124.68, 123.90, 121.98, 120.98, 118.70, 112.46, 55.72, 50.39, 50.16, 47.17, 38.65, 37.96, 36.86, 34.17, 30.86, 30.41, 28.34, 20.63, 19.41, 16.07. HRMS (ESI) (M+H)+ m/z calcd for C32H37N4O 493.2962, found: 493.2969.

### Example 9:

### N-((3S,10R,13S)-17-(1H-benzo[d]imidazol-1-yl)-10,13-dimethyl-2,3,4,7,8,9,10,11,12,13,14,15-dodecahydro-1H-cyclopenta[a]phenanthren-3-yl)pyrazine-4-carboxamide (Compound 9)

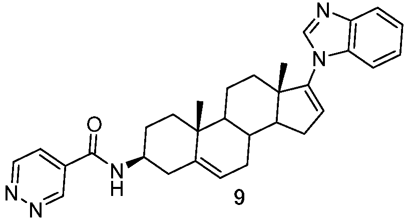

Compound 9 was synthesized using the same method as for compound 4. Starting from compound 51 (50 mg, 0.129 mmol), 24 mg of white solid compound 9 was obtained with a yield of 37.8%. ¹H NMR (600 MHz, CDCl₃) δ 9.53 (dd, J = 2.1, 1.2 Hz, 1H), 9.28 (dd, J = 5.2, 1.0 Hz, 1H), 7.94 (s, 1H), 7.86 (dd, J = 5.2, 2.3 Hz, 1H), 7.78 - 7.73 (m, 1H), 7.49 - 7.44 (m, 1H), 7.30 - 7.25 (m, 2H), 7.18 (d, J = 7.9 Hz, 1H), 5.97 (dd, J = 2.8, 1.5 Hz, 1H), 5.43 (d, J = 5.0 Hz, 1H), 3.94 (tdd, J = 12.1, 8.1, 4.2 Hz, 1H), 1.01 (s, 3H), 0.99 (s, 3H). 13C NMR (151 MHz, CDCl₃) δ 162.79, 151.81, 148.56, 142.98, 141.55, 140.26, 134.53, 132.28, 124.39, 124.16, 123.54, 122.62, 121.80, 120.02, 111.22, 55.77, 50.74, 50.42, 47.22, 38.87, 37.72, 36.81, 34.81, 31.05, 30.30, 28.71, 20.56, 19.19, 16.01. HRMS (ESI) (M+H)+ m/z calcd for C31H36N5O 494.2914, found: 494.2917.

### Example 10:

### N-((3S,10R,13S)-10,13-dimethyl-17-(pyrimidin-5-yl)-2,3,4,7,8,9,10,11,12,13,14,15-dodecahydro-1H-cyclopenta[a]phenanthren-3-yl)pyrazine-4-carboxamide (Compound 10)

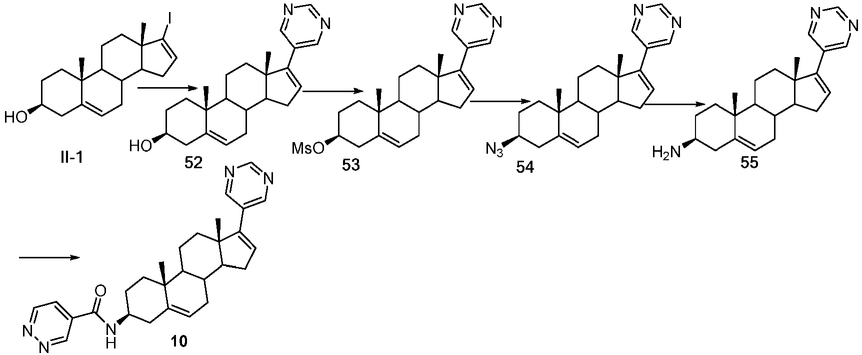

### (3S,10R,13S)-10,13-dimethyl-17-(pyrimidin-5-yl)-2,3,4,7,8,9,10,11,12,13,14,15-dodecahydro-1H-cyclopenta[a]phenanthren-3-ol (52)

To a flask containing compound II-1 (7 g, 17.6 mmol), 5-pyrimidinylboronic acid (2.68 g, 21.6 mmol), potassium carbonate (7.45 g, 54.02 mmol), and bis(triphenylphosphine)palladium(II) dichloride (1.28 g, 1.82 mmol), 60 mL of 1,4-dioxane and 20 mL of water were added and stirred at room temperature. The mixture was degassed with argon three times, then slowly heated to 100°C and reacted overnight. The next day, TLC monitoring showed the reaction was complete. The reaction mixture was extracted with ethyl acetate and water. The organic phase was collected, washed twice with saturated brine, dried over anhydrous sodium sulfate, filtered, and then concentrated with silica gel. The crude product was purified by automated column chromatography (30-50% ethyl acetate/petroleum ether) to give the desired product. Finally, 2.2 g of yellow solid compound 52 was obtained with a yield of 35.78%. ¹H NMR (400 MHz, CDCl₃) δ 9.05 (s, 1H), 8.72 (s, 2H), 6.10 (s, 1H), 5.38 (d, J = 5.1 Hz, 1H), 3.58 - 3.49 (m, 1H), 1.06 (s, 3H), 1.04 (s, 3H).

### (3S,10R,13S)-10,13-dimethyl-17-(pyrimidin-5-yl)-2,3,4,7,8,9,10,11,12,13,14,15-dodecahydro-1H-cyclopenta[a]phenanthren-3-yl methanesulfonate (53)

To a flask containing compound 52 (2.2 g, 6.28 mmol) and 4-dimethylaminopyridine (80 mg, 0.65 mmol), anhydrous dichloromethane was added as solvent and stirred in an ice bath. Triethylamine (2.6 mL, 19.34 mmol) was added and stirred for a while. Then methanesulfonyl chloride (1 mL, 11.54 mmol) was slowly added. After the addition was complete, the mixture was stirred at room temperature for 5 h. TLC monitoring showed the reaction was complete. Ice water was added to quench the reaction, followed by extraction with dichloromethane. The organic phase was washed twice with saturated brine, dried over anhydrous sodium sulfate, filtered, and then concentrated with silica gel. The crude product was purified by automated column chromatography (20-40% ethyl acetate/petroleum ether) to give the desired product. Finally, 534 mg of white solid compound 53 was obtained with a yield of 19.9%. ¹H NMR (400 MHz, CDCl₃) δ 9.09 (s, 1H), 8.74 (s, 2H), 6.13 (dd, J = 3.1, 1.7 Hz, 1H), 5.49 (d, J = 5.1 Hz, 2H), 4.60 - 4.51 (m, 1H), 3.04 (s, 3H), 1.11 (s, 3H), 1.07 (s, 3H).

### 5-((3S,10R,13S)-3-azido-10,13-dimethyl-2,3,4,7,8,9,10,11,12,13,14,15 -dodecahydro-1H-cyclopenta[a]phenanthren-17-yl)pyrimidine (54)

Compound 53 (534 mg, 1.24 mmol) was dissolved in anhydrous dichloromethane and stirred at room temperature. Then azidotrimethylsilane (0.5 mL, 3.77 mmol) and boron trifluoride etherate (0.6 mL, 4.83 mmol) were added sequentially via plastic syringe. After addition, the mixture was stirred overnight at room temperature and monitored by TLC until completion. The reaction was quenched by adding saturated sodium bicarbonate solution, followed by extraction with dichloromethane. The organic layer was washed twice with saturated brine, dried over anhydrous sodium sulfate, filtered, and concentrated with silica gel added. Purification by automated column chromatography (10-20% ethyl acetate/petroleum ether) afforded the desired product. Finally, 231 mg of compound 54 was obtained as a white solid in 49.4% yield.

### (3S,10R,13S)-10,13-dimethyl-17-(pyrimidin-5-yl)-2,3,4,7,8,9,10,11,12,13,14,15-dodecahydro-1H-cyclopenta[a]phenanthren-3-amine (55)

Compound 54 (231 mg, 0.615 mmol) was dissolved in anhydrous tetrahydrofuran and stirred in an ice bath. Then 1 M lithium aluminum hydride in tetrahydrofuran solution (0.9 mL, 0.9 mmol) was slowly added dropwise via plastic syringe. After addition, the mixture was stirred overnight at room temperature and monitored by TLC until completion. Upon reaction completion, the system was placed in an ice bath and quenched with ice water, followed by extraction with ethyl acetate. The organic layer was washed twice with saturated brine, dried over anhydrous sodium sulfate, filtered, and concentrated with silica gel added. Purification by automated column chromatography (5-8% methanol/dichloromethane) afforded the desired product. Finally, 101 mg of compound 55 was obtained as a white solid in 47.02% yield.

### N-((3S,10R,13S)-10,13-dimethyl-17-(pyrimidin-5-yl)-2,3,4,7,8,9,10,11,12,13,14,15-dodecahydro-1H-cyclopenta[a]phenanthren-3-yl)pyrazine-4-carboxamide (Compound 10)

Compound 55 (43 mg, 0.123 mmol) was used to obtain 42 mg of compound 10 as a yellow solid in 75% yield. ¹H NMR (400 MHz, DMSO) δ 9.52 (s, 1H), 9.41 (d, J = 5.3 Hz, 1H), 9.04 (s, 1H), 8.81 (s, 3H), 7.97 (dd, J = 5.2, 2.2 Hz, 1H), 6.28 (s, 1H), 5.37 (d, J = 4.1 Hz, 1H), 3.71 (dd, J = 11.8, 4.1 Hz, 1H), 1.04 (s, 3H), 1.01 (s,3H). ¹³C NMR (126 MHz, DMSO) δ 16221,156.70, 153.81,152.12,148.82, 147.92,140.88, 131.64, 131.25, 130.07, 124.07, 120.85, 56.89, 49.87, 49.61, 46.67, 38.17, 37.47, 36.37, 34.30, 31.52, 30.91, 29.88, 27.87, 20.35, 18.97, 16.08. HRMS (ESI) (M+H)+ m/z calcd for C28H34N5O 456.2758, found: 456.2762.

### Example 11:

### N-((3S,10R,13S)-10,13-dimethyl-17-(1H-1,2,3-triazol-1-yl)-2,3,4,7,8,9,10,11,12,13,14,15-dodecahydro-1H-cyclopenta[a]phenanthren-3-yl)pyrazine-4-carboxamide (Compound 11)

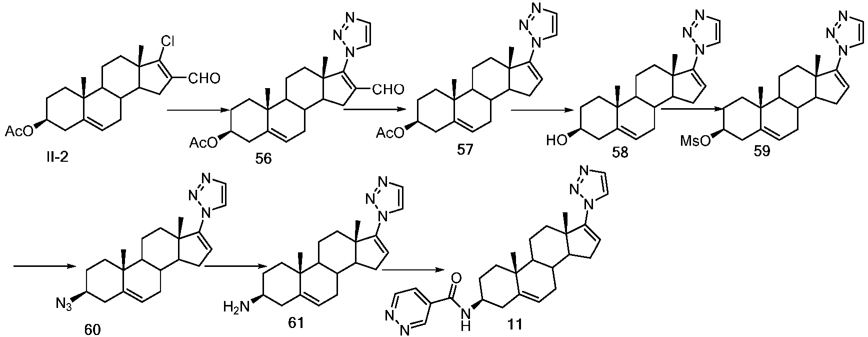

### (3S,10R,13S)-16-formyl-10,13-dimethyl-17-(1H-1,2,3-triazol-1-yl)-2,3,4,7,8,9,10,11,12,13,14,15-dodecahydro-1H-cyclopenta[a]phenanthren-3-yl acetate (56)

Compound II-2 (2 g, 5.3 mmol) and potassium carbonate (2.56 g, 18.6 mmol) were added to a flask. Anhydrous N,N-dimethylformamide was added as solvent and the mixture was stirred at room temperature for half an hour. Then 1,2,3-triazole (0.9 mL, 15.9 mmol) was added to the system. The temperature was slowly raised to 80°C and stirred overnight at this temperature, monitored by TLC until completion. After the reaction was complete, the mixture was extracted with ethyl acetate. The organic phase was collected, washed twice with saturated brine, dried over anhydrous sodium sulfate, filtered, and concentrated with silica gel added. Purification by automated column chromatography (30-50% ethyl acetate/petroleum ether) afforded the desired product. Finally, 1.26 g of compound 56 was obtained as a white solid in 58% yield. ¹H NMR (400 MHz, CDCl₃) δ 9.92 (s, 1H), 7.85 (d, J = 1.1 Hz, 1H), 7.83 (d, J = 1.1 Hz, 1H), 5.41 (d, J = 4.9 Hz, 1H), 4.59 (dt, J = 10.8, 6.1 Hz, 1H), 2.03 (s, 3H), 1.16 (s, 3H), 1.07 (s, 3H).

### (3S,10R,13S)-10,13-dimethyl-17-(1H-1,2,3-triazol-1-yl)-2,3,4,7,8,9,10,11,12,13,14,15-dodecahydro-1H-cyclopenta[a]phenanthren-3-yl acetate (57)

Compound 56 (2 g, 4.89 mmol) was dissolved in anhydrous N,N-dimethylformamide and stirred at room temperature. Palladium on carbon (1 g) was added, and the system was purged with argon three times. The temperature was slowly raised to 160°C and the reaction was carried out for 3 days, with TLC monitoring until completion. After the reaction was complete, the reaction system was cooled to room temperature, and a certain amount of ethyl acetate was added. After stirring for a period of time, the mixture was filtered through diatomaceous earth, and the filtrate was collected. The organic phase was extracted with ethyl acetate and water, washed twice with saturated brine, dried over anhydrous sodium sulfate, and filtered. Silica gel was added to the system, concentrated, and purified by automated column chromatography (20-25% ethyl acetate/petroleum ether) to obtain the desired product. Finally, 720 mg of yellow solid compound 57 was obtained with a yield of 38.6%. ¹H NMR (400 MHz, CDCl₃) δ 7.72 (d, J = 1.0 Hz, 1H), 7.71 (d, J = 1.0 Hz, 1H), 5.96 (dd, J = 3.1, 1.7 Hz, 1H), 5.41 (d, J = 5.0 Hz, 1H), 4.61 (dt, J = 11.4, 4.4 Hz, 1H), 2.03 (s, 3H), 1.13 (s, 3H), 1.08 (s, 3H).

### (3S,10R,13S)-10,13-dimethyl-17-(1H-1,2,3-triazol-1-yl)-2,3,4,7,8,9,10,11,12,13,14,15-dodecahydro-1H-cyclopenta[a]phenanthren-3-ol (58)

Compound 57 (720 mg, 1.88 mmol) was dissolved in anhydrous methanol and stirred at room temperature. Potassium hydroxide (0.4 g, 7.3 mmol) was added, and the mixture was stirred at room temperature for 4 h, with TLC monitoring until completion. After the reaction was complete, part of the solvent was removed by rotary evaporation. The organic phase was extracted with ethyl acetate and water, washed twice with saturated brine, dried over anhydrous sodium sulfate, and filtered. The target compound was obtained by direct rotary evaporation and drying. 554 mg of yellow solid compound 58 was obtained with a yield of 86.4%. ¹H NMR (400 MHz, CDCl₃) δ 7.72 (s, 1H), 7.71 (s, 1H), 5.97 (s, 1H), 5.38 (d, J = 4.7 Hz, 1H), 3.54 (m, 1H), 1.13 (s, 3H), 1.07 (s, 3H).

### (3S,10R,13S)-10,13-dimethyl-17-(1H-1,2,3-triazol-1-yl)-2,3,4,7,8,9,10,11,12,13,14,15-dodecahydro-1H-cyclopenta[a]phenanthren-3-yl methanesulfonate (59)

Compound 58 (550 mg, 1.62 mmol) and 4-dimethylaminopyridine (20 mg, 0.16 mmol) were placed in a flask, and anhydrous dichloromethane was added as a solvent. The mixture was stirred in an ice bath, then triethylamine (0.7 mL, 5.04 mmol) was added and stirred for a period of time. Finally, methanesulfonyl chloride (0.25 mL, 3.3 mmol) was slowly added. After the addition was complete, the mixture was stirred at room temperature for 5 h, with TLC monitoring until completion. Ice water was added to quench the reaction, followed by extraction with dichloromethane. The organic phase was washed twice with saturated brine, dried over anhydrous sodium sulfate, filtered, and the solvent was removed by rotary evaporation to obtain the desired product. Finally, 500 mg of white solid 59 was obtained with a yield of 74%. ¹H NMR (400 MHz, CDCl₃) δ 7.75 (s, 1H), 7.73 (s, 1H), 5.98 (s, 1H), 5.47 (d, J = 5.1 Hz, 1H), 4.60 - 4.49 (m, 1H), 3.03 (s, 3H), 1.15 (s, 3H), 1.10 (s, 3H).

### 1-((3S,10R,13S)-3-azido-10,13-dimethyl-2,3,4,7,8,9,10,11,12,13,14,15-dodecahydro-1H-cyclopenta[a]phenanthren-17-yl)-1H-1,2,3-triazole (60)

Compound 59 (100 mg, 0.24 mmol) was dissolved in anhydrous dichloromethane and stirred at room temperature. Azidotrimethylsilane (47 µL, 0.26 mmol) and boron trifluoride etherate (59 µL, 0.48 mmol) were added sequentially using a plastic syringe. After the addition was complete, the mixture was stirred at room temperature overnight, with TLC monitoring until completion. Saturated sodium bicarbonate solution was added to quench the reaction, followed by extraction with dichloromethane. The organic phase was washed twice with saturated brine, dried over anhydrous sodium sulfate, filtered, and silica gel was added to the organic phase. The mixture was concentrated and purified by automated column chromatography (10-20% ethyl acetate/petroleum ether) to obtain the desired product. Finally, 63 mg of white solid 60 was obtained with a yield of 72.16%. ¹H NMR (400 MHz, CDCl₃) δ 7.75 (s, 1H), 7.74 (s, 1H), 5.99 (dd, J = 3.1, 1.8 Hz, 1H), 5.45 (d, J = 5.1 Hz, 1H), 3.24 (ddd, J = 10.4, 8.8, 4.1 Hz, 1H), 1.15 (s, 3H), 1.08 (s, 3H).

### (3S,10R,13S)-10,13-dimethyl-17-(1H-1,2,3-triazol-1-yl)-2,3,4,7,8,9,10,11,12,13,14,15-dodecahydro-1H-cyclopenta[a]phenanthren-3-amine (61)

Compound 60 (63 mg, 0.173 mmol) was dissolved in anhydrous tetrahydrofuran and stirred at room temperature. A 1 M solution of lithium aluminum hydride in tetrahydrofuran (0.3 mL, 0.3 mmol) was added, and the reaction was carried out for 2 hours with TLC monitoring until completion. Saturated ammonium chloride solution was added to the reaction system to quench the reaction, followed by extraction with ethyl acetate. The organic phase was collected, washed twice with saturated brine, dried over anhydrous sodium sulfate, filtered, and finally concentrated by rotary evaporation to obtain the desired product. Finally, 30 mg of white solid compound 61 was obtained with a yield of 51.2%. ¹H NMR (400 MHz, DMSO) δ 8.41 (s, 1H), 7.81 (d, J = 0.9 Hz, 1H), 6.12 (s, 1H), 5.27 (s, 1H), 1.05 (s, 3H), 0.99 (s, 3H).

### N-((3S,10R,13S)-10,13-dimethyl-17-(1H-1,2,3-triazol-1-yl)-2,3,4,7,8,9,10,11,12,13,14,15-dodecahydro-1H-cyclopenta[a]phenanthren-3-yl)pyrazine-4-carboxamide (Compound 11)

Starting from compound 61 (50 mg, 0.148 mmol), 58 mg of white solid compound 11 was obtained, with a yield of 88.3%. ¹H NMR (400 MHz, DMSO) δ 9.55 - 9.53 (m, 1H), 9.43 (dd, J = 5.3, 1.0 Hz, 1H), 8.85 (d, J = 7.9 Hz, 1H), 8.45 (d, J = 0.8 Hz, 1H), 8.00 (dd, J = 5.3, 2.3 Hz, 1H), 7.83 (d, J = 0.9 Hz, 1H), 6.15 (s, 1H), 5.39 (d, J = 4.5 Hz, 1H), 3.79 - 3.67 (m, 1H), 1.08 (s, 3H), 1.06 (s, 3H). 13C NMR (151 MHz, DMSO) δ 162.12, 152.10, 148.82, 133.25, 124.13, 123.46, 120.75, 118.75, 67.03, 55.84, 49.84, 45.88, 38.16, 37.41, 36.39, 34.25, 30.48, 29.64, 29.49, 27.84, 25.14, 20.08, 18.86, 15.51. HRMS (ESI) (M+H)+ m/z calculated for C26H33N6O 445.271, found: 445.2722.

### Example 12:

### N-((3S,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl)nicotinamide (Compound 12)

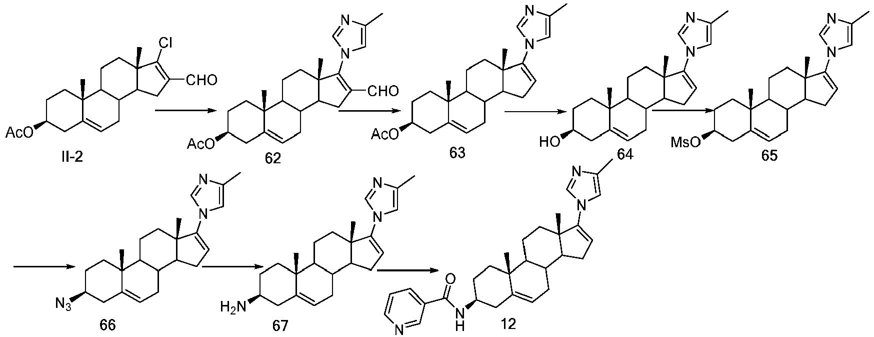

### (3S,10R,13S)-16-formyl-10,13-dimethyl-17-(4-methyl-1H-imidazol-1-yl)-2,3,4,7,8,9,10,11,12,13,14,15-dodecahydro-1H-cyclopenta[a]phenanthren-3-yl acetate (62)

Compound II-2 (7.90 g, 20.96 mmol) and potassium carbonate (7.24 g, 52.40 mmol) were added to a flask, followed by the addition of anhydrous N,N-dimethylformamide as solvent. After stirring at room temperature for half an hour, 4-methylimidazole (2.06 g, 25.09 mmol) was added to the system. The temperature was slowly raised to 80°C and stirred overnight at this temperature. The reaction was monitored by TLC until completion. After the reaction was complete, the mixture was extracted with ethyl acetate. The organic phase was collected, washed twice with saturated brine, dried over anhydrous sodium sulfate, filtered, and then concentrated with silica gel. The product was purified by automated column chromatography (30-50% ethyl acetate/petroleum ether). Finally, 7.0 g of yellow solid compound 62 was obtained with ayield of 79%. ¹H NMR (400 MHz, CDCl₃) δ 9.77 (s, 1H), 7.54 (d, J = 1.1 Hz, 1H), 6.84 (s, 1H), 5.43 (d, J = 5.2 Hz, 1H), 4.62 (tt, J = 10.5, 5.4 Hz, 1H), 2.29 (d, J = 0.6 Hz, 3H), 2.06 (s, 3H), 1.08 (s, 6H).

### (3S,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl acetate (63)

Compound 62 (6.30 g, 14.91 mmol) was dissolved in anhydrous N,N-dimethylformamide and stirred at room temperature. Then, 5 g of palladium on carbon was added. The system was purged with argon three times, and the temperature was slowly raised to 160°C. The reaction was carried out for 18 hours, monitored by TLC until completion. After the reaction was complete, the reaction system was cooled to room temperature, and a certain amount of ethyl acetate was added. After stirring for a period of time, the mixture was filtered through diatomaceous earth. The filtrate was collected and extracted with ethyl acetate and water. The organic phase was collected, washed twice with saturated brine, dried over anhydrous sodium sulfate, filtered, and then concentrated with silica gel. The product was purified by automated column chromatography (20-30% ethyl acetate/petroleum ether). Finally, 3.0 g of yellow solid compound 63 was obtained with a yield of 50.6%. ¹H NMR (400 MHz, CDCl₃) δ 7.50 (s, 1H), 6.75 (s, 1H), 5.60 (s, 1H), 5.40 (d, J = 4.8 Hz, 1H), 4.60 (tt, J = 10.8, 5.3 Hz, 1H), 2.22 (s, 3H), 2.02 (s, 3H), 1.06 (s, 3H), 0.98 (s, 3H).

### (3S,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3 -ol (64)

Compound 63 (1.80 g, 4.56 mmol) was dissolved in anhydrous methanol and stirred at room temperature. Then, potassium hydroxide (1.01 g, 18.24 mmol) was added. After the addition was complete, the mixture was stirred at room temperature for 4 h. The reaction was monitored by TLC until completion. After the reaction was complete, part of the solvent was removed by rotary evaporation. The mixture was then extracted with ethyl acetate and water. The organic phase was collected, washed twice with saturated brine, dried over anhydrous sodium sulfate, filtered, and then directly dried by rotary evaporation to obtain the target compound. 1.6 g of yellow solid compound 64 was obtained with a yield of 99.5%. ¹H NMR (400 MHz, CDCl₃) δ 7.53 (d, J = 0.9 Hz, 1H), 6.78 (s, 1H), 5.63 (dd, J = 3.0, 1.7 Hz, 1H), 5.40 (d, J = 5.2 Hz, 1H), 3.61 - 3.50 (m, 1H), 2.25 (s, 3H), 1.07 (s, 3H), 1.01 (s, 3H).

### (3S,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl methanesulfonate (65)

Compound 64 (1.06 g, 3.01 mmol) and 4-dimethylaminopyridine (37 mg, 0.30 mmol) were added to a flask, followed by addition of anhydrous dichloromethane as solvent with stirring in an ice bath. Triethylamine (1.25 mL, 9.03 mmol) was then added and stirred for a period of time. Methanesulfonyl chloride (0.47 mL, 6.01 mmol) was slowly added dropwise. After completion of addition, the mixture was stirred at room temperature for 10 h and monitored by TLC until the reaction was complete. Ice water was then added for quenching, followed by extraction with dichloromethane. The organic phase was washed twice with saturated brine, dried over anhydrous sodium sulfate, filtered, and then silica gel was added to the system for concentration and purification by automated column chromatography (30-50% ethyl acetate/petroleum ether) to obtain the desired product. Finally, 1.05 g of yellow solid compound 65 was obtained with a yield of 81.4%. ¹H NMR (400 MHz, CDCl₃) δ 7.69 (s, 1H), 6.80 (s, 1H), 5.69 (d, J = 1.3 Hz, 1H), 5.47 (d, J = 4.8 Hz, 1H), 4.60 - 4.49 (m, 1H), 3.04 (s, 3H), 2.28 (s, 3H), 1.09 (s, 3H), 1.01 (s, 3H).

### 1-((3S,10R,13S)-3-azido-10,13-dimethyl-2,3,4,7,8,9,10,11,12,13,14,15-dodecahydro-1H-cyclopenta[a]phenanthren-17-yl)-4-methyl-1H-imidazole (66)

Compound 65 (1.95 g, 4.53 mmol) was dissolved in anhydrous dichloromethane and stirred at room temperature. Azidotrimethylsilane (3.6 mL, 27.18 mmol) and boron trifluoride etherate (5.6 mL, 45.30 mmol) were added sequentially using a plastic syringe. After completion of addition, the mixture was stirred at room temperature for 5 hours and monitored by TLC until the reaction was complete. Saturated sodium bicarbonate solution was then added for quenching, followed by extraction with dichloromethane. The organic phase was washed twice with saturated brine, dried over anhydrous sodium sulfate, filtered, and then silica gel was added to the organic phase for concentration and purification by automated column chromatography (20-40% ethyl acetate/petroleum ether) to obtain the desired product. Finally, 1.55 g of white solid 66 was obtained with a yield of 90.64%. ¹H NMR (400 MHz, DMSO) δ 8.06 (s, 1H), 6.84 (s, 1H), 5.92 - 5.88 (m, 1H), 5.42 (d, J = 4.9 Hz, 1H), 3.27 - 3.17 (m, 1H), 2.39 (s, 3H), 1.05 (s, 3H), 1.01 (s, 3H).

### (3S,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3 -amine (67)

Compound 66 (1.55 g, 4.11 mmol) was dissolved in 16 mL of tetrahydrofuran, 16 mL of methanol and 4 mL of water, and stirred at room temperature. Triphenylphosphine (3.23 g, 12.33 mmol) was added, and then the temperature was slowly raised to 60°C and stirred overnight at this temperature. The reaction was monitored by TLC until completion. The solvent was partially removed by rotary evaporation, 20 mL of dichloromethane was added to dissolve, and 2 M hydrochloric acid was slowly added to adjust the pH of the solution to about 2. The layers were separated and the aqueous phase was collected. Then 2 M sodium hydroxide solution was added to adjust the pH to weakly alkaline, at which point a large amount of white solid precipitated. The aqueous phase was extracted three times with a small amount of dichloromethane, and the organic phases were combined, dried over anhydrous sodium sulfate, and rotary evaporated to remove the solvent to obtain 350 mg of white solid 67, with a yield of 24.3%. ¹H NMR (400 MHz, CDCl₃) δ 7.53 (s, 1H), 6.78 (s, 1H), 5.63 (dd, J = 3.0, 1.7 Hz, 1H), 5.37 (d, J = 5.2 Hz, 1H), 2.25 (s, 3H), 1.05 (s, 3H), 1.01 (s, 3H).

### N-((3S,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl)nicotinamide (Compound 12)

Compound 67 (55 mg, 0.156 mmol) was used to obtain 55 mg of coffee-colored solid compound 12, with a yield of 77%. ¹H NMR (400 MHz, DMSO) δ 8.98 (d, J = 1.7 Hz, 1H), 8.67 (dd, J = 4.8, 1.5 Hz, 1H), 8.49 (d, J = 7.9 Hz, 1H), 8.18 - 8.14 (m, 1H), 7.73 (s, 1H), 7.48 (dd, J = 7.9, 4.9 Hz, 1H), 7.05 (s, 1H), 5.73 (s, 1H), 5.36 (d, J = 4.0 Hz, 1H), 3.77 - 3.65 (m, 1H), 2.09 (s, 3H), 1.03 (s, 3H), 0.96 (s, 3H). 13C NMR (126 MHz, DMSO) δ 163.90, 151.73, 148.40, 141.15, 137.05, 134.95, 134.73, 123.31, 120.51, 116.43, 114.56, 55.84, 49.81, 49.50, 45.37, 38.36, 37.53, 36.40, 34.09, 30.38, 29.68, 29.17, 28.01, 20.13, 18.95, 15.64, 13.35. HRMS (ESI) (M+H)+ m/z calculated for C29H37N4O 445.271, found: 445.2722.

### Example 13:

### N-((3S,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl)pyrazine-4-carboxamide (Compound 13)

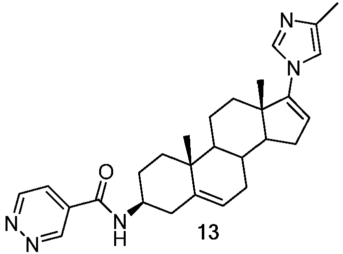

Compound 67 (55 mg, 0.156 mmol) was used to obtain 46 mg of pale yellow solid compound 13 with a yield of 64.3%. ¹H NMR (400 MHz, DMSO) δ 9.52 (s, 1H), 9.41 (d, J = 4.4 Hz, 1H), 8.82 (d, J = 7.8 Hz, 1H), 7.97 (dd, J = 5.2, 2.2 Hz, 1H), 7.68 (s, 1H), 7.02 (s, 1H), 5.71 (s, 1H), 5.36 (d, J = 3.3 Hz, 1H), 3.71 (d, J = 7.2 Hz, 1H), 2.08 (s, 3H), 1.03 (s, 3H), 0.95 (s, 3H). ¹³C NMR (126 MHz, DMSO) δ 162.20,152.08, 148.82,140.88, 134.77, 124.12, 120.74, 116.14, 114.38, 55.82, 54.92, 49.86, 49.79, 45.39, 38.15, 37.42, 36.36, 34.08, 30.46, 29.66, 29.10, 27.85, 20.18, 18.92, 15.64, 13.47. HRMS (ESI) (M+H)+ m/z calcd for C28H36N5O 458.2914, found: 458.2912.

### Example 14:

### N-((3S,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl)isonicotinamide (Compound 14)

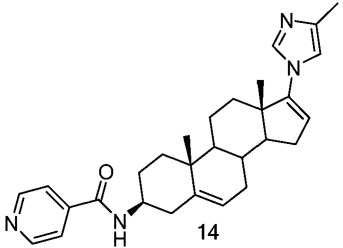

Compound 67 (55 mg, 0.156 mmol) was used to obtain 52 mg of white solid compound 14 with a yield of 72.8%. ¹H NMR (500 MHz, DMSO) δ 8.72 (dd, J = 4.4, 1.6 Hz, 2H), 8.59 (d, J = 8.0 Hz, 1H), 7.81 (d, J = 0.7 Hz, 1H), 7.75 (dd, J = 4.5, 1.6 Hz, 2H), 7.10 (s, 1H), 5.79 - 5.76 (m, 1H), 5.39 (d, J = 4.9 Hz, 1H), 3.78 - 3.68 (m, 1H), 2.12 (d, J = 0.5 Hz, 3H), 1.06 (s, 3H), 0.99 (s, 3H). 13C NMR (126 MHz, DMSO) δ 164.19, 150.62, 148.26, 142.08, 137.21, 135.12, 121.64, 121.01, 115.15, 56.30, 50.35, 50.10, 45.87, 38.67, 37.96, 36.85, 34.47, 30.92, 30.14, 29.64, 28.30, 20.63, 19.40, 16.08, 13.65. HRMS (ESI) (M+H)+ m/z calcd for C29H37N4O 457.2962, found: 457.2959.

### Example 15:

### N-((3S,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl)-3-fluoroisonicotinamide (Compound 15)

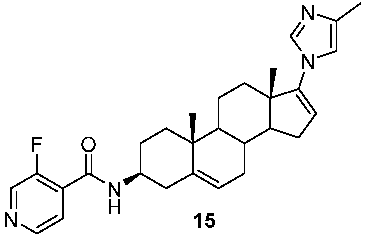

Compound 67 (100 mg, 0.285 mmol) was used to obtain 89 mg of brown solid compound 15 with a yield of 65.9%. ¹H NMR (600 MHz, DMSO) δ 8.68 (s, 1H), 8.59 (d, J = 7.9 Hz, 1H), 8.53 (d, J = 4.7 Hz, 1H), 7.71 (s, 1H), 7.57 - 7.54 (m, 1H), 7.06 (s, 1H), 5.74 (s, 1H), 5.40 (d, J = 4.6 Hz, 1H), 3.68 (dt, J = 16.9, 8.6 Hz, 1H), 2.11 (s, 3H), 1.03 (s, 3H), 0.97 (s, 3H). 13C NMR (151 MHz, DMSO) δ 161.60, 148.38, 146.64, 141.34, 139.13, 137.71, 135.23, 123.72, 121.12, 116.60, 114.86, 56.29, 50.20, 50.15, 45.85, 38.65, 37.86, 36.79, 34.52, 30.93, 30.11, 29.62, 28.37, 20.65, 19.39, 16.11, 13.93. HRMS (ESI) (M+H)+ m/z calcd for C29H36FN4O 457.2868, found: 457.2874.

### Example 16:

### N-((3S,10R,13S)-17-(1H-imidazol-1-yl)-10,13-dimethyl-2,3,4,7,8,9,10,11,12,13,14,15-dodecahydro-1H-cyclopenta[a]phenanthren-3-yl)pyrazine-4-carboxamide (Compound 16)

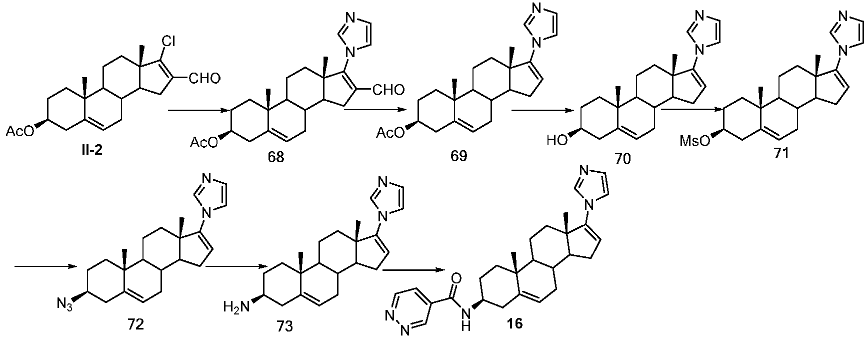

### (3S,10R,13S)-16-formyl-17-(1H-imidazol-1-yl)-10,13-dimethyl-2,3,4,7,8,9,10,11,12,13,14,15-dodecahydro-1H-cyclopenta[a]phenanthren-3-yl acetate (68)

Compound II-2 (6 g, 15.92 mmol) and potassium carbonate (4.39 g, 31.83 mmol) were added to a flask. Anhydrous N,N-dimethylformamide was added as solvent and stirred at room temperature for half an hour. Imidazole (1.08 g, 15.86 mmol) was then added to the system. The temperature was slowly raised to 80°C and stirred overnight. The reaction was monitored by TLC until completion. After the reaction was complete, the mixture was extracted with ethyl acetate. The organic phase was collected, washed twice with saturated brine, dried over anhydrous sodium sulfate, filtered, and then silica gel was added to the system. The mixture was concentrated and purified by automated column chromatography (30-50% ethyl acetate/petroleum ether) to obtain the desired product. Finally, 2.27 g of yellow solid compound 68 was obtained with a yield of 34.97%. ¹H NMR (400 MHz, CDCl₃) δ 9.72 (s, 1H), 7.61 (s, 1H), 7.20 (s, 1H), 7.09 (s, 1H), 5.40 (d, J = 4.6 Hz, 1H), 4.58 (dt, J = 10.8, 5.7 Hz, 1H), 2.01 (s, 3H), 1.05 (s, 6H).

### (3S,10R,13S)-17-(1H-imidazol-1-yl)-10,13-dimethyl-2,3,4,7,8,9,10,11,12,13,14,15-dodecahydro-1H-cyclopenta[a]phenanthren-3-yl acetate (69)

Compound 68 (2.27 g, 5.56 mmol) was dissolved in anhydrous N,N-dimethylformamide and stirred at room temperature. Then 2.27 g of palladium on carbon was added, followed by purging with argon three times. The system temperature was slowly raised to 160°C and reacted for 20 hours, with TLC monitoring until the reaction was complete. After the reaction was finished, the reaction system was cooled to room temperature and a certain amount of ethyl acetate was added. After stirring for a period of time, the mixture was filtered through diatomaceous earth and the filtrate was collected. The filtrate was then extracted with ethyl acetate and water. The organic phase was collected, washed twice with saturated brine, dried over anhydrous sodium sulfate, filtered, and then silica gel was added to the system for concentration and purification by automated column chromatography (30-50% ethyl acetate/petroleum ether) to obtain the desired product. Finally, 875 mg of yellow solid compound 69 was obtained with a yield of 41.47%. The product was directly used in the next step reaction.

### (3S,10R,13S)-17-(1H-imidazol-1-yl)-10,13-dimethyl-2,3,4,7,8,9,10,11,12,13,14,15-dodecahydro-1H-cyclopenta[a]phenanthren-3-ol (70)

Compound 69 (860 mg, 2.26 mmol) was dissolved in anhydrous methanol and stirred at room temperature. Then pre-weighed potassium hydroxide (0.37 g, 6.78 mmol) was added. After addition was complete, the mixture was stirred at room temperature for 4 h, with TLC monitoring until the reaction was complete. After the reaction was finished, part of the solvent was removed by rotary evaporation, and then extracted with ethyl acetate and water. The organic phase was collected, washed twice with saturated brine, dried over anhydrous sodium sulfate, filtered, and then directly concentrated by rotary evaporation to obtain the target compound. 730 mg of yellow solid compound 70 was obtained with a yield of 95.4%. The product was then used in the next step.

### (3S,10R,13S)-17-(1H-imidazol-1-yl)-10,13-dimethyl-2,3,4,7,8,9,10,11,12,13,14,15-dodecahydro-1H-cyclopenta[a]phenanthren-3-yl methanesulfonate (71)

Compound 70 (105 mg, 0.31 mmol) and 4-dimethylaminopyridine (4 mg, 0.03 mmol) were placed in a flask, and anhydrous dichloromethane was added as solvent. The mixture was stirred in an ice bath, then triethylamine (129 µL, 0.93 mmol) was added and stirred for a period of time. Finally, methanesulfonyl chloride (48 µL, 0.62 mmol) was slowly added. After the addition was complete, the mixture was stirred at room temperature for 5 h, with TLC monitoring until the reaction was complete. Ice water was then added to quench the reaction, followed by extraction with dichloromethane. The organic phase was washed twice with saturated brine, dried over anhydrous sodium sulfate, filtered, and then silica gel was added to the system for concentration and purification by automated column chromatography (40-60% ethyl acetate/petroleum ether) to obtain the desired product. Finally, 111 mg of white solid compound 71 was obtained with a yield of 86.3%. ¹H NMR (400 MHz, CDCl₃) δ 7.62 (s, 1H), 7.08 (s, 1H), 7.05 (s, 1H), 5.69 (dd, J = 3.0, 1.6 Hz, 1H), 5.46 (d, J = 5.1 Hz, 1H), 4.58 - 4.48 (m, 1H), 3.02 (s, 3H), 1.07 (s, 3H), 1.00 (s, 3H).

### 1-((3S,10R,13S)-3-azido-10,13-dimethyl-2,3,4,7,8,9,10,11,12,13,14,15-dodecahydro-1H-cyclopenta[a]phenanthren-17-yl)-1H-imidazole (72)

Compound 71 (44 mg, 0.106 mmol) was dissolved in anhydrous dichloromethane and stirred at room temperature. Then azidotrimethylsilane (0.15 mL, 1.13 mmol) and boron trifluoride etherate (0.15 mL, 1.21 mmol) were added sequentially using a plastic syringe. After the addition was complete, the mixture was stirred at room temperature for 5 hours, with TLC monitoring until the reaction was complete. Saturated sodium bicarbonate solution was then added to quench the reaction, followed by extraction with dichloromethane. The organic phase was washed twice with saturated brine, dried over anhydrous sodium sulfate, filtered, and then silica gel was added to the organic phase for concentration and purification by automated column chromatography (20-40% ethyl acetate/petroleum ether) to obtain the desired product. Finally, 10 mg of white solid 72 was obtained with a yield of 26.04%. ¹H NMR (400 MHz, CDCl₃) δ 8.16 (s, 1H), 7.39 (s, 1H), 7.16 (s, 1H), 6.01 (s, 1H), 5.45 (d, J = 4.9 Hz, 1H), 3.24 (dt, J = 16.1, 5.8 Hz, 1H), 1.08 (s, 3H), 1.05 (s, 3H).

### (3S,10R,13S)-17-(1H-imidazol-1-yl)-10,13-dimethyl-2,3,4,7,8,9,10,11,12,13,14,15-dodecahydro-1H-cyclopenta[a]phenanthren-3-amine (73)

Compound 72 (157 mg, 0.432 mmol) was dissolved in 6 mL of tetrahydrofuran, 6 mL of methanol and 1.5 mL of water, and stirred at room temperature. Then pre-weighed triphenylphosphine (453 mg, 1.73 mmol) was added, and the temperature was slowly raised to 60° C. The mixture was stirred overnight at this temperature, with TLC monitoring until the reaction was complete. Then part of the solvent was removed by rotary evaporation, 20 mL of dichloromethane was added to dissolve the residue, and 2 M hydrochloric acid was slowly added to adjust the pH of the solution to about 2. The layers were separated and the aqueous phase was collected. Then 2 M sodium hydroxide solution was added to adjust the pH to weakly alkaline, at which point a large amount of white solid precipitated. The aqueous phase was extracted three times with a small amount of dichloromethane, and the combined organic phases were collected, dried over anhydrous sodium sulfate, and concentrated by rotary evaporation to remove the solvent, yielding 80 mg of white solid 73 with a yield of 54.9%. ¹H NMR (400 MHz, DMSO) δ 8.10 (s, 2H), 7.84 (s, 1H), 7.36 (s, 1H), 7.00 (s, 1H), 5.83 (s, 1H), 5.41 (d, J = 4.8 Hz, 1H), 1.01 (s, 3H), 0.99 (s, 3H).

### N-((3S,10R,13S)-17-(1H-imidazol-1-yl)-10,13-dimethyl-2,3,4,7,8,9,10,11,12,13,14,15-dodecahydro-1H-cyclopenta[a]phenanthren-3-yl)pyrazine-2-carboxamide (Compound 16)

Compound 73 (35 mg, 0.104 mmol) was used to obtain 28 mg of white solid compound 16, with a yield of 61%. ¹H NMR (500 MHz, DMSO) δ 9.56 (d, J = 0.7 Hz, 1H), 9.42 (d, J = 5.2 Hz, 1H), 9.25 (s, 1H), 8.97 (d, J = 7.8 Hz, 1H), 8.05 (dd, J = 5.3, 2.3 Hz, 1H), 7.90 (s, 1H), 7.80 (s, 1H), 6.26 (d, J = 1.3 Hz, 1H), 5.39 (d, J = 4.6 Hz, 1H), 3.78 - 3.69 (m, 1H), 1.06 (s, 3H), 1.02 (s, 3H). 13C NMR (126 MHz, DMSO) δ 162.64, 152.53, 149.31, 146.43, 141.41, 132.14, 124.63, 121.57, 121.42, 121.00, 56.29, 50.40, 50.17, 46.07, 38.55, 37.87, 36.83, 33.43, 30.80, 30.04, 29.95, 28.19, 20.35, 19.37, 15.66. HRMS (ESI) (M+H)+m/z calculated for C27H34N5O 444.2758, measured: 444.2757.

### Example 17:

### N-((3S,10R,13S)-17-(1H-imidazol-1-yl)-10,13-dimethyl-2,3,4,7,8,9,10,11,12,13,14,15-dodecahydro-1H-cyclopenta[a]phenanthren-3-yl)isonicotinamide (Compound 17)

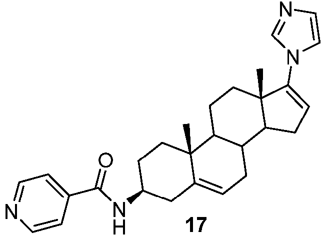

Compound 73 (35 mg, 0.104 mmol) was used to obtain 17 mg of white solid compound 17, with a yield of 37%. ¹H NMR (500 MHz, DMSO) δ 9.33 (s, 1H), 8.88 (d, J = 6.0 Hz, 2H), 8.79 (d, J = 7.9 Hz, 1H), 8.01 (d, J = 6.2 Hz, 2H), 7.95 (s, 1H), 7.87 (s, 1H), 6.29 (s, 1H), 5.40 (d, J = 4.4 Hz, 1H), 3.79 - 3.69 (m, 1H), 1.07 (s, 3H), 1.03 (s, 3H). 13C NMR (126 MHz, DMSO) δ 163.24, 147.80, 146.19, 141.47, 134.57, 125.71, 123.31, 121.65, 121.00, 56.29, 50.33, 50.17, 46.03, 38.55, 37.88, 36.84, 33.44, 30.79, 30.04, 29.99, 28.25, 20.34, 19.37, 15.63. HRMS (ESI) (M+H)+ m/z calculated for C28H35N4O 443.2805, measured: 443.2812.

### Example 18:

### N-((3S,10R,13S)-17-(4-ethyl-1H-imidazol-1-yl)-10,13-dimethyl-2,3,4,7,8,9,10,11,12,13,14,15-dodecahydro-1H-cyclopenta[a]phenanthren-3-yl)isonicotinamide (Compound 18)

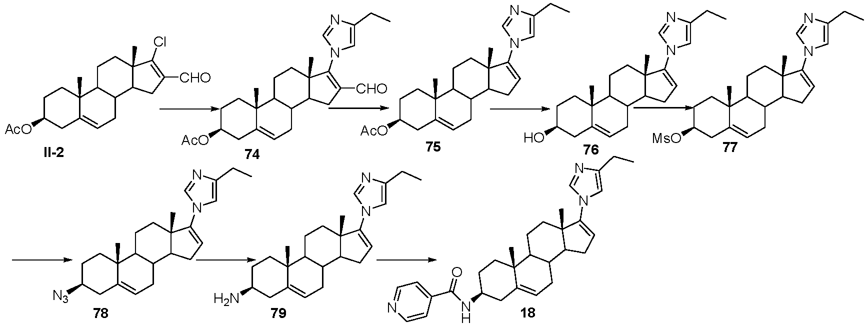

### (3S,10R,13S)-16-formyl-17-(4-ethyl-1H-imidazol-1-yl)-10,13-dimethyl-2,3,4,7,8,9,10,11,12,13,14,15-dodecahydro-1H-cyclopenta[a]phenanthren-3-yl acetate (74)

Compound II-2 (4 g, 10.61 mmol) and potassium carbonate (2.15 g, 15.59 mmol) were added to a flask, followed by the addition of anhydrous N,N-dimethylformamide as solvent. The mixture was stirred at room temperature for half an hour, then 4-ethylimidazole (1 g, 10.4 mmol) was added to the system. The temperature was slowly raised to 80°C and stirred overnight. The reaction was monitored by TLC until completion. After the reaction, the mixture was extracted with ethyl acetate, and the organic phase was collected and washed twice with saturated brine. The organic layer was dried over anhydrous sodium sulfate, filtered, and then silica gel was added to the system for concentration. The product was purified by automated column chromatography (30-50% ethyl acetate/petroleum ether) to obtain the desired product. Finally, 3.386 g of coffee-colored solid compound 74 was obtained with a yield of 73%. ¹H NMR (400 MHz, DMSO) δ 9.77 (s, 1H), 7.54 (s, 1H), 6.81 (s, 1H), 5.42 (s, 1H), 4.66 - 4.55 (m, 1H), 2.63 (q, J = 7.5 Hz, 2H), 2.04 (s, 3H), 1.26 (t, J = 7.5 Hz, 3H), 1.01 (s, 3H), 0.88 (s, 3H).

### (3S,10R,13S)-17-(4-ethyl-1H-imidazol-1-yl)-10,13-dimethyl-2,3,4,7,8,9,10,11,12,13,14,15-dodecahydro-1H-cyclopenta[a]phenanthren-3-yl acetate (75)

Compound 74 (3.386 g, 5.56 mmol) was dissolved in anhydrous N,N-dimethylformamide and stirred at room temperature. Then 1.5 g of palladium on carbon was added, and the system was purged with argon three times. The temperature was slowly raised to 160°C and reacted for 36 hours. The reaction was monitored by TLC until completion. After the reaction, the system was cooled to room temperature, and a certain amount of ethyl acetate was added. After stirring for a period of time, the mixture was filtered through diatomaceous earth, and the filtrate was collected. The filtrate was then extracted with ethyl acetate and water. The organic phase was collected, washed twice with saturated brine, dried over anhydrous sodium sulfate, and filtered. Silica gel was added to the system for concentration, and the product was purified by automated column chromatography (1-4% methanol/dichloromethane) to obtain the desired product. Finally, 1.264 g of yellow solid compound 75 was obtained with a yield of 39.9%. ¹H NMR (400 MHz, CDCl₃) δ 7.54 (d, J = 1.2 Hz, 1H), 6.76 (d, J = 0.9 Hz, 1H), 5.41 (d, J = 3.8 Hz, 1H), 4.67 - 4.56 (m, 1H), 2.63 (q, J = 7.5 Hz, 2H), 2.05 (s, 3H), 1.26 (t, J = 7.5 Hz, 3H), 1.01 (s, 3H), 0.88 (s, 3H).

### (3S,10R,13S)-17-(4-ethyl-1H-imidazol-1-yl)-10,13-dimethyl-2,3,4,7,8,9,10,11,12,13,14,15-dodecahydro-1H-cyclopenta[a]phenanthren-3 -ol (76)

Compound 75 (1.26 g, 3.08 mmol) was dissolved in anhydrous methanol and stirred at room temperature. Then, pre-weighed potassium hydroxide (0.52 g, 9.27 mmol) was added. After addition, the mixture was stirred at room temperature for 4 h, and the reaction completion was monitored by TLC. After the reaction was complete, part of the solvent was removed by rotary evaporation. The mixture was then extracted with ethyl acetate and water. The organic phase was collected, washed twice with saturated brine, dried over anhydrous sodium sulfate, and filtered. Silica gel was added to the system, concentrated, and purified by automated column chromatography (1-3% methanol/dichloromethane) to obtain the desired product. Finally, 537 mg of yellow solid compound 76 was obtained with a yield of 47.5%. ¹H NMR (400 MHz, CDCl₃) δ 7.56 (d, J = 0.8 Hz, 1H), 6.78 (s, 1H), 5.64 (dd, J = 3.0, 1.7 Hz, 1H), 5.41 (d, J = 5.2 Hz, 1H), 3.56 (dt, J = 15.5, 5.4 Hz, 1H), 2.63 (q, J = 7.5 Hz, 2H), 1.26 (t, J = 7.5 Hz, 3H), 1.08 (s, 3H), 1.02 (s, 3H).

### (3S,10R,13S)-17-(4-Ethyl-1H-imidazol-1-yl)-10,13-dimethyl-2,3,4,7,8,9,10,11,12,13,14,15-dodecahydro-1H-cyclopenta[a]phenanthren-3-yl methanesulfonate (77)

Compound 76 (537 mg, 1.47 mmol) and 4-dimethylaminopyridine (18 mg, 0.15 mmol) were placed in a flask. Super-dry dichloromethane was added as a solvent and stirred in an ice bath. Triethylamine (0.6 mL, 4.41 mmol) was then added and stirred for a period of time. Finally, methanesulfonyl chloride (0.24 mL, 2.94 mmol) was slowly added. After the addition was complete, the mixture was stirred at room temperature for 5 h, and the reaction completion was monitored by TLC. Ice water was then added for quenching, followed by extraction with dichloromethane. The organic phase was washed twice with saturated brine, dried over anhydrous sodium sulfate, and filtered. Silica gel was added to the system, concentrated, and purified by automated column chromatography (40-60% ethyl acetate/petroleum ether) to obtain the desired product. Finally, 489 mg of yellow solid compound 77 was obtained with a yield of 75.1%. ¹H NMR (400 MHz, CDCl₃) δ 7.59 (s, 1H), 6.77 (s, 1H), 5.67 - 5.64 (m, 1H), 5.48 (d, J = 5.1 Hz, 1H), 4.59 - 4.50 (m, 1H), 3.04 (s, 3H), 2.63 (q, J = 7.5 Hz, 2H), 1.26 (t, J = 7.5 Hz, 3H), 1.09 (s, 3H), 1.02 (s, 3H).

### 1-((3S,10R,13S)-3-Azido-10,13-dimethyl-2,3,4,7,8,9,10,11,12,13,14,15-dodecahydro-1H-cyclopenta[a]phenanthren-17-yl)-4-ethyl-1H-imidazole (78)

Compound 77 (900 mg, 2.02 mmol) was dissolved in super-dry dichloromethane and stirred at room temperature. Azidotrimethylsilane (0.8 mL, 6.06 mmol) and boron trifluoride etherate (1 mL, 8.08 mmol) were added sequentially using a plastic syringe. After the addition was complete, the mixture was stirred at room temperature for 5 hours, and the reaction completion was monitored by TLC. Saturated sodium bicarbonate solution was then added for quenching, followed by extraction with dichloromethane. The organic phase was washed twice with saturated brine, dried over anhydrous sodium sulfate, filtered, and silica gel was added to the organic phase. The mixture was concentrated and purified by automated column chromatography (20-40% ethyl acetate/petroleum ether) to obtain the desired product. Finally, 156 mg of white solid 78 was obtained with a yield of 19.7%. ¹H NMR (400 MHz, CDCl₃) δ 8.09 (d, J = 1.3 Hz, 1H), 6.84 (s, 1H), 5.94 (dd, J = 3.1, 1.7 Hz, 1H), 5.44 (d, J = 5.0 Hz, 1H), 3.25 (ddd, J = 15.9, 11.6, 4.1 Hz, 1H), 2.87 (q, J = 7.5 Hz, 2H), 1.29 (t, J = 7.5 Hz, 3H), 1.08 (s, 3H), 1.04 (s, 3H).

### (3S,10R,13S)-17-(4-Ethyl-1H-imidazol-1-yl)-10,13-dimethyl-2,3,4,7,8,9,10,11,12,13,14,15-dodecahydro-1H-cyclopenta[a]phenanthren-3-atnine (79)

Compound 78 (150 mg, 0.383 mmol) was dissolved in 8 mL of tetrahydrofuran and 3 mL of water and stirred at room temperature. Pre-weighed triphenylphosphine (390 mg, 1.49 mmol) was then added. The temperature was slowly raised to 60°C and stirred overnight at this temperature. The reaction completion was monitored by TLC. Part of the solvent was then removed by rotary evaporation, and 20 mL of dichloromethane was added to dissolve the mixture. 2 M hydrochloric acid was slowly added to adjust the pH of the solution to about 2. The mixture was separated, and the aqueous phase was collected. 2 M sodium hydroxide solution was then added to adjust the pH to slightly alkaline, at which point a large amount of white solid precipitated. The aqueous phase was extracted three times with a small amount of dichloromethane. The organic phases were collected, combined, dried over anhydrous sodium sulfate, and the solvent was removed by rotary evaporation to obtain 75 mg of white solid 79 with a yield of 53.57%. ¹H NMR (400 MHz, CDCl₃) δ 7.51 (s, 1H), 6.73 (s, 1H), 5.58 (s, 1H), 5.31 (d, J = 5.0 Hz, 1H), 2.57 (q, J = 7.4 Hz, 2H), 1.21 (t, J = 7.5 Hz, 3H), 1.01 (s, 3H), 0.97 (s, 3H).

### N-((3S,10R,13S)-17-(4-Ethyl-1H-imidazol-1-yl)-10,13-dimethyl-2,3,4,7,8,9,10,11,12,13,14,15-dodecahydro-1H-cyclopenta[a]phenanthren-3-yl)isonicotinamide (Compound 18)

Compound 18 was obtained as 57 mg of white solid with a yield of 59.07% from compound 79 (75 mg, 0.205 mmol). ¹H NMR (600 MHz, DMSO) δ 8.68 (dd, J = 4.5, 1.4 Hz, 2H), 8.54 (d, J = 8.0 Hz, 1H), 7.71 (dd, J = 4.5, 1.5 Hz, 2H), 7.67 (s, 1H), 7.00 (s, 1H), 5.71 (s, 1H), 5.35 (d, J = 4.8 Hz, 1H), 3.73 - 3.65 (m, 1H), 2.44 (dd, J = 15.0, 7.5 Hz, 3H), 1.12 (t, J = 7.5 Hz, 2H), 1.02 (s, 3H), 0.95 (s, 3H). 13C NMR (151 MHz, DMSO) δ 164.18, 150.62, 148.49, 144.30, 142.07, 141.59, 135.19, 121.72, 121.04, 116.48, 113.77, 56.31, 50.26, 50.10, 45.86, 38.68, 37.95, 36.85, 34.56, 30.90, 30.14, 29.62, 28.36, 21.55, 20.69, 19.40, 16.13, 13.99. HRMS (ESI) (M+H)+ m/z calculated for C30H39N4O 471.3118, found: 471.3125.

### Example 19:

### N-((3S,10R,13S)-17-(4-ethyl-1H-imidazol-1-yl)-10,13-dimethyl-2,3,4,7,8,9,10,11,12,13,14,15-dodecahydro-1H-cyclopenta[a]phenanthren-3-yl)pyrazine-4-carboxamide (Compound 19)

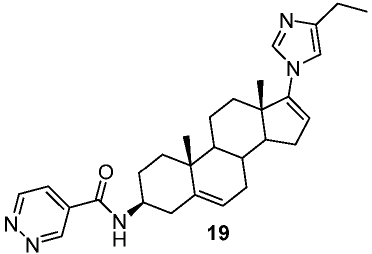

Compound 19 was obtained as 10 mg of white solid with a yield of 24.3% from compound 79 (32 mg, 0.08 mmol). ¹H NMR (500 MHz, DMSO) δ 9.56 (s, 1H), 9.43 (d, J = 5.1 Hz, 1H), 8.95 (s, 1H), 8.79 (s, 1H), 8.04 (s, 1H), 7.50 (s, 1H), 6.11 (s, 1H), 5.40 (s, 1H), 3.78 - 3.70 (m, 1H), 2.62 (q, J = 7.5 Hz, 2H), 1.22 (t, J = 7.6 Hz, 3H), 1.07 (s, 3H), 1.02 (s, 3H). 13CNMR (126 MHz, DMSO) δ 162.57, 152.59, 149.25, 147.01, 141.43, 138.76, 134.10, 132.08, 124.62, 122.40, 121.06, 116.21, 56.33, 50.32, 50.18, 45.96, 38.57, 37.92, 36.84, 33.82, 30.83, 30.01, 29.82, 28.26, 20.38, 19.34, 19.02, 15.80, 13.24. HRMS (ESI) (M+H)+ m/z calculated for C29H38N5O 472.3071, found: 472.3066.

### Example 20:

### N-((3S,10R,13S)-17-(4-isopropyl-1H-imidazol-1-yl)-10,13-dimethyl-2,3,4,7,8,9,10,11,12,13,14,15-dodecahydro-1H-cyclopenta[a]phenanthren-3-yl)isonicotinamide (Compound 20)

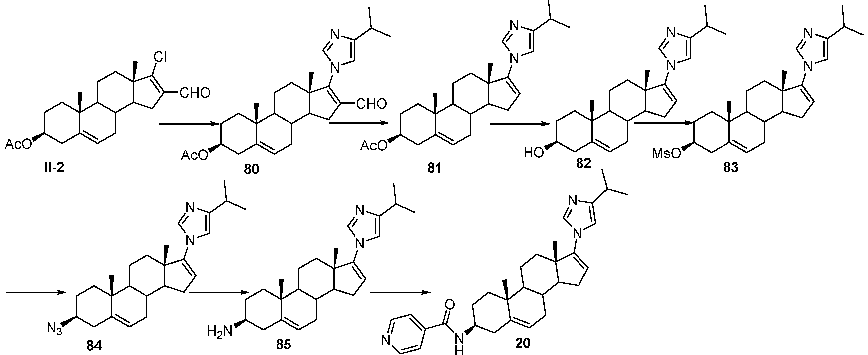

### (3S,1R,13S)-16-formyl-17-(4-isopropyl-1H-imidazol-1-yl)-10,13-dimethyl-2,3,4,7,8,9,10,11,12,13,14,15-dodecahydro-1H-cyclopenta[a]phenanthren-3-yl acetate (80)

Compound II-2 (7 g, 18.57 mmol) and potassium carbonate (3.8 g, 27.55 mmol) were added to a flask. Anhydrous N,N-dimethylformamide was added as solvent and the mixture was stirred at room temperature for half an hour. Then 5-isopropylimidazole (1.98 g, 17.97 mmol) was added to the system. The temperature was slowly raised to 80°C and stirred overnight. The reaction was monitored by TLC until completion. After the reaction was complete, the mixture was extracted with ethyl acetate. The organic phase was collected, washed twice with saturated brine, dried over anhydrous sodium sulfate, filtered, and then silica gel was added to the system. The mixture was concentrated and purified by automated column chromatography (30-50% ethyl acetate/petroleum ether) to obtain the desired product. Finally, 2.28 g of brown solid compound 80 was obtained with a yield of 27.3%. ¹H NMR (400 MHz, CDCl₃) δ 9.76 (s, 1H), 7.54 (s, 1H), 6.78 (s, 1H), 5.42 (d, J = 5.0 Hz, 1H), 4.60 - 4.54 (m, 1H), 2.96 - 2.80 (m, 2H), 2.04 (s, 3H), 1.26 (t, J = 6.1 Hz, 6H), 1.08 (s, 3H), 0.96 (s, 3H).

### (3S,10R,13S)-17-(4-isopropyl-1H-imidazol-1-yl)-10,13-dimethyl-2,3,4,7,8,9,10,11,12,13,14,15-dodecahydro-1H-cyclopenta[a]phenanthren-3-yl acetate (81)

Compound 80 (2.28 g, 5.06 mmol) was dissolved in anhydrous N,N-dimethylformamide and stirred at room temperature. Then 1 g of palladium on carbon was added. The system was purged with argon three times, and the temperature was slowly raised to 160°C. The reaction was carried out for 24 hours and monitored by TLC until completion. After the reaction was complete, the reaction system was cooled to room temperature, and a certain amount of ethyl acetate was added. After stirring for a period of time, the mixture was filtered through diatomaceous earth. The filtrate was collected and extracted with ethyl acetate and water. The organic phase was collected, washed twice with saturated brine, dried over anhydrous sodium sulfate, filtered, and then silica gel was added to the system. The mixture was concentrated and purified by automated column chromatography (20-30% ethyl acetate/petroleum ether) to obtain the desired product. Finally, 1.13 g of yellow solid compound 81 was obtained with a yield of 52.8%. ¹H NMR (400 MHz, CDCl₃) δ 7.75 (d, J = 0.8 Hz, 1H), 6.96 (s, 1H), 5.43 (d, J = 5.0 Hz, 1H), 4.65 - 4.54 (m, 1H), 2.96 - 2.80 (m, 2H), 2.04 (s, 3H), 1.26 (t, J = 6.1 Hz, 6H), 1.08 (s, 3H), 0.96 (s, 3H).

### (3S,10R,13S)-17-(4-isopropyl-1H-imidazol-1-yl)-10,13-dimethyl-2,3,4,7,8,9,10,11,12,13,14,15-dodecahydro-1H-cyclopenta[a]phenanthren-3-ol (82)

Compound 81 (1 g, 2.37 mmol) was dissolved in anhydrous methanol and stirred at room temperature. Pre-weighed potassium hydroxide (0.45 g, 8.02 mmol) was then added. After addition was complete, the mixture was stirred at room temperature for 4 h, with TLC monitoring until the reaction was complete. Upon completion, the solvent was partially removed by rotary evaporation. The mixture was then extracted with ethyl acetate and water. The organic phase was collected, washed twice with saturated brine, dried over anhydrous sodium sulfate, and filtered. Silica gel was added to the system, concentrated, and purified by automated column chromatography (1-2% methanol/dichloromethane) to obtain the desired product. Finally, 426 mg of white solid compound 82 was obtained with a yield of 47.3%. ¹H NMR (400 MHz, CDCl₃) δ 7.57 (d, J = 1.2 Hz, 1H), 6.75 (s, 1H), 5.64 (dd, J = 3.1, 1.7 Hz, 1H), 5.42 - 5.39 (m, 1H), 3.60 - 3.51 (m, 1H), 2.91 (dt, J = 13.7, 6.9 Hz, 1H), 1.28 (d, J = 6.9 Hz, 6H), 1.08 (s, 3H), 1.03 (s, 3H).

### (3S,10R,3S)-17-(4-isopropyl-1H-imidazol-1-yl)-10,13-dimethyl-2,3,4,7,8,9,10,11,12,13,14,15-dodecahydro-1H-cyclopenta[a]phenanthren-3-yl methanesulfonate (83)

Compound 82 (426 mg, 1.12 mmol) and 4-dimethylaminopyridine (14 mg, 0.11 mmol) were placed in a flask. Super-dry dichloromethane was added as a solvent and stirred in an ice bath. Triethylamine (0.47 mL, 3.45 mmol) was then added and stirred for a period of time. Finally, methanesulfonyl chloride (0.18 mL, 2.21 mmol) was slowly added. After the addition was complete, the mixture was stirred at room temperature for 5 h, with TLC monitoring until the reaction was complete. Ice water was then added to quench the reaction, followed by extraction with dichloromethane. The organic phase was washed twice with saturated brine, dried over anhydrous sodium sulfate, and filtered. Silica gel was added to the system, concentrated, and purified by automated column chromatography (40-60% ethyl acetate/petroleum ether) to obtain the desired product. Finally, 490 mg of white solid compound 83 was obtained with a yield of 95.5%. ¹H NMR (400 MHz, DMSO) δ 7.56 (s, 1H), 6.74 (s, 1H), 5.63 (s, 1H), 5.46 (s, 1H), 4.59 - 4.49 (m, 1H), 3.02 (s, 3H), 2.90 (dt, J = 13.7, 6.9 Hz, 1H), 1.27 (d, J = 6.9 Hz, 6H), 1.08 (s, 3H), 1.01 (s, 3H).

### 1-((3S,10R,13S)-3-azido-10,13-dimethyl-2,3,4,7,8,9,10,11,12,13,14,15-dodecahydro-1H-cyclopenta[a]phenanthren-17-yl)-4-isopropyl-1H-imidazole (84)

Compound 83 (490 mg, 1.07 mmol) was dissolved in super-dry dichloromethane and stirred at room temperature. Azidotrimethylsilane (0.45 mL, 3.4 mmol) and boron trifluoride etherate (0.53 mL, 4.28 mmol) were added sequentially using a plastic syringe. After addition was complete, the mixture was stirred at room temperature overnight, with TLC monitoring until the reaction was complete. Saturated sodium bicarbonate solution was then added to quench the reaction, followed by extraction with dichloromethane. The organic phase was washed twice with saturated brine, dried over anhydrous sodium sulfate, filtered, and silica gel was added to the organic phase. The mixture was concentrated and purified by automated column chromatography (1-3% methanol/dichloromethane) to obtain the desired product. Finally, 151 mg of white solid 84 was obtained with a yield of 34.87%. ¹H NMR (400 MHz, CDCl₃) δ 7.65 (s, 1H), 6.74 (s, 1H), 5.67 - 5.65 (m, 1H), 5.41 (d, J = 4.9 Hz, 1H), 3.21 (ddd, J = 10.2, 8.7, 4.7 Hz, 1H), 2.88 (dq, J = 13.8, 6.9 Hz, 1H), 1.25 (d, J = 6.9 Hz, 6H), 1.04 (s, 3H), 1.00 (s, 3H).

### (3S,10R,13S)-17-(4-isopropyl-1H-imidazol-1-yl)-10,13-dimethyl-2,3,4,7,8,9,10,11,12,13,14,15-dodecahydro-1H-cyclopenta[a]phenanthren-3-amine (85)

Compound 84 (151 mg, 0.372 mmol) was dissolved in 6 mL of tetrahydrofuran and 4 mL of water and stirred at room temperature. Pre-weighed triphenylphosphine (390 mg, 1.49 mmol) was then added. The temperature was slowly raised to 60°C and stirred overnight at this temperature, with TLC monitoring until the reaction was complete. The solvent was partially removed by rotary evaporation, and 20 mL of dichloromethane was added to dissolve the mixture. 2 M hydrochloric acid was slowly added to adjust the pH of the solution to around 2. The mixture was separated, and the aqueous phase was collected. 2 M sodium hydroxide solution was then added to adjust the pH to slightly alkaline, at which point a large amount of white solid precipitated. The aqueous phase was extracted three times with a small amount of dichloromethane. The organic phases were collected, combined, dried over anhydrous sodium sulfate, and the solvent was removed by rotary evaporation to obtain 100 mg of white solid 85, with a yield of 70.77%. ¹H NMR (400 MHz, CDCl₃) δ 7.54 (s, 1H), 6.72 (s, 1H), 5.60 (s, 1H), 5.34 (d, J = 5.0 Hz, 1H), 2.86 (dq, J = 13.6, 6.8 Hz, 1H), 1.24 (d, J = 6.9 Hz, 6H), 1.03 (s, 3H), 0.99 (s, 3H).

### N-((3S,10R,13S)-17-(4-isopropyl-1H-imidazol-1-yl)-10,13-dimethyl-2,3,4,7,8,9,10,11,12,13,14,15-dodecahydro-1H-cyclopenta[a]phenanthren-3-yl)isonicotinamide (Compound 20)

Compound 85 (100 mg, 0.264 mmol) was used to obtain 63 mg of white solid compound 20, with a yield of 49.2%. ¹H NMR (500 MHz, DMSO) δ 9.28 (d, J = 1.5 Hz, 1H), 8.89 (d, J = 8.0 Hz, 1H), 8.84 (d, J = 5.7 Hz, 2H), 8.00 (d, J = 6.2 Hz, 2H), 7.68 (s, 1H), 6.30 - 6.27 (m, 1H), 5.39 (d, J = 5.0 Hz, 1H), 3.75 (qd, J = 12.4, 5.0 Hz, 1H), 3.03 (dq, J = 14.0, 7.0 Hz, 1H), 1.29 (d, J = 6.9 Hz, 6H), 1.07 (s, 3H), 1.04 (s, 3H). 13C NMR (126 MHz, DMSO) δ 163.32, 158.66, 148.22, 146.41, 144.36, 141.55, 141.00, 133.74, 124.80, 122.97, 120.92, 116.31, 56.36, 50.34, 50.16, 45.99, 38.56, 37.91, 36.85, 33.45, 30.80, 30.04, 29.89, 28.24, 25.20, 21.77, 20.41, 19.37, 15.63. HRMS (ESI) (M+H)+ m/z calculated for C31H41N4O 485.3275, found: 485.3279.

### Example 21:

### N-((3S,10R,13S)-17-(4-nitro-1H-imidazol-1-yl)-10,13-dimethyl-2,3,4,7,8,9,10,11,12,13,14,15-dodecahydro-1H-cyclopenta[a]phenanthren-3-yl)pyrazine-4-carboxamide (Compound 21)

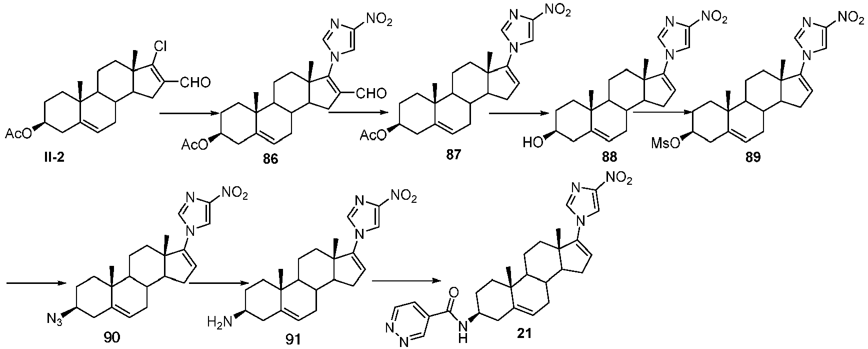

### (3S,10R,13S)-16-formyl-17-(4-nitro-1H-imidazol-1-yl)-10,13-dimethyl-2,3,4,7,8,9,10,11,12,13,14,15-dodecahydro-1H-cyclopenta[a]phenanthren-3-yl acetate (86)

Compound II-2 (10 g, 26.53 mmol) and potassium carbonate (5.49 g, 39.8 mmol) were added to a flask, followed by the addition of anhydrous N,N-dimethylformamide as solvent. After stirring at room temperature for half an hour, 4-nitroimidazole (3 g, 26.53 mmol) was added to the system. The temperature was slowly raised to 80°C and stirred overnight at this temperature. The reaction was monitored by TLC until completion. After the reaction was complete, the mixture was extracted with ethyl acetate. The organic phase was collected, washed twice with saturated brine, dried over anhydrous sodium sulfate, filtered, and then concentrated with silica gel. The product was purified by automated column chromatography (30-50% ethyl acetate/petroleum ether) to obtain the desired product. Finally, 7 g of grayish-green solid compound 86 was obtained with a yield of 58.3%. ¹H NMR (400 MHz, CDCl₃) δ 9.77 (s, 1H), 7.93 (d, J = 1.5 Hz, 1H), 7.59 (d, J = 1.5 Hz, 1H), 5.44 (d, J = 5.1 Hz, 1H), 4.63 (ddd, J = 16.0, 11.1, 5.1 Hz, 1H), 2.77 (dd, J = 15.7, 6.0 Hz, 1H), 2.06 (s, 3H), 1.13 (s, 3H), 1.10 (s, 3H).

### (3S,10R,13S)-17-(4-nitro-1H-imidazol-1-yl)-10,13-dimethyl-2,3,4,7,8,9,10,11,12,13,14,15-dodecahydro-1H-cyclopenta[a]phenanthren-3-yl acetate (87)

Compound 86 (7 g, 15.44 mmol) was dissolved in anhydrous N,N-dimethylformamide and stirred at room temperature. Then, 3.5 g of palladium on carbon was added, and the system was purged with argon three times. The temperature of the system was slowly raised to 160°C and reacted for 39 hours. The reaction was monitored by TLC until completion. After the reaction was complete, the reaction system was cooled to room temperature, and a certain amount of ethyl acetate was added. After stirring for a period of time, the mixture was filtered through diatomaceous earth, and the filtrate was collected. The filtrate was then extracted with ethyl acetate and water. The organic phase was collected, washed twice with saturated brine, dried over anhydrous sodium sulfate, filtered, and then concentrated with silica gel. The product was purified by automated column chromatography (20-30% ethyl acetate/petroleum ether) to obtain the desired product. Finally, 1.07 g of yellow solid compound 87 was obtained with a yield of 16.3%. ¹H NMR (400 MHz, CDCl₃) δ 7.87 (d, J = 1.4 Hz, 1H), 7.56 (d, J = 1.3 Hz, 1H), 5.96 - 5.92 (m, 1H), 5.43 (d, J = 5.1 Hz, 1H), 4.68 - 4.57 (m, 1H), 2.06 (s, 3H), 1.09 (s, 3H), 1.05 (s, 3H).

### (3S,10R,13S)-17-(4-nitro-1H-imidazol-1-yl)-10,13-dimethyl-2,3,4,7,8,9,10,11,12,13,14,15-dodecahydro-1H-cyclopenta[a]phenanthren-3-ol (88)

Compound 87 (1.07 g, 2.52 mmol) was dissolved in anhydrous methanol and stirred at room temperature. Then, pre-weighed potassium hydroxide (0.45 g, 8.02 mmol) was added. After addition, the mixture was stirred at room temperature for 4 h. The reaction was monitored by TLC until completion. After the reaction was complete, part of the solvent was removed by rotary evaporation, and then the mixture was extracted with ethyl acetate and water. The organic phase was collected, washed twice with saturated brine, dried over anhydrous sodium sulfate, filtered, and then concentrated with silica gel. The product was purified by automated column chromatography (1-2% methanol/dichloromethane) to obtain the desired product. Finally, 910 mg of white solid compound 88 was obtained with a yield of 94.3%. ¹H NMR (400 MHz, CDCl₃) δ 7.87 (d, J = 1.5 Hz, 1H), 7.56 (d, J = 1.4 Hz, 1H), 5.95 (dd, J = 3.0, 1.6 Hz, 1H), 5.40 (d, J = 5.1 Hz, 1H), 3.61 - 3.51 (m, 1H), 1.08 (s, 3H), 1.05 (s, 3H).

### (3S,10R,13S)-17-(4-nitro-1H-imidazol-1-yl)-10,13-dimethyl-2,3,4,7,8,9,10,11,12,13,14,15-dodecahydro-1H-cyclopenta[a]phenanthren-3-yl methanesulfonate (89)

Compound 88 (910 mg, 2.375 mmol) and 4-dimethylaminopyridine (30 mg, 0.245 mmol) were added to a flask, followed by addition of anhydrous dichloromethane as solvent. The mixture was stirred in an ice bath, then triethylamine (1 mL, 7.34 mmol) was added and stirred for a period of time. Methanesulfonyl chloride (0.4 mL, 4.91 mmol) was slowly added dropwise. After completion of addition, the reaction was stirred at room temperature for 5 h and monitored by TLC until completion. Ice water was then added to quench the reaction, followed by extraction with dichloromethane. The organic phase was washed twice with saturated brine, dried over anhydrous sodium sulfate, filtered, and concentrated by rotary evaporation to obtain the desired product. Finally, 1.02 g of yellow-green solid compound 89 was obtained in 93.1% yield. ¹H NMR (400 MHz, CDCl₃) δ 7.87 (d, J = 1.5 Hz, 1H), 7.56 (d, J = 1.5 Hz, 1H), 5.95 (dd, J = 3.1, 1.7 Hz, 1H), 5.48 (d, J = 4.8 Hz, 1H), 4.60 - 4.51 (m, 1H), 3.04 (s, 3H), 1.10 (s, 3H), 1.05 (s, 3H).

### 1-((3S,10R,13S)-3-azido-10,13-dimethyl-2,3,4,7,8,9,10,11,12,13,14,15-dodecahydro-1H-cyclopenta[a]phenanthren-17-yl)-4-nitro-1H-imidazole (90)

Compound 89 (1.02 g, 2.21 mmol) was dissolved in anhydrous dichloromethane and stirred at room temperature. Azidotrimethylsilane (0.88 mL, 6.65 mmol) and boron trifluoride etherate (1.1 mL, 8.88 mmol) were added sequentially using a plastic syringe. After completion of addition, the reaction was stirred at room temperature overnight and monitored by TLC until completion. Saturated sodium bicarbonate solution was added to quench the reaction, followed by extraction with dichloromethane. The organic phase was washed twice with saturated brine, dried over anhydrous sodium sulfate, filtered, and purified by automated column chromatography (1-3% methanol/dichloromethane) to obtain the desired product. Finally, 714 mg of yellow solid 90 was obtained in 78.63% yield. ¹H NMR (400 MHz, CDCl₃) δ 7.87 (d, J = 1.5 Hz, 1H), 7.56 (d, J = 1.4 Hz, 1H), 5.95 (dd, J = 3.2, 1.7 Hz, 1H), 5.45 (d, J = 5.1 Hz, 1H), 3.30 - 3.20 (m, 1H), 1.08 (s, 3H), 1.05 (s, 3H).

### (3S,10R,13S)-17-(4-nitro-1H-imidazol-1-yl)-10,13-dimethyl-2,3,4,7,8,9,10,11,12,13,14,15-dodecahydro-1H-cyclopenta[a]phenanthren-3-amine (91)

Compound 90 (713 mg, 1.75 mmol) was dissolved in 20 mL of tetrahydrofuran and 10 mL of water, and stirred at room temperature. Triphenylphosphine (1.8 g, 6.86 mmol) was added, and the temperature was slowly raised to 60°C. The reaction was stirred overnight at this temperature and monitored by TLC until completion. The solvent was partially removed by rotary evaporation, and 20 mL of dichloromethane was added to dissolve the residue. 2 M hydrochloric acid was slowly added to adjust the pH to about 2. The layers were separated and the aqueous phase was collected. 2 M sodium hydroxide solution was then added to adjust the pH to slightly basic, resulting in precipitation of a large amount of white solid. The aqueous phase was extracted three times with small amounts of dichloromethane. The combined organic phases were dried over anhydrous sodium sulfate and concentrated by rotary evaporation to give 75 mg of white solid 91 in 11.2% yield, which was used directly in the next step.

### N-((3S,10R,13S)-17-(4-nitro-1H-imidazol-1-yl)-10,13-dimethyl-2,3,4,7,8,9,10,11,12,13,14,15-dodecahydro-1H-cyclopenta[a]phenanthren-3-yl)pyrazine-4-carboxamide (Compound 21)

Compound 91 (75 mg, 0.196 mmol) was used to obtain 68 mg of white solid compound 21 in 70.8% yield. ¹H NMR (500 MHz, DMSO) δ 9.54 (dd, J = 2.1, 1.3 Hz, 1H), 9.43 (dd, J= 5.3, 1.1 Hz, 1H), 8.82 (d, J = 7.9 Hz, 1H), 8.50 (d, J = 1.4 Hz, 1H), 8.06 (d, J = 1.4 Hz, 1H), 7.99 (dd, J = 5.3, 2.3 Hz, 1H), 6.17 (d, J = 1.2 Hz, 1H), 5.39 (d, J = 4.8 Hz, 1H), 3.79 - 3.68 (m, 1H), 1.06 (s, 3H), 1.01 (s, 3H). 13C NMR (126 MHz, DMSO) δ 162.69, 159.92, 152.51, 149.26, 148.07, 146.98, 141.37, 136.23, 132.03, 124.59, 123.21, 121.03, 120.00, 56.22, 50.32, 50.15, 46.07, 38.59, 37.87, 36.83, 33.78, 30.84, 30.08, 29.91, 28.37, 20.49, 19.38, 15.78. HRMS (ESI) (M+H)+ m/z calcd for C27H33N6O₃ 489.2609, found: 489.2603.

### Example 22:

### N-((3S,10R,13S)-17-(4-(trifluoromethyl)-1H-imidazol-1-yl)-10,13-dimethyl-2,3,4,7, 8,9,10,11,12,13,14,15-dodecahydro-1H-cyclopenta[a]phenanthren-3-yl)pyrazine-4-carboxamide (Compound 22)

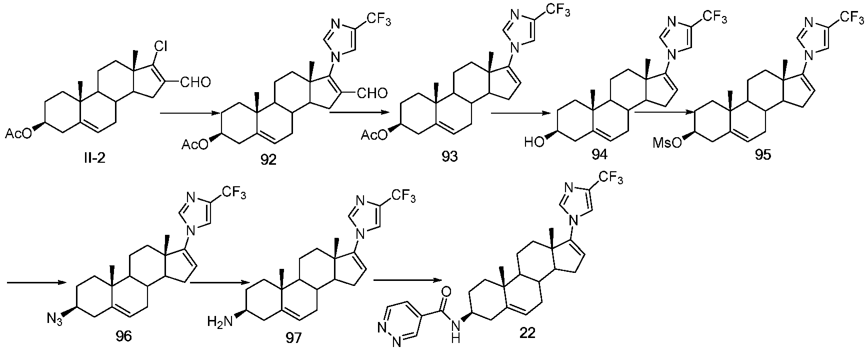

### (3S,10R,13S)-16-formyl-17-(4-(trifluoromethyl)-1H-imidazol-1-yl)-10,13-dimethyl-2,3,4,7,8,9,10,11,12,13,14,15-dodecahydro-1H-cyclopenta[a]phenanthren-3-yl acetate (92)

Compound II-2 (3.6 g, 9.55 mmol) and potassium carbonate (3.96 g, 28.7 mmol) were added to a flask, followed by the addition of anhydrous N,N-dimethylformamide as solvent. The mixture was stirred at room temperature for half an hour, then 4-trifluoromethylimidazole (1.56 g, 11.46 mmol) was added to the system. The temperature was slowly raised to 80°C and stirred overnight. The reaction was monitored by TLC until completion. After the reaction was complete, the mixture was extracted with ethyl acetate. The organic phase was collected, washed twice with saturated brine, dried over anhydrous sodium sulfate, filtered, and then concentrated with silica gel. The crude product was purified by automated column chromatography (20-40% ethyl acetate/petroleum ether) to obtain the desired product. Finally, 2.2 g of yellow solid compound 92 was obtained with a yield of 48.4%. ¹H NMR (400 MHz, CDCl₃) δ 9.75 (s, 1H), 7.67 (s, 1H), 7.45 (s, 1H), 5.43 (d, J = 5.1 Hz, 1H), 4.63 (dq, J = 15.9, 5.2 Hz, 1H), 2.73 (dd, J = 15.5, 6.1 Hz, 1H), 2.06 (s, 3H), 1.10 (s, 3H), 1.09 (s, 3H).

### (3S,10R,13S)-17-(4-Trifluoromethyl-1H-imidazol-1-yl)-10,13-dimethyl-2,3,4,7,8,9,10,11,12,13,14,15-dodecahydro-1H-cyclopenta[a]phenanthren-3-yl acetate (93)

Compound 92 (2.2 g, 4.62 mmol) was dissolved in anhydrous N,N-dimethylformamide and stirred at room temperature. Then 2 g of palladium on carbon was added, and the system was purged with argon three times. The temperature was slowly raised to 160°C and reacted for 38 hours. The reaction was monitored by TLC until completion. After the reaction was complete, the reaction system was cooled to room temperature, and a certain amount of ethyl acetate was added. After stirring for a period of time, the mixture was filtered through diatomaceous earth, and the filtrate was collected. The filtrate was then extracted with ethyl acetate and water. The organic phase was collected, washed twice with saturated brine, dried over anhydrous sodium sulfate, filtered, and then concentrated with silica gel. The crude product was purified by automated column chromatography (10-40% ethyl acetate/petroleum ether) to obtain the desired product. Finally, 725 mg of white solid compound 93 was obtained with a yield of 35.0%. ¹H NMR (400 MHz, CDCl₃) δ 7.64 (s, 1H), 7.36 (s, 1H), 5.80 (s, 1H), 5.41 (d, J = 4.8 Hz, 1H), 4.66 - 4.56 (m, 1H), 2.04 (s, 3H), 1.07 (s, 3H), 1.00 (s, 3H).

### (3S,10R,13S)-17-(4-Trifluoromethyl-1H-imidazol-1-yl)-10,13-dimethyl-2,3,4,7,8,9,10,11,12,13,14,15-dodecahydro-1H-cyclopenta[a]phenanthren-3 -ol (94)

Compound 93 (720 mg, 1.61 mmol) was dissolved in anhydrous methanol and stirred at room temperature. Then potassium hydroxide (0.36 g, 6.02 mmol) was added. After addition, the mixture was stirred at room temperature for 4 h. The reaction was monitored by TLC until completion. After the reaction was complete, part of the solvent was removed by rotary evaporation, and then the mixture was extracted with ethyl acetate and water. The organic phase was collected, washed twice with saturated brine, dried over anhydrous sodium sulfate, filtered, and directly concentrated by rotary evaporation to obtain the desired product. Finally, 650 mg of light yellow solid compound 94 was obtained with a yield of 99.5%. ¹H NMR (400 MHz, CDCl₃) δ 7.62 (s, 1H), 7.36 (s, 1H), 5.80 (s, 1H), 5.39 (s, 1H), 3.61 - 3.48 (m, 1H), 1.06 (s, 3H), 1.00 (s, 3H).

### (3S,10R,13S)-17-(4-Trifluoromethyl-1H-imidazol-1-yl)-10,13-dimethyl-2,3,4,7,8,9,10,11,12,13,14,15-dodecahydro-1H-cyclopenta[a]phenanthren-3-yl methanesulfonate (95)

Compound 4 (650 mg, 1.60 mmol) and 4-dimethylaminopyridine (20 mg, 0.164 mmol) were added to a flask, followed by the addition of anhydrous dichloromethane as solvent. The mixture was stirred in an ice bath, then triethylamine (0.66 mL, 4.84 mmol) was added and stirred for a period of time. Finally, methanesulfonyl chloride (0.25 mL, 3.06 mmol) was slowly added. After the addition was complete, the mixture was stirred at room temperature for 5 h. The reaction was monitored by TLC until completion. Ice water was then added to quench the reaction, followed by extraction with dichloromethane. The organic phase was washed twice with saturated brine, dried over anhydrous sodium sulfate, filtered, and directly concentrated by rotary evaporation to obtain the desired product. Finally, 0.747 g of light yellow solid compound 95 was obtained with a yield of 96.4%. ¹H NMR (400 MHz, CDCl₃) δ 7.71 (s, 1H), 7.39 (s, 1H), 5.84 (d, J = 1.3 Hz, 1H), 5.48 (d, J = 5.0 Hz, 1H), 4.56 (tdd, J = 11.3, 7.0, 4.7 Hz, 1H), 3.04 (s, 3H), 1.09 (s, 3H), 1.03 (s, 3H).

### 1-((3S,10R,13S)-3-Azido-10,13-dimethyl-2,3,4,7,8,9,10,11,12,13,14,15-dodecahydro-1H-cyclopenta[a]phenanthren-17-yl)-4-(trifluoromethyl)-1H-imidazole (96)

Compound 95 (650 mg, 1.34 mmol) was dissolved in anhydrous dichloromethane and stirred at room temperature. Then azidotrimethylsilane (1.06 mL, 8.01 mmol) and boron trifluoride etherate (1.66 mL, 13.4 mmol) were added sequentially using a plastic syringe. After completion of addition, the reaction mixture was stirred overnight at room temperature and monitored by TLC until completion. The reaction was quenched by adding saturated sodium bicarbonate solution, followed by extraction with dichloromethane. The organic phase was washed twice with saturated brine, dried over anhydrous sodium sulfate, filtered, and concentrated onto silica gel. Purification by automated column chromatography (1-3% methanol/dichloromethane) afforded the desired product. Finally, 570 mg of yellow solid 96 was obtained in 98.4% yield. ¹H NMR (400 MHz, CDCl₃) δ 7.65 (s, 1H), 7.38 (s, 1H), 5.82 (dd, J = 3.0, 1.6 Hz, 1H), 5.45 (d, J = 5.0 Hz, 1H), 3.24 (ddd, J = 11.4, 8.8, 4.0 Hz, 1H), 1.07 (s, 3H), 1.03 (s, 3H).

### (3S,10R,13S)-17-(4-(Trifluoromethyl)-1H-imidazol-1-yl)-10,13-dimethyl-2,3,4,7,8,9,10,11,12,13,14,15-dodecahydro-1H-cyclopenta[a]phenanthren-3 -amine (97)

Compound 96 (650 mg, 1.51 mmol) was dissolved in 6 mL of tetrahydrofuran and 6 mL of water and stirred at room temperature. Then triphenylphosphine (435 mg, 1.66 mmol) was added. The temperature was slowly raised to 60°C and stirred overnight at this temperature. The reaction was monitored by TLC until completion. The solvent was partially removed by rotary evaporation, and 20 mL of dichloromethane was added to dissolve the residue. 2 M hydrochloric acid was slowly added to adjust the pH to about 2. The layers were separated and the aqueous phase was collected. 2 M sodium hydroxide solution was then added to adjust the pH to slightly basic, resulting in precipitation of a large amount of white solid. The aqueous phase was extracted three times with a small amount of dichloromethane. The combined organic phases were dried over anhydrous sodium sulfate and concentrated by rotary evaporation to give 355 mg of white solid 97 in 58.1% yield. ¹H NMR (400 MHz, CDCl₃) δ 8.46 (s, 2H), 7.72 (s, 1H), 7.38 (s, 1H), 5.84 (s, 1H), 5.49 (d, J = 3.6 Hz, 1H), 3.13 (s, 1H), 1.10 (s, 3H), 1.03 (s, 3H).

### N-((3S,10R,13S)-17-(4-(Trifluoromethyl)-1H-imidazol-1-yl)-10,13-dimethyl-2,3,4,7, 8,9,10,11,12,13,14,15-dodecahydro-1H-cyclopenta[a]phenanthren-3-yl)pyrazine-2-carboxamide (Compound 22)

Compound 97 (100 mg, 0.247 mmol) was used to obtain 96 mg of white solid compound 22 in 76.2% yield. ¹H NMR (400 MHz, CDCl₃) δ 9.60 (s, 1H), 9.36 (d, J = 5.0 Hz, 1H), 7.92 (dd, J = 5.1, 1.9 Hz, 1H), 7.64 (s, 1H), 7.38 (s, 1H), 6.98 (d, J = 7.9 Hz, 1H), 5.83 (d, J = 1.1 Hz, 1H), 5.46 (d, J = 4.9 Hz, 1H), 4.03 - 3.91 (m, 1H), 1.09 (s, 3H), 1.02 (s, 3H). 13C NMR (151 MHz, DMSO) δ 162.60, 152.55, 149.22, 147.44, 141.36, 137.99, 131.95, 124.58, 121.15, 56.25, 50.30, 50.23, 45.96, 38.60, 37.87, 36.83, 33.99, 31.43, 30.87, 30.10, 29.78, 28.29, 22.53, 20.52, 19.38, 15.88, 14.35. HRMS (ESI) (M+H)+ m/z calcd for C28H31F3N5O 510.2486, found: 510.2489.

### Example 23:

### N-((3S,10R,13S)-17-(4-Methoxy-1H-imidazol-1-yl)-10,13-dimethyl-2,3,4,7,8,9,10,11,12,13,14,15-dodecahydro-1H-cyclopenta[a]phenanthren-3-yl)isonicotinamide (Compound 23)

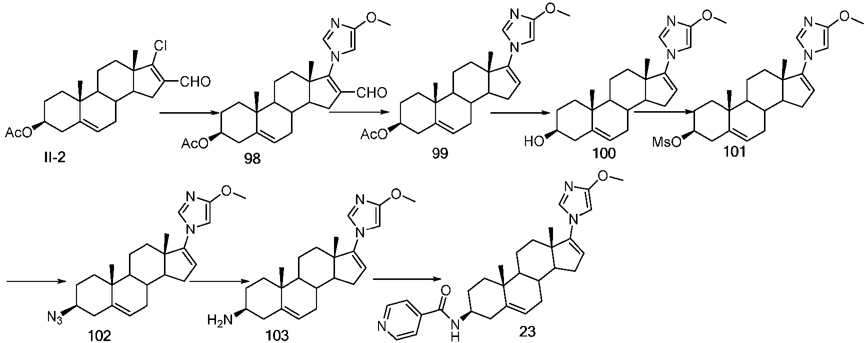

### (3S,10R,13S)-16-Formyl-17-(4-methoxy-1H-imidazol-1-yl)-10,13-dimethyl-2,3,4,7,8,9,10,11,12,13,14,15-dodecahydro-1H-cyclopenta[a]phenanthren-3-yl acetate (98)

Compound II-2 (4.2 g, 11.14 mmol) and potassium carbonate (4.6 g, 33.35 mmol) were placed in a flask. Anhydrous N,N-dimethylformamide was added as solvent and the mixture was stirred at room temperature for half an hour. Then 4-methoxyimidazole (1.09 g, 11.1 mmol) was added to the system. The temperature was slowly raised to 80°C and stirred overnight at this temperature. The reaction was monitored by TLC until completion. After the reaction was complete, the mixture was extracted with ethyl acetate. The organic phase was collected, washed twice with saturated brine, dried over anhydrous sodium sulfate, filtered, and concentrated onto silica gel. Purification by automated column chromatography (10-25% ethyl acetate/petroleum ether) afforded the desired product. Finally, 2 g of yellow solid compound 98 was obtained in 41.08% yield. ¹H NMR (400 MHz, CDCl₃) δ 9.80 (s, 1H), 7.31 (d, J = 1.5 Hz, 1H), 6.44 (d, J = 1.6 Hz, 1H), 5.44 (d, J = 5.0 Hz, 1H), 4.63 (ddd, J = 15.9, 10.7, 5.3 Hz, 1H), 3.88 (s, 3H), 2.06 (s, 3H), 1.09 (d, J = 1.9 Hz, 6H).

### (3S,10R,13S)-17-(4-Methoxy-1H-imidazol-1-yl)-10,13-dimethyl-2,3,4,7,8,9,10,11,12,13,14,15-dodecahydro-1H-cyclopenta[a]phenanthren-3-yl acetate (99)

Compound 98 (2.6 g, 5.93 mmol) was dissolved in anhydrous N,N-dimethylformamide and stirred at room temperature. Then 2.6 g of palladium on carbon was added, followed by purging with argon three times. The system temperature was slowly raised to 160°C and reacted for 30 hours, with TLC monitoring until the reaction was complete. After the reaction was finished, the reaction system was cooled to room temperature and a certain amount of ethyl acetate was added. After stirring for a period of time, the mixture was filtered through diatomaceous earth and the filtrate was collected. The filtrate was then extracted with ethyl acetate and water. The organic phase was collected, washed twice with saturated brine, dried over anhydrous sodium sulfate, and filtered. Silica gel was added to the system, concentrated, and purified by automated column chromatography (10-30% ethyl acetate/petroleum ether) to obtain the desired product. Finally, 0.45 g of yellow solid compound 99 was obtained with a yield of 18.75%. ¹H NMR (400 MHz, DMSO) δ 7.28 (s, 1H), 6.34 (s, 1H), 5.61 (s, 1H), 5.40 (d, J = 4.2 Hz, 1H), 4.66 - 4.55 (m, 1H), 3.81 (d, J = 0.5 Hz, 3H), 2.03 (s, 3H), 1.06 (s, 3H), 1.00 (s, 3H).

### (3S,10R,13S)-17-(4-Methoxy-1H-imidazol-1-yl)-10,13-dimethyl-2,3,4,7,8,9,10,11,12,13,14,15-dodecahydro-1H-cyclopenta[a]phenanthren-3 -ol (100)

Compound 99 (0.45 g, 1.10 mmol) was dissolved in anhydrous methanol and stirred at room temperature. Then, pre-weighed potassium hydroxide (0.246 g, 4.38 mmol) was added. After addition, the mixture was stirred at room temperature for 4 h, with TLC monitoring until the reaction was complete. After the reaction was finished, part of the solvent was removed by rotary evaporation. The mixture was then extracted with ethyl acetate and water. The organic phase was collected, washed twice with saturated brine, dried over anhydrous sodium sulfate, and filtered. Silica gel was added to the system, concentrated, and purified by automated column chromatography (1-2% methanol/dichloromethane) to obtain the desired product. Finally, 330 mg of yellow solid compound 100 was obtained with a yield of 81.88%. ¹H NMR (400 MHz, CDCl₃) δ 7.30 (d, J = 1.3 Hz, 1H), 6.37 (d, J = 1.5 Hz, 1H), 5.63 (d, J = 1.2 Hz, 1H), 5.40 (d, J = 5.1 Hz, 1H), 3.84 (s, 3H), 3.55 (ddd, J = 15.7, 11.0, 4.5 Hz, 1H), 1.07 (s, 3H), 1.02 (s, 3H).

### (3S,10R,13S)-17-(4-Methoxy-1H-imidazol-1-yl)-10,13-dimethyl-2,3,4,7,8,9,10,11,12,13,14,15-dodecahydro-1H-cyclopenta[a]phenanthren-3 -yl methanesulfonate (101)

Compound 100 (330 mg, 0.896 mmol) and 4-dimethylaminopyridine (11 mg, 0.09 mmol) were placed in a flask. Super-dry dichloromethane was added as a solvent and the mixture was stirred in an ice bath. Triethylamine (0.4 mL, 2.936 mmol) was then added and stirred for a period of time. Finally, methanesulfonyl chloride (0.14 mL, 1.72 mmol) was slowly added. After the addition was complete, the mixture was stirred at room temperature for 5 h, with TLC monitoring until the reaction was complete. Ice water was then added to quench the reaction, followed by extraction with dichloromethane. The organic phase was washed twice with saturated brine, dried over anhydrous sodium sulfate, and filtered. The solution was directly concentrated by rotary evaporation to obtain the desired product. Finally, 0.38 g of orange-yellow solid compound 101 was obtained with a yield of 95.2%. ¹H NMR (400 MHz, CDCl₃) δ 7.30 (d, J = 1.4 Hz, 1H), 6.37 (d, J = 1.5 Hz, 1H), 5.64 (dd, J = 3.0, 1.6 Hz, 1H), 5.48 (d, J = 5.0 Hz, 1H), 4.60 - 4.50 (m, 1H), 3.84 (s, 3H), 3.04 (s, 3H), 1.09 (s, 3H), 1.02 (s, 3H).

### 1-((3S,10R,13S)-3-Azido-10,13-dimethyl-2,3,4,7,8,9,10,11,12,13,14,15-dodecahydro-1H-cyclopenta[a]phenanthren-17-yl)-4-methoxy-1H-imidazole (102)

Compound 101 (0.4 g, 0.896 mmol) was dissolved in super-dry dichloromethane and stirred at room temperature. Azidotrimethylsilane (0.54 mL, 4.08 mmol) and boron trifluoride etherate (1.74 mL, 14 mmol) were added sequentially using a plastic syringe. After the addition was complete, the mixture was stirred at room temperature overnight, with TLC monitoring until the reaction was complete. Saturated sodium bicarbonate solution was then added to quench the reaction, followed by extraction with dichloromethane. The organic phase was washed twice with saturated brine, dried over anhydrous sodium sulfate, filtered, and silica gel was added to the organic phase. The mixture was concentrated and purified by automated column chromatography (1-3% methanol/dichloromethane) to obtain the desired product. Finally, 300 mg of yellow solid 102 was obtained with a yield of 84.98%. ¹H NMR (400 MHz, CDCl₃) δ 7.33 (s, 1H), 6.37 (s, 1H), 5.65 (s, 1H), 5.44 (d, J = 4.8 Hz, 1H), 3.84 (s, 3H), 3.23 (dt, J = 10.2, 4.7 Hz, 1H), 1.07 (s, 3H), 1.03 (s, 3H).

### (3S,10R,13S)-17-(4-Methoxy-1H-imidazol-1-yl)-10,13-dimethyl-2,3,4,7,8,9,10,11,12,13,14,15-dodecahydro-1H-cyclopenta[a]phenanthren-3 -amine (103)

Compound 102 (300 mg, 0.763 mmol) was dissolved in 10 mL of tetrahydrofuran and 50 mL of water and stirred at room temperature. Then, pre-weighed triphenylphosphine (0.6 g, 2.29 mmol) was added. The temperature was slowly raised to 60°C and stirred overnight at this temperature. The reaction was monitored by TLC until completion. Then, part of the solvent was removed by rotary evaporation. 20 mL of dichloromethane was added to dissolve the residue, followed by slow addition of 2 M hydrochloric acid to adjust the pH of the solution to about 2. The layers were separated and the aqueous phase was collected. 2 M sodium hydroxide solution was then added to adjust the pH to slightly alkaline, at which point a large amount of white solid precipitated. The aqueous phase was extracted three times with a small amount of dichloromethane. The organic phases were combined, dried over anhydrous sodium sulfate, and concentrated by rotary evaporation to remove the solvent, yielding 50 mg of light yellow solid 103 with a yield of 11.2%. ¹H NMR (400 MHz, CDCl₃) δ 7.30 (s, 1H), 6.37 (s, 1H), 5.63 (s, 1H), 5.36 (d, J = 4.8 Hz, 1H), 3.83 (s, 3H), 2.63 (td, J = 11.3, 5.6 Hz, 1H), 1.05 (s, 3H), 1.02 (s, 3H).

### N-((3S,10R,13S)-17-(4-methoxy-1H-imidazol-1-yl)-10,13-dimethyl-2,3,4,7,8,9,10,11,12,13,14,15-dodecahydro-1H-cyclopenta[a]phenanthren-3-yl)isonicotinamide (Compound 23)

Compound 103 (61 mg, 0.166 mmol) was used to obtain 52 mg of white solid compound 23 with a yield of 66.67%. ¹H NMR (500 MHz, DMSO) δ 8.81 (d, J = 4.9 Hz, 1H), 8.71 (d, J = 7.9 Hz, 1H), 7.90 (d, J = 3.5 Hz, 1H), 7.64 - 7.61 (m, 1H), 7.56 (d, J = 2.6 Hz, 1H), 6.89 (d, J = 1.5 Hz, 1H), 5.89 (s, 1H), 5.39 (d, J = 4.7 Hz, 1H), 3.76 (s, 3H), 1.06 (s, 3H), 1.02 (s, 3H). 13C NMR (126 MHz, DMSO) δ 163.68, 155.36, 149.08, 143.72, 141.49, 132.66, 132.04, 130.36, 129.17, 122.53, 121.04, 96.85, 57.74, 56.39, 50.23, 45.86, 38.62, 37.97, 36.76, 34.30, 30.82, 30.02, 29.58, 28.31, 20.55, 19.30, 15.97. HRMS (ESI) (M+H)+ m/z calculated for C29H37N4O₂ 473.2911, found: 473.2917.

### Example 24:

### N-((3S,10R,13S)-17-(4-methoxy-1H-imidazol-1-yl)-10,13-dimethyl-2,3,4,7,8,9,10,11,12,13,14,15-dodecahydro-1H-cyclopenta[a]phenanthren-3-yl)pyrazine-4-carboxamide (Compound 24)

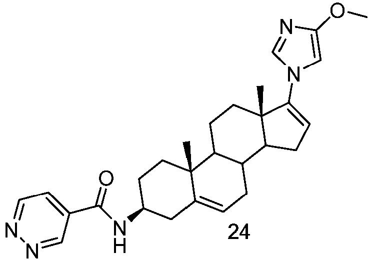

Compound 103 (70 mg, 0.191 mmol) was used to obtain 58 mg of white solid compound 24 with a yield of 64.4%. ¹H NMR (400 MHz, CDCl₃) δ 9.57 (s, 1H), 9.31 (d, J = 5.0 Hz, 1H), 7.93 - 7.90 (m, 1H), 7.49 (d, J = 7.8 Hz, 1H), 7.29 (s, 1H), 6.36 (s, 1H), 5.64 (s, 1H), 5.43 (d, J = 4.3 Hz, 1H), 4.01 - 3.89 (m, 1H), 3.80 (s, 3H), 1.05 (s, 3H), 1.01 (s, 3H). 13C NMR (126 MHz, CDCl₃) δ 162.84, 157.25, 151.79, 148.64, 148.55, 140.32, 132.36, 130.58, 124.22, 121.70, 118.09, 95.87, 56.87, 56.16, 50.86, 50.32, 46.09, 38.77, 37.72, 36.77, 34.83, 30.89, 30.12, 29.66, 28.62, 20.56, 19.13, 15.93. HRMS (ESI) (M+H)+ m/z calculated for C28H36N5O₂ 474.2864, found: 474.2875.

### Example 25:

### N-((3S,10R,13S)-17-(4-cyano-1H-imidazol-1-yl)-10,13-dimethyl-2,3,4,7,8,9,10,11,12,13,14,15-dodecahydro-1H-cyclopenta[a]phenanthren-3-yl)pyrazine-4-carboxamide (Compound 25)

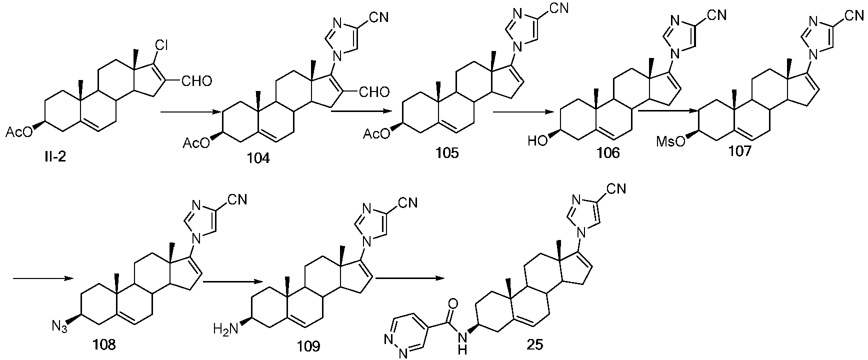

### (3S,10R,13S)-16-formyl-17-(4-cyano-1H-imidazol-1-yl)-10,13-dimethyl-2,3,4,7,8,9,10,11,12,13,14,15-dodecahydro-1H-cyclopenta[a]phenanthren-3-yl acetate (104)

Compound II-2 (3.8 g, 10.08 mmol) and potassium carbonate (4.07 g, 29.5 mmol) were placed in a flask. Anhydrous N,N-dimethylformamide was added as a solvent and stirred at room temperature for half an hour. Then 4-cyanoimidazole (1.03 g, 11.06 mmol) was added to the system. The temperature was slowly raised to 80°C and stirred overnight at this temperature. The reaction was monitored by TLC until completion. After the reaction was complete, the mixture was extracted with ethyl acetate. The organic phase was collected, washed twice with saturated brine, dried over anhydrous sodium sulfate, filtered, and then concentrated with silica gel. The product was purified by automated column chromatography (10-30% ethyl acetate/petroleum ether) to obtain the desired product. Finally, 3.64 g of yellow solid compound 104 was obtained with a yield of 82.4%. ¹H NMR (400 MHz, CDCl₃) δ 9.72 (s, 1H), 7.66 (s, 2H), 5.43 (d, J = 5.0 Hz, 1H), 4.66 - 4.57 (m, 1H), 2.74 (dd, J = 15.6, 6.1 Hz, 1H), 2.05 (s, 3H), 1.09 (s, 6H).

### (3S,10R,13S)-17-(4-cyano-1H-imidazol-1-yl)-10,13-dimethyl-2,3,4,7,8,9,10,11,12,13,14,15-dodecahydro-1H-cyclopenta[a]phenanthren-3-yl acetate (105)

Compound 104 (3.6 g, 8.31 mmol) was dissolved in anhydrous N,N-dimethylformamide and stirred at room temperature. Then 3.6 g of palladium on carbon was added, followed by purging with argon three times. The system temperature was slowly raised to 160°C and reacted for 30 hours, with TLC monitoring until the reaction was complete. After the reaction was finished, the reaction system was cooled to room temperature and a certain amount of ethyl acetate was added. After stirring for a period of time, the mixture was filtered through diatomaceous earth and the filtrate was collected. The filtrate was then extracted with ethyl acetate and water. The organic phase was collected, washed twice with saturated brine, dried over anhydrous sodium sulfate, and filtered. Silica gel was added to the system, concentrated, and purified by automated column chromatography (10-30% ethyl acetate/petroleum ether) to obtain the desired product. Finally, 1.8 g of yellow solid compound 105 was obtained with a yield of 53.57%. ¹H NMR (400 MHz, CDCl₃) δ 7.64 (d, J = 1.1 Hz, 1H), 7.59 (d, J = 1.2 Hz, 1H), 5.86 (dd, J = 3.2, 1.7 Hz, 1H), 5.43 (d, J = 5.1 Hz, 1H), 4.63 (ddd, J = 15.9, 11.1,5.1 Hz, 1H), 2.06 (s, 3H), 1.09 (s, 3H), 1.01 (s, 3H).

### (3S,10R,13S)-17-(4-cyano-1H-imidazol-1-yl)-10,13-dimethyl-2,3,4,7,8,9,10,11,12,13,14,15-dodecahydro-1H-cyclopenta[a]phenanthren-3 -ol (106)

Compound 105 (0.315 g, 0.78 mmol) was dissolved in anhydrous methanol and stirred at room temperature. Then, pre-weighed potassium hydroxide (43 mg, 0.78 mmol) was added. After addition, the mixture was stirred at room temperature for 4 h, with TLC monitoring until the reaction was complete. After the reaction was finished, part of the solvent was removed by rotary evaporation. The mixture was then extracted with ethyl acetate and water. The organic phase was collected, washed twice with saturated brine, dried over anhydrous sodium sulfate, and filtered. The solution was concentrated by rotary evaporation to obtain the desired product. Finally, 175 mg of white solid compound 106 was obtained with a yield of 62.05%. ¹H NMR (400 MHz, DMSO) δ 8.37 (d, J = 0.9 Hz, 1H), 8.11 (d, J = 0.8 Hz, 1H), 6.04 (s, 1H), 5.31 (d, J = 4.4 Hz, 1H), 4.65 (d, J = 4.5 Hz, 1H), 3.27 (td, J = 10.8, 5.5 Hz, 1H), 0.99 (s, 3H), 0.97 (s, 3H).

### (3S,10R,13S)-17-(4-cyano-1H-imidazol-1-yl)-10,13-dimethyl-2,3,4,7,8,9,10,11,12,13,14,15-dodecahydro-1H-cyclopenta[a]phenanthren-3-yl methanesulfonate (107)

Compound 106 (175 mg, 0.482 mmol) and 4-dimethylaminopyridine (6 mg, 0.05 mmol) were placed in a flask. Anhydrous dichloromethane was added as a solvent and the mixture was stirred in an ice bath. Triethylamine (0.2 mL, 1.64 mmol) was then added and stirred for a period of time. Finally, methanesulfonyl chloride (75 µL, 0.856 mmol) was slowly added. After the addition was complete, the mixture was stirred at room temperature for 5 h, with TLC monitoring until the reaction was complete. Ice water was then added to quench the reaction, followed by extraction with dichloromethane. The organic phase was washed twice with saturated brine, dried over anhydrous sodium sulfate, and filtered. The solution was directly concentrated by rotary evaporation to obtain the desired product. Finally, 0.21 g of light yellow solid compound 107 was obtained with a yield of 99%. ¹H NMR (400 MHz, CDCl₃) δ 7.65 (s, 1H), 7.59 (d, J = 1.0 Hz, 1H), 5.87 (dd, J = 3.1, 1.7 Hz, 1H), 5.48 (d, J = 4.9 Hz, 1H), 4.55 (tdd, J = 11.5, 7.2, 4.7 Hz, 1H), 3.04 (s, 3H), 1.09 (s, 3H), 1.02 (s, 3H).

### 4-cyano-1-((3S,10R,13S)-3-azido-10,13-dimethyl-2,3,4,7,8,9,10,11,12,13,14,15-dodecahydro-1H-cyclopenta[a]phenanthren-17-yl)-1H-imidazole (108)

Compound 107 (0.21 g, 0.48 mmol) was dissolved in anhydrous dichloromethane and stirred at room temperature. Azidotrimethylsilane (0.19 mL, 1.42 mmol) and boron trifluoride etherate (0.3 mL, 2.42 mmol) were added sequentially using a plastic syringe. After the addition was complete, the mixture was stirred at room temperature overnight, with TLC monitoring until the reaction was complete. Saturated sodium bicarbonate solution was then added to quench the reaction, followed by extraction with dichloromethane. The organic phase was washed twice with saturated brine, dried over anhydrous sodium sulfate, and filtered. Silica gel was added to the organic phase, concentrated, and purified by automated column chromatography (10-20% ethyl acetate/petroleum ether) to obtain the desired product. Finally, 110 mg of yellow solid 108 was obtained with a yield of 59.45%. ¹H NMR (400 MHz, CDCl₃) δ 7.64 (s, 1H), 7.59 (d, J = 1.0 Hz, 1H), 5.86 (dd, J = 3.1, 1.7 Hz, 1H), 5.45 (d, J = 5.1 Hz, 1H), 3.24 (td, J = 12.4, 5.9 Hz, 1H), 1.08 (s, 3H), 1.02 (s, 3H).

### (3S,10R,13S)-17-(4-cyano-1H-imidazol-1-yl)-10,13-dimethyl-2,3,4,7,8,9,10,11,12,13,14,15-dodecahydro-1H-cyclopenta[a]phenanthren-3 -amine (109)

Compound 108 (110 mg, 0.283 mmol) was dissolved in 6 mL of tetrahydrofuran and 3 mL of water and stirred at room temperature. Then, pre-weighed triphenylphosphine (90 mg, 0.343 mmol) was added. The temperature was slowly raised to 60°C and stirred overnight at this temperature. The reaction was monitored by TLC until completion. Then, part of the solvent was removed by rotary evaporation. 20 mL of dichloromethane was added to dissolve the residue, followed by slow addition of 2 M hydrochloric acid to adjust the pH to about 2. The layers were separated and the aqueous phase was collected. 2 M sodium hydroxide solution was then added to adjust the pH to slightly basic, at which point a large amount of white solid precipitated. The aqueous phase was extracted three times with a small amount of dichloromethane. The organic phases were combined, dried over anhydrous sodium sulfate, and concentrated by rotary evaporation to remove the solvent, yielding 60 mg of white solid 109 with a yield of 58.2%. This was used directly in the next step.

### N-((3S,10R,13S)-17-(4-cyano-1H-imidazol-1-yl)-10,13-dimethyl-2,3,4,7,8,9,10,11,12,13,14,15-dodecahydro-1H-cyclopenta[a]phenanthren-3-yl)pyrazine-4-carboxamide (Compound 25)

Compound 109 (60 mg, 0.166 mmol) was used to obtain 25 mg of yellow solid compound 25, with a yield of 32.5%. ¹H NMR (400 MHz, DMSO) δ 9.57 (s, 1H), 9.36 (s, 1H), 7.88 (s, 1H), 7.61 (s, 1H), 7.57 (s, 1H), 6.78 (s, 1H), 5.85 (s, 1H), 5.44 (d, J = 4.6 Hz, 1H), 3.96 (s, 1H), 1.07 (s, 3H), 0.99 (s, 3H). 13C NMR (126 MHz, CDCl₃) δ 162.68, 152.00, 148.45, 147.18, 140.19, 137.62, 132.40, 126.60, 124.25, 122.79, 121.64, 114.77, 114.44, 56.11, 50.66, 50.26, 46.32, 38.92, 37.69, 36.78, 34.57, 30.88, 30.11, 29.94, 28.69, 20.60, 19.31, 15.88. HRMS (ESI) (M+H)+ m/z calculated for C28H33N6O: 469.271, found: 469.2706.

### Example 26:

### N-((3S,10R,13S)-10,13-dimethyl-17-(4-(methylcarbamoyl)-1H-imidazol-1-yl)-2,3,4,7, 8,9,10,11,12,13,14,15-dodecahydro-1H-cyclopenta[a]phenanthren-3-yl)pyrazine-4-carboxamide (Compound 26)

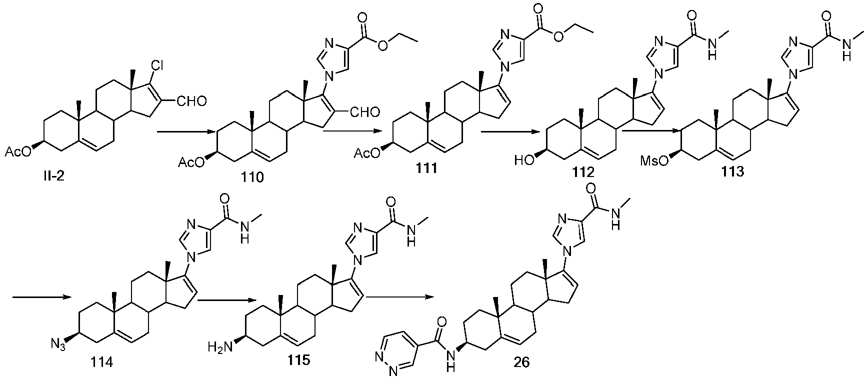

### Ethyl 1-((3S,10R,13S)-3-acetoxy-16-formyl-10,13-dimethyl-2,3,4,7,8,9,10,11,12,13,14,15-dodecahydro-1H-cyclopenta[a]phenanthren-17-yl)-1H-imidazole-4-carboxylate (110)

Compound II-2 (2 g, 5.3 mmol) and potassium carbonate (1.1 g, 7.97 mmol) were placed in a flask. Super-dry N,N-dimethylformamide was added as solvent and stirred at room temperature for half an hour. Then, ethyl imidazole-4-carboxylate (0.75 g, 5.35 mmol) was added to the system. The temperature was slowly raised to 80°C and stirred at this temperature for 1 h. The reaction was monitored by TLC until completion. After the reaction was complete, it was extracted with ethyl acetate. The organic phase was collected, washed twice with saturated brine, dried over anhydrous sodium sulfate, filtered, and then concentrated with silica gel and purified by automated column chromatography (1-3% methanol/dichloromethane) to obtain the desired product. Finally, 1.97 g of white solid compound 110 was obtained with a yield of 77.25%. ¹H NMR (400 MHz, CDCl₃) δ 9.75 (s, 1H), 7.77 (s, 1H), 7.65 (s, 1H), 5.43 (d, J = 4.8 Hz, 1H), 4.67 - 4.56 (m, 1H), 4.42 (q, J = 7.1 Hz, 2H), 2.73 (dd, J = 15.5, 6.2 Hz, 1H), 2.06 (s, 3H), 1.43 (t, J = 7.1 Hz, 3H), 1.10 (s, 3H), 1.09 (s, 3H).

### Ethyl 1-((3S,10R,13S)-3-acetoxy-10,13-dimethyl-2,3,4,7,8,9,10,11,12,13,14,15-dodecahydro-1H-cyclopenta[a]phenanthren- 17-yl)-1 H-imidazole-4-carboxylate (111)

Compound 110 (317 mg, 0.66 mmol) was dissolved in dry N,N-dimethylformamide and stirred at room temperature. Then 0.3 g of palladium on carbon was added. The system was purged with argon three times, and the temperature was slowly raised to 160°C. The reaction was carried out for 18 hours and monitored by TLC until completion. After the reaction was complete, the reaction system was cooled to room temperature, and a certain amount of ethyl acetate was added. After stirring for a period of time, it was filtered through diatomaceous earth. The filtrate was collected and then extracted with ethyl acetate and water. The organic phase was collected, washed twice with saturated brine, dried over anhydrous sodium sulfate, filtered, and then concentrated with silica gel and purified by automated column chromatography (20-40% ethyl acetate/petroleum ether) to obtain the desired product. Finally, 108 mg of white solid compound 111 was obtained with a yield of 36.24%. ¹H NMR (400 MHz, CDCl₃) δ 7.73 (d, J = 1.1 Hz, 1H), 7.65 (d, J = 1.0 Hz, 1H), 5.83 (d, J = 1.3 Hz, 1H), 5.43 (d, J = 5.0 Hz, 1H), 4.63 (ddd, J = 16.0, 10.8, 5.3 Hz, 1H), 4.40 (q, J = 7.1 Hz, 2H), 2.06 (s, 3H), 1.42 (t, J = 7.1 Hz, 3H), 1.09 (s, 3H), 1.02 (s, 3H).

### 1-((3S,10R,13S)-3-hydroxy-10,13-dimethyl-2,3,4,7,8,9,10,11,12,13,14,15-dodecahydro-1H-cyclopenta[a]phenanthren-17-yl)-N-methyl-1H-imidazole-4-carboxamide (112)

Compound 111 (0.75 g, 1.66 mmol) was placed in a sealed tube, followed by addition of 10 mL of methylamine alcohol solution. The sealed tube was reacted overnight at 50°C, and the reaction was monitored by TLC until completion. After the reaction was complete, the system was cooled to room temperature, then extracted with ethyl acetate and water. The organic phase was collected, washed twice with saturated brine, dried over anhydrous sodium sulfate, filtered, and then silica gel was added to the system for concentration and purification by automated column chromatography (1-3% methanol/dichloromethane) to obtain the desired product. Finally, 408 mg of white solid compound 112 was obtained with a yield of 62.29%. ¹H NMR (400 MHz, CDCl₃) δ 7.69 (d, J = 1.3 Hz, 1H), 7.55 (d, J = 1.3 Hz, 1H), 7.17 (d, J = 4.4 Hz, 1H), 5.79 (dd, J = 3.1, 1.7 Hz, 1H), 5.39 (d, J = 5.2 Hz, 1H), 3.61 - 3.51 (m, 1H), 2.99 (d, J = 5.0 Hz, 3H), 1.07 (s, 3H), 1.02 (s, 3H).

### (3S,10R,13S)-10,13-dimethyl-17-(4-(methylcarbamoyl)-1H-imidazol-1-yl)-2,3,4,7,8,9,10,11,12,13,14,15-dodecahydro-1H-cyclopenta[a]phenanthren-3-yl methanesulfonate (113)

Compound 112 (408 mg, 1.03 mmol) and 4-dimethylaminopyridine (12 mg, 0.1 mmol) were placed in a flask, and anhydrous dichloromethane was added as a solvent and stirred in an ice bath. Triethylamine (0.43 mL, 3.28 mmol) was then added and stirred for a period of time. Finally, methanesulfonyl chloride (160 µL, 2.06 mmol) was slowly added. After the addition was complete, the mixture was stirred at room temperature for 4 h, and the reaction was monitored by TLC until completion. Ice water was then added for quenching, followed by extraction with dichloromethane. The organic phase was washed twice with saturated brine, dried over anhydrous sodium sulfate, filtered, and directly concentrated by rotary evaporation to obtain the desired product. Finally, 0.46 g of light yellow solid compound 113 was obtained with a yield of 94.26%. ¹H NMR (400 MHz, CDCl₃) δ 7.71 (s, 1H), 7.71 (s, 1H), 7.39 (s, 1H), 5.86 (dd, J = 3.0, 1.6 Hz, 1H), 5.48 (d, J = 4.7 Hz, 1H), 4.56 (ddd, J = 14.2, 11.4, 4.8 Hz, 1H), 3.04 (s, 3H), 3.00 (d, J = 5.0 Hz, 3H), 1.09 (s, 3H), 1.03 (s, 3H).

### 1-((3S,10R,13S)-3-azido-10,13-dimethyl-2,3,4,7,8,9,10,11,12,13,14,15-dodecahydro-1H-cyclopenta[a]phenanthren-17-yl)-N-methyl-1H-imidazole-4-carboxamide (114)

Compound 113 (0.46 g, 0.97 mmol) was dissolved in anhydrous dichloromethane and stirred at room temperature. Azidotrimethylsilane (0.5 mL, 3.55 mmol) and boron trifluoride etherate (0.6 mL, 4.84 mmol) were added sequentially using a plastic syringe. After the addition was complete, the mixture was stirred at room temperature overnight, and the reaction was monitored by TLC until completion. Saturated sodium bicarbonate solution was then added for quenching, followed by extraction with dichloromethane. The organic phase was washed twice with saturated brine, dried over anhydrous sodium sulfate, filtered, and then silica gel was added to the organic phase for concentration and purification by automated column chromatography (30-60% ethyl acetate/petroleum ether) to obtain the desired product. Finally, 219 mg of white solid 114 was obtained with a yield of 53.68%. ¹H NMR (400 MHz, CDCl₃) δ 7.70 (d, J = 1.3 Hz, 1H), 7.58 (d, J = 1.2 Hz, 1H), 7.22 (d, J = 4.4 Hz, 1H), 5.81 (dd, J = 3.1, 1.7 Hz, 1H), 5.44 (d, J = 5.1 Hz, 1H), 3.24 (ddd, J = 9.9, 8.7, 3.9 Hz, 1H), 2.99 (d, J = 5.0 Hz, 3H), 1.07 (s, 3H), 1.02 (s, 3H).

### 1-((3S,10R,13S)-3-amino-10,13-dimethyl-2,3,4,7,8,9,10,11,12,13,14,15-dodecahydro-1H-cyclopenta[a]phenanthren-17-yl)-N-methyl-1H-imidazole-4-carboxamide (115)

Compound 114 (219 mg, 0.52 mmol) was dissolved in 10 mL of tetrahydrofuran and 5 mL of water and stirred at room temperature. Pre-weighed triphenylphosphine (205 mg, 0.78 mmol) was then added, and the temperature was slowly raised to 60°C. The mixture was stirred overnight at this temperature, and the reaction was monitored by TLC until completion. The solvent was partially removed by rotary evaporation, 20 mL of dichloromethane was added to dissolve, and 2M hydrochloric acid was slowly added to adjust the pH of the solution to about 2. The layers were separated and the aqueous phase was collected. 2M sodium hydroxide solution was then added to adjust the pH to slightly alkaline, at which point a large amount of white solid precipitated. The aqueous phase was extracted three times with a small amount of dichloromethane, and the combined organic phases were collected, dried over anhydrous sodium sulfate, and rotary evaporated to remove the solvent, yielding 58 mg of white solid 115 with a yield of 28.3%. The product was directly used in the next step.

### N-((3S,10R,13S)-10,13-dimethyl-17-(4-(methylcarbamoyl)-1H-imidazol-1-yl)-2,3,4,7, 8,9,10,11,12,13,14,15-dodecahydro-1H-cyclopenta[a]phenanthren-3-yl)pyrazine-4-carboxamide (Compound 26)

Compound 115 (58 mg, 0.147 mmol) was used to obtain 53 mg of white solid compound 26, with a yield of 72.1%. ¹H NMR (500 MHz, DMSO) δ 9.52 - 9.50 (m, 1H), 9.41 (dd, J = 5.3, 1.0 Hz, 1H), 8.79 (d, J = 7.9 Hz, 1H), 7.98 - 7.94 (m, 2H), 7.88 (d, J = 1.1 Hz, 1H), 7.72 (d, J = 1.1 Hz, 1H), 5.96 (s, 1H), 5.37 (d, J = 4.6 Hz, 1H), 3.76 - 3.65 (m, 1H), 2.72 (d, J = 4.8 Hz, 3H), 1.03 (s, 3H), 0.97 (s, 3H). 13C NMR (126 MHz, DMSO) δ 162.69, 162.63, 152.55, 149.27, 147.76, 141.36, 137.68, 136.34, 132.12, 124.59, 121.17, 120.30, 119.79, 56.20, 50.32, 50.22, 46.06, 38.61, 37.88, 36.84, 34.28, 30.89, 30.12, 29.73, 28.30, 25.92, 20.58, 19.39, 15.98. HRMS (ESI) (M+H)+ m/z calculated for C28H41N2O₆ 501.2959, found: 501.2954.

### Example 27:

### 1-((3S,10R,13S)-10,13-dimethyl-3-(pyrazine-4-carboxamido)-2,3,4,7,8,9,10,11,12,13,14,15-dodecahydro-1H-cyclopenta[a]phenanthren-17-yl)-1H-imidazole-4-carboxylic acid (Compound 27)

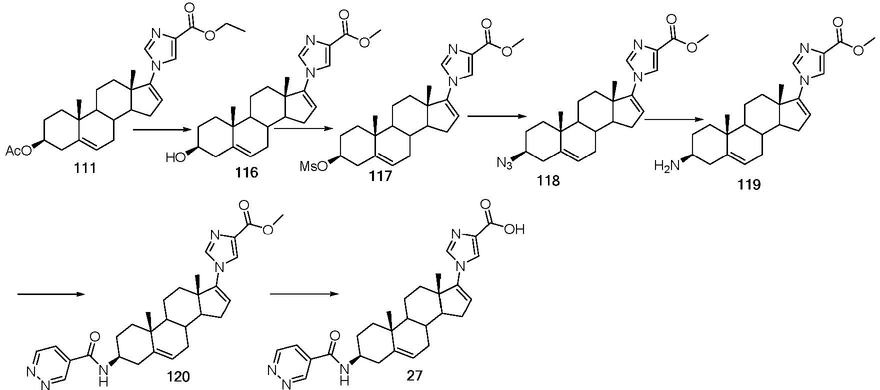

### Methyl 1-((3S,10R,13S)-3-hydroxy-10,13-dimethyl-2,3,4,7,8,9,10,11,12,13,14,15-dodecahydro-1H-cyclopenta[a]phenanthren-17-yl)-1H-imidazole-4-carboxylate (116)

Compound 111 (2.14 g, 4.73 mmol) was placed in a flask, followed by addition of 20 mL of sodium methoxide in methanol solution. The mixture was stirred at room temperature overnight, and the reaction was monitored by TLC until completion. After the reaction was complete, it was neutralized with 4M hydrochloric acid in methanol solution. When the solution changed from yellow to colorless, it was extracted with dichloromethane and water. The organic phase was collected, dried over anhydrous sodium sulfate, filtered, and directly rotary evaporated to obtain the desired product. Finally, 1.85 g of yellow solid compound 116 was obtained with a yield of 98.9%. ¹H NMR (400 MHz, DMSO) δ 7.96 (d, J = 1.2 Hz, 1H), 7.94 (s, 1H), 6.00 (s, 1H), 5.28 (d, J = 4.4 Hz, 1H), 4.65 (s, 1H), 3.74 (s, 3H), 3.25 (s, 1H), 0.97 (s, 3H), 0.95 (s, 3H).

### Methyl 1-((3S,10R,13S)-10,13-dimethyl-3-((methylsulfonyl)oxy)-2,3,4,7,8,9,10,11,12,13,14,15-dodecahydro-1H-cyclopenta[a]phenanthren-17-yl)-1H-imidazole-4-carboxylate (117)

Compound 116 (105 mg, 0.25 mmol) and 4-dimethylaminopyridine (3 mg, 0.025 mmol) were placed in a flask, and anhydrous dichloromethane was added as solvent. The mixture was stirred in an ice bath, followed by addition of triethylamine (0.1 mL, 0.75 mmol) and stirred for a period of time. Finally, methanesulfonyl chloride (39 µL, 0.5 mmol) was slowly added. After the addition was complete, the mixture was stirred at room temperature for 4 h, and the reaction was monitored by TLC until completion. Ice water was then added for quenching, followed by extraction with dichloromethane. The organic phase was washed twice with saturated brine, dried over anhydrous sodium sulfate, filtered, and directly rotary evaporated to obtain the desired product. Finally, 117 mg of white solid compound 117 was obtained with a yield of 97.8%. ¹H NMR (400 MHz, CDCl₃) δ 7.72 (s, 2H), 5.83 (s, 1H), 5.45 (d, J = 4.9 Hz, 1H), 4.54 (d, J = 5.0 Hz, 1H), 3.91 (s, 3H), 3.02 (s, 3H), 1.07 (s, 3H), 1.00 (s, 3H).

### Methyl 1-((3S,10R,13S)-3-azido-10,13-dimethyl-2,3,4,7,8,9,10,11,12,13,14,15-dodecahydro-1H-cyclopenta[a]phenanthren-17-yl)-1H-imidazole-4-carboxylate (118)

Compound 117 (0.11 g, 0.23 mmol) was dissolved in anhydrous dichloromethane and stirred at room temperature. Azidotrimethylsilane (92 µL, 0.69 mmol) and boron trifluoride etherate (143 µL, 1.15 mmol) were added sequentially using a plastic syringe. After the addition was complete, the mixture was stirred at room temperature overnight, and the reaction was monitored by TLC until completion. Saturated sodium bicarbonate solution was then added for quenching, followed by extraction with dichloromethane. The organic phase was washed twice with saturated brine, dried over anhydrous sodium sulfate, filtered, and silica gel was added to the organic phase for concentration. The mixture was purified by automated column chromatography (30-60% ethyl acetate/petroleum ether) to obtain the desired product. Finally, 66 mg of white solid 118 was obtained with a yield of 68.7%. ¹H NMR (400 MHz, CDCl₃) δ 7.74 (s, 1H), 7.68 (s, 1H), 5.84 (s, 1H), 5.44 (d, J = 5.0 Hz, 1H), 3.93 (s, 3H), 3.28 - 3.19 (m, 1H), 1.07 (s, 3H), 1.02 (s, 3H).

### Methyl 1-((3 S,10R,13S)-3-amino-10,13-dimethyl-2,3,4,7,8,9,10,11,12,13,14,15-dodecahydro-1H-cyclopenta[a]phenanthren-17-yl)-1H-imidazole-4-carboxylate (119)

Compound 118 (55 mg, 0.13 mmol) was dissolved in 6 mL of tetrahydrofuran and 6 mL of water and stirred at room temperature. Then, pre-weighed triphenylphosphine (51 mg, 0.19 mmol) was added. The temperature was slowly raised to 60°C and stirred overnight at this temperature. The reaction was monitored by TLC until completion. Then, part of the solvent was removed by rotary evaporation. 20 mL of dichloromethane was added to dissolve the residue, followed by slow addition of 2M hydrochloric acid to adjust the pH to about 2. The layers were separated and the aqueous phase was collected. 2M sodium hydroxide solution was then added to adjust the pH to weakly basic, at which point a large amount of white solid precipitated. The aqueous phase was extracted three times with a small amount of dichloromethane. The organic phases were combined, dried over anhydrous sodium sulfate, and concentrated by rotary evaporation to remove the solvent, yielding 29 mg of white solid 119 with a yield of 55.8%. ¹H NMR (400 MHz, CDCl₃) δ 7.74 (s, 1H), 7.63 (s, 1H), 5.82 (s, 1H), 5.38 (s, 1H), 3.92 (d, J = 2.1 Hz, 3H), 1.06 (s, 3H), 1.01 (s, 3H).

### Methyl 1-((3S,10R,13S)-10,13-dimethyl-3-(pyrazine-4-carboxamido)-2,3,4,7,8,9,10,11,12,13,14,15-dodecahydro-1H-cyclopenta[a]phenanthren-17-yl)-1H-imidazole-4-carboxylate (120)

Compound 119 (24 mg, 0.06 mmol) was used to obtain 22 mg of white solid compound 120 with a yield of 73.3%. ¹H NMR (500 MHz, CDCl₃) δ 9.58 (s, 1H), 9.31 (d, J = 5.2 Hz, 1H), 7.93 (dd, J = 5.0, 1.8 Hz, 1H), 7.72 (s, 1H), 7.63 (s, 1H), 7.29 (s, 1H), 5.83 (s, 1H), 5.44 (d, J = 4.6 Hz, 1H), 3.95 (qd, J = 12.3, 6.4 Hz, 1H), 3.89 (s, 3H), 1.05 (s, 3H), 1.01 (s,3H).

### 1-((3S,10R,13S)-10,13-dimethyl-3-(pyrazine-4-carboxamido)-2,3,4,7,8,9,10,11,12,13,14,15-dodecahydro-1H-cyclopenta[a]phenanthren-17-yl)-1H-imidazole-4-carboxyfic acid (Compound 27)

Compound 120 (50 mg, 0.1 mmol) was dissolved in 5 mL of methanol and 5 mL of tetrahydrofuran and stirred at room temperature. Then, 1 mL of 2M sodium hydroxide solution was added and reacted at room temperature for 2 h. The reaction was monitored by TLC until completion. Part of the solution was then removed by rotary evaporation, followed by adjustment of the pH to acidic with dilute hydrochloric acid. The mixture was extracted with ethyl acetate, and the organic phase was washed twice with saturated brine. After drying over anhydrous sodium sulfate, the solution was filtered and directly concentrated by rotary evaporation to obtain the desired product. Finally, 33 mg of white solid 27 was obtained with a yield of 67.8%. ¹H NMR (500 MHz, DMSO) δ 9.54 (s, 1H), 9.43 (d, J = 4.7 Hz, 1H), 8.82 (d, J = 7.4 Hz, 1H), 8.00 (d, J = 2.9 Hz, 1H), 7.93 (s, 1H), 7.90 (s, 1H), 6.00 (s, 1H), 5.39 (s, 1H), 3.73 (s, 1H), 1.05 (s, 3H), 0.99 (s, 3H). 13C NMR (126 MHz, DMSO) δ 163.85, 162.63, 152.54, 149.22, 147.59, 141.49, 137.45, 134.15, 132.11, 124.58, 124.28, 121.15, 120.50, 56.23, 50.32, 50.22, 46.00, 38.60, 37.88, 36.83, 34.25, 30.81, 30.18, 29.80, 28.22, 21.45, 20.56, 19.31, 15.90. HRMS (ESI) (M+H)+ m/z calculated for C28H34N5O₃ 488.2656, found: 488.2657.

### Example 28:

### 4-fluoro-N-((3S,10R,13S)-17-(4-fluoro-1H-imidazol-1-yl)-10,13-dimethyl-2,3,4,7,8,9,10,11,12,13,14,15-dodecahydro-1H-cyclopenta[a]phenanthren-3-yl)benzamide (Compound 28)

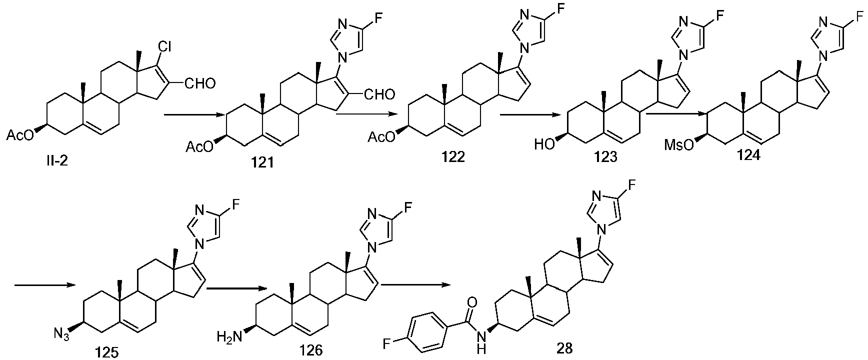

### (3S,10R,13S)-17-(4-fluoro-1H-imidazol-1-yl)-16-formyl-10,13-dimethyl-2,3,4,7,8,9,10,11,12,13,14,15-dodecahydro-1H-cyclopenta[a]phenanthren-3-yl acetate (121)

Compound II-2 (4.38 g, 11.62 mmol) and potassium carbonate (2.4 g, 17.5 mmol) were placed in a flask. Anhydrous N,N-dimethylformamide was added as solvent and stirred at room temperature for half an hour. Then 4-fluoroimidazole (1 g, 11.62 mmol) was added to the system. The temperature was slowly raised to 80°C and stirred overnight at this temperature. The reaction was monitored by TLC until completion. After the reaction was complete, the mixture was extracted with ethyl acetate. The organic phase was collected, washed twice with saturated brine, dried over anhydrous sodium sulfate, filtered, and then concentrated with silica gel. The product was purified by automated column chromatography (10-30% ethyl acetate/petroleum ether) to obtain the desired product. Finally, 1.8 g of light yellow solid compound 121 was obtained with a yield of 36.3%. ¹H NMR (400 MHz, CDCl₃) δ 9.78 (s, 1H), 7.25 (s, 1H), 6.69 (dd, J = 8.1, 1.6 Hz, 1H), 5.43 (d, J = 5.2 Hz, 1H), 4.67 - 4.58 (m, 1H), 2.06 (s, 3H), 1.09 (s, 6H).

### (3S,10R,13S)-17-(4-fluoro-1H-imidazol-1-yl)-10,13-dimethyl-2,3,4,7,8,9,10,11,12,13,14,15-dodecahydro-1H-cyclopenta[a]phenanthren-3-yl acetate (122)

Compound 1,3-bis(diphenylphosphino)propane (0.48 g, 1.16 mmol) and carbonylbis(triphenylphosphine)rhodium(I) chloride (0.48 g, 0.69 mmol) were dissolved in anhydrous xylene. The system was purged with argon three times, then slowly heated to 80°C and stirred for half an hour. Compound 121 (1 g, 2.35 mmol) was then added, and the reaction temperature was raised to 135°C and stirred overnight at this temperature. After the reaction was complete, the reaction system was cooled to room temperature, then extracted with ethyl acetate and water. The organic phase was collected, washed twice with saturated brine, dried over anhydrous sodium sulfate, filtered, and then silica gel was added to the system for concentration and purification by automated column chromatography (10-20% ethyl acetate/petroleum ether) to obtain the desired product. Finally, 230 mg of white solid compound 122 was obtained with a yield of 24.62%. ¹H NMR (400 MHz, CDCl₃) δ 7.24 (s, 1H), 6.59 (d, J = 8.1 Hz, 1H), 5.72 (s, 1H), 5.43 (d, J = 5.1 Hz, 1H), 4.63 (tt, J = 10.7, 5.1 Hz, 1H), 2.06 (s, 3H), 1.09 (s, 3H), 1.02 (s, 3H).

### (3S,10R,13S)-17-(4-fluoro-1H-imidazol-1-yl)-10,13-dimethyl-2,3,4,7,8,9,10,11,12,13,14,15-dodecahydro-1H-cyclopenta[a]phenanthren-3 -ol (123)

Compound 122 (0.22 g, 0.55 mmol) was dissolved in anhydrous methanol and stirred at room temperature. Then, pre-weighed potassium hydroxide (50 mg, 0.9 mmol) was added. After addition, the mixture was stirred at room temperature for 1 h, and the reaction was monitored by TLC until completion. After the reaction was complete, part of the solvent was removed by rotary evaporation, then extracted with ethyl acetate and water. The organic phase was collected, washed twice with saturated brine, dried over anhydrous sodium sulfate, filtered, and then concentrated by rotary evaporation to obtain the desired product. Finally, 167 mg of white solid compound 123 was obtained with a yield of 84.77%. ¹H NMR (600 MHz, CDCl₃) δ 7.14 (s, 1H), 6.50 (dd, J = 8.1, 1.4 Hz, 1H), 5.62 (dd, J = 3.0, 1.7 Hz, 1H), 5.33 - 5.29 (m, 1H), 3.50 - 3.44 (m, 1H), 0.99 (s, 3H), 0.93 (s, 3H).

### (3S,10R,13S)-17-(4-fluoro-1H-imidazol-1-yl)-10,13-dimethyl-2,3,4,7,8,9,10,11,12,13,14,15-dodecahydro-1H-cyclopenta[a]phenanthren-3-yl methanesulfonate (124)

Compound 123 (160 mg, 0.449 mmol) and 4-dimethylaminopyridine (6 mg, 0.05 mmol) were placed in a flask, and anhydrous dichloromethane was added as a solvent. The mixture was stirred in an ice bath, then triethylamine (0.2 mL, 1.64 mmol) was added and stirred for a period of time. Finally, methanesulfonyl chloride (150 µL, 1.71 mmol) was slowly added. After the addition was complete, the mixture was stirred at room temperature for 2 h, and the reaction was monitored by TLC until completion. Ice water was then added for quenching, followed by extraction with dichloromethane. The organic phase was washed twice with saturated brine, dried over anhydrous sodium sulfate, filtered, and directly concentrated by rotary evaporation to obtain the desired product. Finally, 0.29 g of yellow solid compound 124 was obtained and directly used in the next step.

### 1-((3S,10R,13S)-3-azido-10,13-dimethyl-2,3,4,7,8,9,10,11,12,13,14,15-dodecahydro-1H-cyclopenta[a]phenanthren-17-yl)-4-fluoro-1H-imidazole (125)

Compound 124 (0.29 g, 0.68 mmol) was dissolved in anhydrous dichloromethane and stirred at room temperature. Then, azidotrimethylsilane (0.3 mL, 2.24 mmol) and boron trifluoride etherate (0.34 mL, 2.42 mmol) were added sequentially using a plastic syringe. After the addition was complete, the mixture was stirred at room temperature overnight, and the reaction was monitored by TLC until completion. Saturated sodium bicarbonate solution was then added for quenching, followed by extraction with dichloromethane. The organic phase was washed twice with saturated brine, dried over anhydrous sodium sulfate, filtered, and then silica gel was added to the organic phase for concentration and purification by automated column chromatography (5-20% ethyl acetate/petroleum ether) to obtain the desired product. Finally, 146 mg of white solid compound 125 was obtained with a yield of 56.59%. ¹HNMR (600 MHz, CDCl₃) δ 7.16 (s, 1H), 6.50 (dd, J = 8.1, 1.1 Hz, 1H), 5.63 (dd, J = 3.0, 1.6 Hz, 1H), 5.36 (d, J = 5.3 Hz, 1H), 3.18 - 3.12 (m, 1H), 0.98 (s, 3H), 0.93 (s, 3H).

### (3S,10R,13S)-17-(4-fluoro-1H-imidazol-1-yl)-10,13-dimethyl-2,3,4,7,8,9,10,11,12,13,14,15-dodecahydro-1H-cyclopenta[a]phenanthren-3 -amine (126)

Compound 125 (140 mg, 0.367 mmol) was dissolved in 10 mL of tetrahydrofuran and 5 mL of water and stirred at room temperature. Then, pre-weighed triphenylphosphine (145 mg, 0.553 mmol) was added, and the temperature was slowly raised to 60°C. The mixture was stirred at this temperature overnight, and the reaction was monitored by TLC until completion. Then, part of the solvent was removed by rotary evaporation, 20 mL of ethyl acetate was added to dissolve, and 2 M hydrochloric acid was slowly added to adjust the pH of the solution to about 2. The mixture was separated, and the aqueous phase was collected. Then, 2 M sodium hydroxide solution was added to adjust the pH to slightly alkaline, at which point a large amount of white solid precipitated. The aqueous phase was extracted three times with a small amount of ethyl acetate, and the organic phases were combined, dried over anhydrous sodium sulfate, and rotary evaporated to remove the solvent, yielding 72 mg of white solid compound 126 with a yield of 55.38%. The product was directly used in the next step.

### 4-fluoro-N-((3S,10R,13S)-17-(4-fluoro-1H-imidazol-1-yl)-10,13-dimethyl-2,3,4,7,8,9,10,11,12,13,14,15-dodecahydro-1H-cyclopenta[a]phenanthren-3-yl)benzamide (Compound 28)

Compound 126 (47 mg, 0.132 mmol) was used to obtain 47 mg of white solid compound 28 with a yield of 74.6%. ¹H NMR (600 MHz, DMSO) δ 8.28 (d, J = 8.0 Hz, 1H), 7.89 (dd, J = 8.7, 5.6 Hz, 2H), 7.54 (s, 1H), 7.25 (t, J = 8.8 Hz, 2H), 7.09 (dd, J = 8.2, 1.6 Hz, 1H), 5.83 (s, 1H), 5.33 (d, J = 4.7 Hz, 1H), 3.72 - 3.64 (m, 1H), 1.01 (s, 3H), 0.95 (s, 3H). 13CNMR (151 MHz, DMSO) δ 164.17, 162.95, 157.49, 155.96, 147.69, 141.24, 131.13, 129.85, 129.80, 129.62, 129.52, 120.35, 118.37, 115.14, 115.00, 9720, 97.00, 55.84, 49.78, 49.48, 45.37, 38.42, 37.54, 36.38, 33.73, 30.42, 29.63, 29.15, 28.05, 20.14, 18.92, 15.49. HRMS (ESI) (M+H)+m/z calculated for C29H34F2N3O 478.2664, measured: 478.2665.

### Example 29:

### 4-Fluoro-N-((3S,10R,13S)-17-(4-bromo-1H-imidazol-l-yl)-10,13-dimethyl-2,3,4,7,8,9,10,11,12,13,14,15-dodecahydro-1H-cyclopenta[a]phenanthren-3-yl)benzamide (Compound 29)

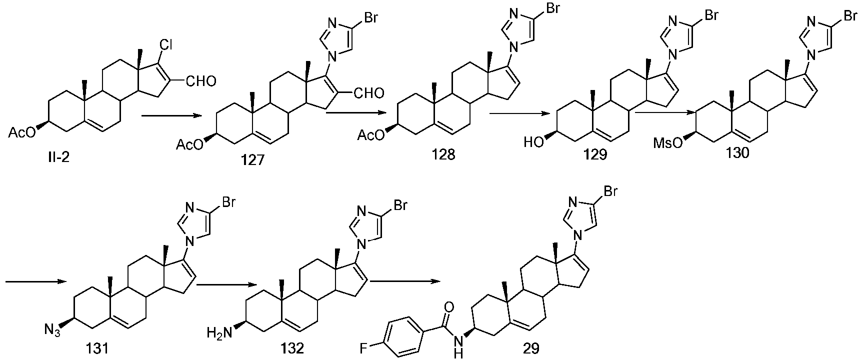

### (3S,10R,13S)-17-(4-Bromo-1H-imidazol-1-yl)-16-formyl-10,13-dimethyl-2,3,4,7,8,9,10,11,12,13,14,15-dodecahydro-1H-cyclopenta[a]phenanthren-3-yl acetate (127)

Compound II-2 (4 g, 11.62 mmol) and potassium carbonate (2.4 g, 17.5 mmol) were added to a flask, followed by the addition of anhydrous N,N-dimethylformamide as solvent. After stirring at room temperature for half an hour, 4-bromoimidazole (1.5 g, 10.27 mmol) was added to the system. The temperature was slowly raised to 80°C and stirred overnight at this temperature. The reaction was monitored by TLC until completion. After the reaction was complete, the mixture was extracted with ethyl acetate. The organic phase was collected, washed twice with saturated brine, dried over anhydrous sodium sulfate, filtered, and then concentrated with silica gel. The product was purified by automated column chromatography (10-30% ethyl acetate/petroleum ether) to obtain the desired product. Finally, 3.4 g of yellow solid compound 127 was obtained with a yield of 68.19%. ¹H NMR (400 MHz, CDCl₃) δ 9.76 (s, 1H), 7.52 (d, J = 1.4 Hz, 1H), 7.11 (d, J = 1.5 Hz, 1H), 5.43 (d, J = 5.1 Hz, 1H), 4.63 (dq, J = 15.9, 5.2 Hz, 1H), 2.71 (dd, J = 15.5, 6.3 Hz, 1H), 2.06 (s, 3H), 1.08 (s, 6H).

### (3S,10R,13S)-17-(4-Bromo-1H-imidazol-1-yl)-10,13-dimethyl-2,3,4,7,8,9,10,11,12,13,14,15-dodecahydro-1H-cyclopenta[a]phenanthren-3-yl acetate (128)

1,3-Bis(diphenylphosphino)propane (0.21 g, 0.51 mmol) and carbonylbis(triphenylphosphine)rhodium(I) chloride (0.17 g, 0.24 mmol) were dissolved in dry xylene. The system was purged with argon three times, then slowly heated to 80°C and stirred for half an hour. Compound 127 (1 g, 2.06 mmol) was then added, and the reaction temperature was raised to 135°C and stirred overnight at this temperature. After the reaction was complete, the reaction system was cooled to room temperature, then extracted with ethyl acetate and water. The organic phase was collected, washed twice with saturated brine, dried over anhydrous sodium sulfate, filtered, and then concentrated with silica gel. The product was purified by automated column chromatography (10-30% ethyl acetate/petroleum ether) to obtain the desired product. Finally, 322 mg of white solid compound 128 was obtained with a yield of 34.18%. ¹H NMR (400 MHz, CDCl₃) δ 7.50 (s, 1H), 7.02 (s, 1H), 5.72 (s, 1H), 5.41 (d, J = 5.0 Hz, 1H), 4.60 (dt, J = 10.9, 5.9 Hz, 1H), 2.04 (s, 3H), 1.07 (s, 3H), 0.99 (s, 3H).

### (3S,10R,13S)-17-(4-Bromo-1H-imidazol-1-yl)-10,13-dimethyl-2,3,4,7,8,9,10,11,12,13,14,15-dodecahydro-1H-cyclopenta[a]phenanthren-3 -ol (129)

Compound 128 (0.497 g, 1.08 mmol) was dissolved in anhydrous methanol and stirred at room temperature. Then, potassium hydroxide (183 mg, 3.26 mmol) was added. After addition, the mixture was stirred at room temperature for 2 h, and the reaction was monitored by TLC until completion. After the reaction was complete, part of the solvent was removed by rotary evaporation, then extracted with ethyl acetate and water. The organic phase was collected, washed twice with saturated brine, dried over anhydrous sodium sulfate, filtered, and then concentrated by rotary evaporation to obtain the desired product. Finally, 278 mg of white solid compound 129 was obtained with a yield of 65.85%. ¹H NMR (400 MHz, DMSO) δ 7.84 (s, 1H), 7.54 (s, 1H), 5.90 (s, 1H), 5.31 (d, J = 4.6 Hz, 1H), 4.64 (d, J = 4.5 Hz, 1H), 3.31 - 3.22 (m, 1H), 1.00 (s, 3H), 0.98 (s, 3H).

### (3S,10R,13S)-17-(4-Bromo-1H-imidazol-1-yl)-10,13-dimethyl-2,3,4,7,8,9,10,11,12,13,14,15-dodecahydro-1H-cyclopenta[a]phenanthren-3-yl methanesulfonate (130)

Compound 129 (278 mg, 0.668 mmol) and 4-dimethylaminopyridine (6 mg, 0.05 mmol) were added to a flask. Anhydrous dichloromethane was added as solvent and the mixture was stirred in an ice bath. Triethylamine (0.28 mL, 2.3 mmol) was then added and stirred for a period of time. Methanesulfonyl chloride (150 µL, 1.71 mmol) was slowly added dropwise. After completion of addition, the mixture was stirred at room temperature for 2 h and monitored by TLC until the reaction was complete. Ice water was then added to quench the reaction, followed by extraction with dichloromethane. The organic phase was washed twice with saturated brine, dried over anhydrous sodium sulfate, filtered, and concentrated by rotary evaporation to obtain the desired product. Finally, 382 mg of yellow oily compound 130 was obtained and used directly in the next step.

### 1-((3S,10R,13S)-3-azido-10,13-dimethyl-2,3,4,7,8,9,10,11,12,13,14,15-dodecahydro-1H-cyclopenta[a]phenanthren-17-yl)-4-bromo-1H-imidazole (131)

Compound 130 (0.382 g, 0.77 mmol) was dissolved in anhydrous dichloromethane and stirred at room temperature. Azidotrimethylsilane (0.3 mL, 2.24 mmol) and boron trifluoride etherate (0.34 mL, 2.42 mmol) were added sequentially using a plastic syringe. After completion of addition, the mixture was stirred at room temperature overnight and monitored by TLC until the reaction was complete. Saturated sodium bicarbonate solution was then added to quench the reaction, followed by extraction with dichloromethane. The organic phase was washed twice with saturated brine, dried over anhydrous sodium sulfate, filtered, and concentrated onto silica gel. Purification was performed using an automated column chromatography system (15-25% ethyl acetate/petroleum ether) to obtain the desired product. Finally, 183 mg of white solid compound 131 was obtained in 53.66% yield. ¹H NMR (400 MHz, DMSO) δ 7.84 (s, 1H), 7.54 (s, 1H), 5.90 (s, 1H), 5.43 (d, J = 4.1 Hz, 1H), 1.01 (s, 3H), 0.98 (s, 3H).

### (3S,10R,13S)-17-(4-bromo-1H-imidazol-1-yl)-10,13-dimethyl-2,3,4,7,8,9,10,11,12,13,14,15-dodecahydro-1H-cyclopenta[a]phenanthren-3 -amine (132)

Compound 131 (180 mg, 0.408 mmol) was dissolved in 10 mL of tetrahydrofuran and 5 mL of water and stirred at room temperature. Triphenylphosphine (160 mg, 0.61 mmol) was added, and the temperature was slowly raised to 60°C. The mixture was stirred overnight at this temperature and monitored by TLC until the reaction was complete. The solvent was partially removed by rotary evaporation, and 20 mL of ethyl acetate was added to dissolve the residue. 2 M hydrochloric acid was slowly added to adjust the pH to about 2. The layers were separated and the aqueous phase was collected. 2 M sodium hydroxide solution was then added to adjust the pH to slightly basic, resulting in the precipitation of a large amount of white solid. The aqueous phase was extracted three times with small amounts of ethyl acetate. The combined organic phases were dried over anhydrous sodium sulfate and concentrated by rotary evaporation to give 147 mg of white solid compound 132 in 86.98% yield. The product was used directly in the next step.

### 4-fluoro-N-((3S,10R,13S)-17-(4-bromo-1H-imidazol-1-yl)-10,13-dimethyl-2,3,4,7,8,9,10,11,12,13,14,15-dodecahydro-1H-cyclopenta[a]phenanthren-3-yl)benzamide (Compound 29)

Using compound 132 (50 mg, 0.12 mmol) as starting material, 45 mg of white solid compound 29 was obtained in 69.55% yield. ¹H NMR (600 MHz, DMSO) δ 8.28 (d, J = 8.0 Hz, 1H), 7.91 - 7.87 (m, 2H), 7.81 (d, J = 1.4 Hz, 1H), 7.52 (d, J = 1.4 Hz, 1H), 7.26 (t, J = 8.8 Hz, 2H), 5.88 (s, 1H), 5.34 (d, J = 4.6 Hz, 1H), 3.68 (ddd, J = 16.3, 12.0, 5.9 Hz, 1H), 1.02 (s, 3H), 0.95 (s, 3H). 13C NMR (151 MHz, DMSO) δ 164.18, 147.23, 141.25, 135.94, 129.86, 129.80, 120.37, 118.99, 117.55, 115.16, 115.02, 114.96, 55.81, 49.78, 49.41, 45.44, 38.41, 37.52, 36.35, 33.67, 30.43, 29.64, 29.16, 28.05, 20.12, 18.95, 15.49. HRMS (ESI) (M+H)+ m/z calcd for C29H34BrFN3O 538.1864, found: 538.1852.

### Example 30:

### N-((3S,10R,13S)-17-(4-chloro-1H-imidazol-1-yl)-10,13-dimethyl-2,3,4,7,8,9,10,11,12,13,14,15-dodecahydro-1H-cyclopenta[a]phenanthren-3-yl)pyrazine-4-carboxamide (Compound 30)

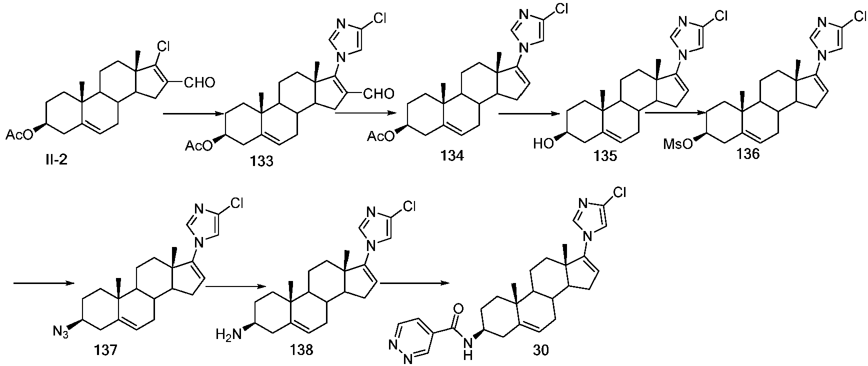

### (3S,10R,13S)-17-(4-chloro-1H-imidazol-1-yl)-16-formyl-10,13-dimethyl-2,3,4,7,8,9,10,11,12,13,14,15-dodecahydro-1H-cyclopenta[a]phenanthren-3-yl acetate (133)

Compound II-2 (4 g, 11.62 mmol) and potassium carbonate (2.4 g, 17.5 mmol) were added to a flask, followed by the addition of anhydrous N,N-dimethylformamide as solvent. The mixture was stirred at room temperature for half an hour, then 4-chloroimidazole (1 g, 9.75 mmol) was added to the system. The temperature was slowly raised to 80°C and stirred overnight at this temperature. The reaction was monitored by TLC until completion. After the reaction was complete, the mixture was extracted with ethyl acetate. The organic phase was collected, washed twice with saturated brine, dried over anhydrous sodium sulfate, filtered, and then concentrated with silica gel. The product was purified by automated column chromatography (10-20% ethyl acetate/petroleum ether) to obtain the desired product. Finally, 1.53 g of yellow solid compound 133 was obtained with a yield of 35.58%. ¹H NMR (400 MHz, CDCl₃) δ 9.76 (s, 1H), 7.50 (d, J = 1.6 Hz, 1H), 7.04 (d, J = 1.6 Hz, 1H), 5.43 (d, J = 5.2 Hz, 1H), 4.63 (ddd, J = 15.9, 10.7, 5.4 Hz, 1H), 2.71 (dd, J = 15.5, 6.3 Hz, 1H), 2.06 (s, 3H), 1.08 (s, 6H).

### (3S,10R,13S)-17-(4-chloro-1H-imidazol-1-yl)-10,13-dimethyl-2,3,4,7,8,9,10,11,12,13,14,15-dodecahydro-1H-cyclopenta[a]phenanthren-3-yl acetate (134)

1,3-bis(diphenylphosphino)propane (0.47 g, 1.14 mmol) and carbonylbis(triphenylphosphine)rhodium(I) chloride (0.47 g, 0.66 mmol) were dissolved in dry xylene. The system was purged with argon three times, then slowly heated to 80°C and stirred for half an hour. Compound 133 (1 g, 2.26 mmol) was added, and the reaction temperature was raised to 135°C and stirred overnight at this temperature. After the reaction was complete, the reaction system was cooled to room temperature, then extracted with ethyl acetate and water. The organic phase was collected, washed twice with saturated brine, dried over anhydrous sodium sulfate, filtered, and then concentrated with silica gel. The product was purified by automated column chromatography (10-20% ethyl acetate/petroleum ether) to obtain the desired product. Finally, 569 mg of white solid compound 134 was obtained with a yield of 60.73%. ¹H NMR (400 MHz, CDCl₃) δ 7.49 (d, J = 1.2 Hz, 1H), 6.97 (d, J = 1.3 Hz, 1H), 5.73 (dd, J = 3.2, 1.7 Hz, 1H), 5.42 (d, J = 5.2 Hz, 1H), 4.67 - 4.57 (m, 1H), 2.05 (s, 3H), 1.08 (s, 3H), 1.00 (s, 3H).

### (3S,10R,13S)-17-(4-chloro-1H-imidazol-1-yl)-10,13-dimethyl-2,3,4,7,8,9,10,11,12,13,14,15-dodecahydro-1H-cyclopenta[a]phenanthren-3-ol (135)

Compound 134 (0.56 g, 1.35 mmol) was dissolved in anhydrous methanol and stirred at room temperature. Potassium hydroxide (183 mg, 3.26 mmol) was added, and the mixture was stirred at room temperature for 2 h. The reaction was monitored by TLC until completion. After the reaction was complete, part of the solvent was removed by rotary evaporation, then extracted with ethyl acetate and water. The organic phase was collected, washed twice with saturated brine, dried over anhydrous sodium sulfate, filtered, and then concentrated by rotary evaporation to obtain the desired product. Finally, 440 mg of white solid compound 135 was obtained with a yield of 87.44%. ¹H NMR (400 MHz, CDCl₃) δ 7.50 (s, 1H), 6.97 (d, J = 1.0 Hz, 1H), 5.74 (dd, J = 3.1, 1.6 Hz, 1H), 5.42 - 5.38 (m, 1H), 3.61 - 3.50 (m, 1H), 1.08 (s, 3H), 1.01 (s, 3H).

### (3S,10R,13S)-17-(4-chloro-1H-imidazol-1-yl)-10,13-dimethyl-2,3,4,7,8,9,10,11,12,13,14,15-dodecahydro-1H-cyclopenta[a]phenanthren-3-yl methanesulfonate (136)

Compound 135 (440 mg, 1.182 mmol) and 4-dimethylaminopyridine (15 mg, 0.12 mmol) were added to a flask. Anhydrous dichloromethane was added as solvent and the mixture was stirred in an ice bath. Triethylamine (0.5 mL, 4.1 mmol) was added and stirred for a while, then methanesulfonyl chloride (0.2 mL, 2.28 mmol) was slowly added. After the addition was complete, the mixture was stirred at room temperature for 2 h. The reaction was monitored by TLC until completion. Ice water was added to quench the reaction, followed by extraction with dichloromethane. The organic phase was washed twice with saturated brine, dried over anhydrous sodium sulfate, filtered, and then concentrated by rotary evaporation to obtain the desired product. Finally, 493 mg of white solid compound 136 was obtained with a yield of 92.67%. ¹H NMR (400 MHz, DMSO) δ 7.83 (d, J = 1.3 Hz, 1H), 7.50 (d, J = 1.3 Hz, 1H), 5.90 (s, 1H), 5.45 (d, J = 3.9 Hz, 1H), 4.43 (d, J = 7.5 Hz, 1H), 3.19 (s, 3H), 1.03 (s, 3H), 0.98 (s, 3H).

### 1-((3S,10R,13S)-3-azido-10,13-dimethyl-2,3,4,7,8,9,10,11,12,13,14,15-dodecahydro-1H-cyclopenta[a]phenanthren-17-yl)-4-chloro-1H-imidazole (137)

Compound 136 (0.486 g, 1.08 mmol) was dissolved in anhydrous dichloromethane and stirred at room temperature. Trimethylsilyl azide (0.5 mL, 3.73 mmol) and boron trifluoride etherate (0.7 mL, 4.84 mmol) were added sequentially via plastic syringe. After addition, the reaction mixture was stirred overnight at room temperature and monitored by TLC until completion. The reaction was then quenched with saturated sodium bicarbonate solution, extracted with dichloromethane, and the organic layer was washed twice with saturated brine. The organic phase was dried over anhydrous sodium sulfate, filtered, concentrated with silica gel and purified by automated column chromatography (5-10% ethyl acetate/petroleum ether) to afford the desired product. Finally, 330 mg of white solid compound 137 was obtained in 76.96% yield. ¹H NMR (400 MHz, CDCl₃) δ 7.47 (s, 1H), 6.94 (d, J = 1.4 Hz, 1H), 5.71 (s, 1H), 5.42 (d, J = 4.6 Hz, 1H), 3.21 (d, J = 4.1 Hz, 1H), 1.05 (s, 3H), 0.99 (s, 3H).

### (3S,10R,13S)-17-(4-Chloro-1H-imidazol-1-yl)-10,13-dimethyl-2,3,4,7,8,9,10,11,12,13,14,15-dodecahydro-1H-cyclopenta[a]phenanthren-3-amine (138)

Compound 137 (330 mg, 0.83 mmol) was dissolved in 10 mL of tetrahydrofuran and 5 mL of water and stirred at room temperature. Triphenylphosphine (326 mg, 1.24 mmol) was added, and the temperature was slowly raised to 60°C. The reaction mixture was stirred overnight at this temperature and monitored by TLC until completion. The solvent was partially removed by rotary evaporation, and 20 mL of ethyl acetate was added to dissolve the residue. The solution was slowly acidified to pH 2 with 2 M hydrochloric acid, and the layers were separated. The aqueous layer was collected and then basified to weakly alkaline with 2 M sodium hydroxide solution, resulting in precipitation of a large amount of white solid. The aqueous phase was extracted three times with small amounts of ethyl acetate. The combined organic phases were dried over anhydrous sodium sulfate and concentrated by rotary evaporation to afford 295 mg of white solid compound 138 in 95.65% yield. ¹H NMR (400 MHz, CDCl₃) δ 7.48 (d, J = 1.5 Hz, 1H), 6.96 (d, J = 1.5 Hz, 1H), 5.72 (dd, J = 3.1, 1.7 Hz, 1H), 5.37 (d, J = 5.3 Hz, 1H), 2.72 - 2.61 (m, 1H), 1.06 (s, 3H), 1.01 (s, 3H).

### N-((3S,10R,13S)-17-(4-Chloro-1H-imidazol-1-yl)-10,13-dimethyl-2,3,4,7,8,9,10,11,12,13,14,15-dodecahydro-1H-cyclopenta[a]phenanthren-3-yl)pyrazine-4-carboxamide (Compound 30)

Using compound 138 (50 mg, 0.136 mmol) as starting material, 49 mg of light yellow solid compound 30 was obtained in 76.23% yield. ¹H NMR (500 MHz, CDCl₃) δ 9.53 (dd, J = 2.0, 1.2 Hz, 1H), 9.42 (dd, J = 5.3, 1.0 Hz, 1H), 8.84 (d, J = 7.8 Hz, 1H), 7.99 (dd, J = 5.3, 2.3 Hz, 1H), 7.83 (d, J = 1.4 Hz, 1H), 7.53 (d, J = 1.4 Hz, 1H), 5.90 (d, J = 1.2 Hz, 1H), 5.38 (d, J = 4.8 Hz, 1H), 3.73 (ddd, J = 15.8, 12.0, 6.1 Hz, 1H), 1.05 (s, 3H), 0.98 (s, 3H). 13C NMR (126 MHz, CDCl₃) δ 167.45, 157.31, 154.05, 152.42, 146.20, 141.17, 136.99, 129.36, 125.92, 124.30, 122.78, 120.16, 61.01, 55.08, 54.98, 50.66, 43.34, 42.63, 41.58, 38.90, 35.62, 34.84, 34.43, 33.04, 25.32, 24.14, 20.70. HRMS (ESI) (M+H)+ m/z calcd for C27H33ClN5O 478.2368, found: 478.2365.

### Example 31:

### 4-Fluoro-N-((3S,10R,13S)-17-(4-chloro-1H-imidazol-1-yl)-10,13-dimethyl-2,3,4,7,8,9,10,11,12,13,14,15-dodecahydro-1H-cyclopenta[a]phenanthren-3-yl)benzatnide (Compound 31)

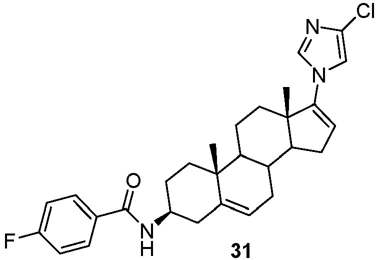

Using compound 138 (50 mg, 0.136 mmol) as starting material, 56 mg of white solid compound 31 was obtained in 84.3% yield. ¹H NMR (500 MHz, CDCl₃) δ 8.29 (d, J = 7.9 Hz, 1H), 7.92 (dd, J = 8.5, 5.6 Hz, 2H), 7.81 (s, 1H), 7.47 (d, J = 1.1 Hz, 1H), 7.28 (t, J = 8.8 Hz, 2H), 5.89 (s, 1H), 5.35 (d, J = 3.4 Hz, 1H), 3.71 (dd, J = 7.6, 4.0 Hz, 1H), 1.04 (s, 3H), 0.97 (s, 3H). 13C NMR (126 MHz, CDCl₃) δ 169.44, 152.41, 146.41, 140.01, 135.00, 133.85, 125.55, 124.18, 120.36, 120.19, 119.56, 61.02, 55.01, 54.72, 50.63, 43.62, 42.76, 41.60, 38.92, 35.63, 34.86, 34.42, 33.26, 25.33, 24.13, 20.68. HRMS (ESI) (M+H)+m/z calcd for C29H34ClFN3O 494.2369, found: 494.2366.

### Example 32:

### N-((3S,10R,13S)-17-(4-Chloro-1H-imidazol-1-yl)-10,13-dimethyl-2,3,4,7,8,9,10,11,12,13,14,15-dodecahydro-1H-cyclopenta[a]phenanthren-3-yl)-4-cyanobenzamide (Compound 32)

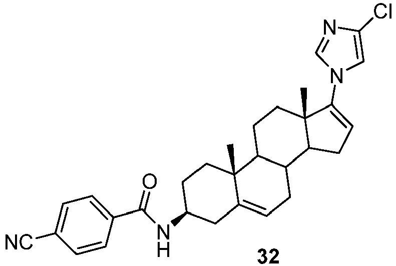

Compound 138 (50 mg, 0.136 mmol) was used to obtain 47 mg of light yellow solid compound 32, with a yield of 69.75%. ¹H NMR (600 MHz, DMSO) δ 8.52 (d, J = 7.9 Hz, 1H), 7.97 (d, J = 8.3 Hz, 2H), 7.92 (d, J = 8.3 Hz, 2H), 7.79 (d, J = 0.9 Hz, 1H), 7.46 (d, J = 0.8 Hz, 1H), 5.86 (s, 1H), 5.33 (d, J = 3.7 Hz, 1H), 3.72 - 3.65 (m, 1H), 1.00 (s, 3H), 0.94 (s, 3H). 13C NMR (151 MHz, DMSO) δ 163.85, 147.28, 141.07, 138.55, 134.79, 132.36, 128.56, 128.09, 120.43, 118.90, 114.34, 113.45, 55.79, 49.75, 49.69, 45.41, 38.26, 37.48, 36.36, 33.67, 30.41, 29.61, 29.20, 27.92, 20.07, 18.92, 15.47. HRMS (ESI) (M+H)+ m/z calculated for C30H34ClN4O 501.2416, measured: 501.2411.

### Example 33: N-((3S,10R,13S)-17-(4-methyl-1H-imidazol-1-yl)-10,13-dimethyl-2,3,4,7, 8,9,10,11,12,13,14,15-dodecahydro-1H-cyclopenta[a]phenanthren-3-yl)-4-fluorobenzamide (Compound 33)

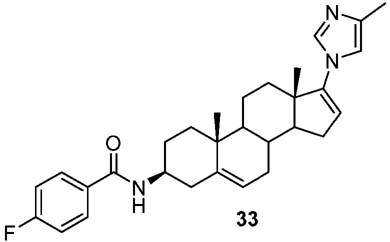

Compound 67 (25 mg, 0.071 mmol) was used to obtain 25 mg of white solid compound 33, with a yield of 74.23%. ¹H NMR (500 MHz, DMSO) δ 8.30 (d, J = 7.9 Hz, 1H), 7.95 - 7.89 (m, 2H), 7.69 (s, 1H), 7.28 (t, J = 8.7 Hz, 2H), 7.05 (s, 1H), 5.74 (s, 1H), 5.37 (s, 1H), 3.72 (d, J = 7.2 Hz, 1H), 2.11 (s, 3H), 1.05 (s, 3H), 0.98 (s, 3H). 13C NMR (126 MHz, DMSO) δ 164.31, 147.82, 141.28, 137.25, 134.77, 131.20, 129.82, 129.75, 120.35, 115.24, 114.94, 114.44, 55.79, 49.79, 49.47, 45.36, 38.38, 37.53, 36.36, 34.06, 30.44, 29.65, 29.12, 28.01, 20.15, 18.90, 15.66, 13.41. HRMS (ESI) (M+H)+ m/z calculated for C30H37FN3O 474.2915, measured: 474.2923.

### Example 34: N-((3S,10R,13S)-17-(4-methyl-1H-imidazol-1-yl)-10,13-dimethyl-2,3,4,7,8,9,10,11,12,13,14,15-dodecahydro-1H-cyclopenta[a]phenanthren-3-yl)-4-nitrobenzatnide (Compound 34)

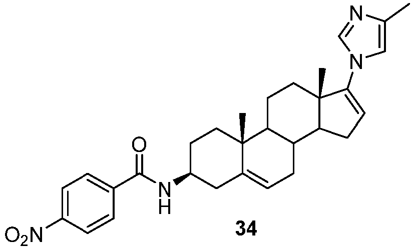

Compound 67 (25 mg, 0.071 mmol) was used to obtain 25 mg of light yellow solid compound 34, with a yield of 70.38%. ¹H NMR (500 MHz, DMSO) δ 8.64 (d, J = 8.0 Hz, 1H), 8.31 (d, J = 8.8 Hz, 2H), 8.08 (d, J = 8.9 Hz, 2H), 7.69 (s, 1H), 7.04 (s, 1H), 5.73 (s, 1H), 5.38 (d, J = 4.6 Hz, 1H), 3.79 - 3.69 (m, 1H), 2.11 (s, 3H), 1.05 (s, 3H), 0.98 (s, 3H). 13C NMR (126 MHz, DMSO) δ 163.69, 148.90, 148.01, 141.09, 140.37, 137.39, 134.73, 128.69, 123.40, 120.57, 116.11, 114.40, 109.50, 55.74, 49.77, 45.35, 38.20, 37.52, 36.40, 34.06, 30.43, 29.64, 29.19, 27.88, 20.14, 18.88, 15.59, 13.42. HRMS (ESI) (M+H)+m/z calculated for C30H37N4O₃ 501.286, measured: 501.2866.

### Example 3 5: N-((3S,10R,13S)-17-(4-methyl-1H-imidazol-1-yl)-10,13-dimethyl-2,3,4,7, 8,9,10,11,12,13,14,15-dodecahydro-1H-cyclopenta[a]phenanthren-3-yl)-4-(methylsulfonyl)benzamide (Compound 35)

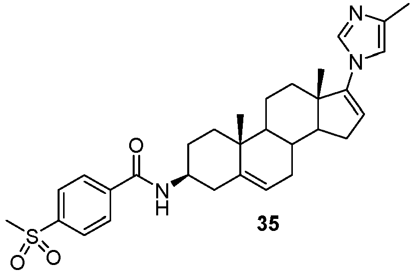

Compound 67 (25 mg, 0.071 mmol) was used to obtain 28 mg of white solid compound 35, with a yield of 73.96%. ¹H NMR (500 MHz, DMSO) δ 8.57 (d, J = 8.0 Hz, 1H), 8.08 (d, J = 8.5 Hz, 2H), 8.02 (d, J = 8.4 Hz, 2H), 7.71 (s, 1H), 7.06 (s, 1H), 5.74 (s, 1H), 5.38 (d, J = 4.4 Hz, 1H), 3.80 - 3.69 (m, 1H), 2.11 (s, 3H), 1.06 (s, 3H), 0.98 (s, 3H). 13C NMR (126 MHz, DMSO) δ 164.07, 147.93, 142.78, 141.21, 139.06, 137.21, 134.76, 128.18, 126.93, 120.43, 116.16, 114.45, 55.79, 49.78, 49.68, 45.36, 43.32, 38.24, 37.48, 36.36, 34.04, 30.44, 29.65, 29.07, 27.92, 20.15, 18.90, 15.60, 13.37. HRMS (ESI) (M+H)+ m/z calculated for C31H40N3OS 534.2785, measured: 534.2786.

### Example 36: N-((3S,10R,13S)-17-(4-methyl-1H-imidazol-1-yl)-10,13-dimethyl-2,3,4,7, 8,9,10,11,12,13,14,15-dodecahydro-1H-cyclopenta[a]phenanthren-3-yl)-4-cyanobenzamide (Compound 36)

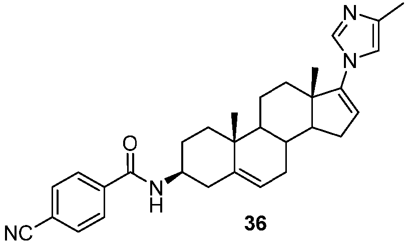

Compound 36 (25 mg, white solid) was obtained from compound 67 (25 mg, 0.071 mmol) in 73.31% yield. ¹H NMR (500 MHz, DMSO) δ 8.56 (d, J = 7.9 Hz, 1H), 8.01 (d, J = 8.4 Hz, 2H), 7.95 (d, J = 8.4 Hz, 2H), 7.71 (s, 1H), 7.05 (s, 1H), 5.74 (s, 1H), 5.37 (d, J = 4.4 Hz, 1H), 3.78 - 3.68 (m, 1H), 2.11 (s, 3H), 1.05 (s, 3H), 0.98 (s, 3H). 13C NMR (126 MHz, DMSO) δ 163.93, 147.91,140.99, 138.72,137.21, 134.72, 132.31,128.05, 120.48, 116.16, 114.43, 113.51, 55.78, 49.77, 49.68, 45.36, 38.22, 37.47, 36.35, 34.11, 30.43, 29.69, 29.12, 27.89, 20.21, 18.88, 15.60, 13.39. HRMS (ESI) (M+H)+ m/z calcd for C31H37N4O 481.2962, found: 481.2959.

### Example 37: N-((3S,10R,13S)-17-(4-methyl-1H-imidazol-1-yl)-10,13-dimethyl-2,3,4,7, 8,9,10,11,12,13,14,15-dodecahydro-1H-cyclopenta[a]phenanthren-3-yl)-2,4-difluorobenzamide (Compound 37)

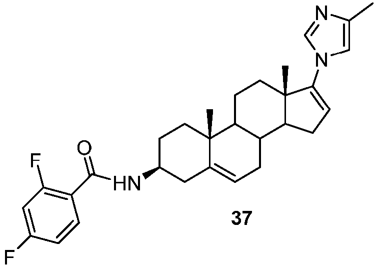

Compound 37 (25 mg, pale yellow solid) was obtained from compound 67 (25 mg, 0.071 mmol) in 71.67% yield. ¹H NMR (500 MHz, DMSO) δ 8.23 (d, J = 7.7 Hz, 1H), 7.69 (s, 1H), 7.64 (dt, J = 15.2, 7.8 Hz, 1H), 7.32 (t, J = 9.9 Hz, 1H), 7.15 (t, J = 7.6 Hz, 1H), 5.74 (s, 1H), 5.38 (d, J = 3.8 Hz, 1H), 3.67 (dd, J = 7.8, 3.9 Hz, 1H), 2.11 (s, 3H), 1.03 (s, 3H), 0.98 (s, 3H). 13C NMR (126 MHz, DMSO) δ 162.59, 148.46, 141.64, 137.69, 135.30, 132.22, 120.85, 116.72, 114.98, 112.19, 112.08, 105.07, 104.85, 104.67, 56.38, 50.22, 50.15, 45.94, 38.69, 37.89, 36.79, 34.61, 30.98, 30.06, 29.68, 28.35, 20.57, 19.47, 16.01, 13.85. HRMS (ESI) (M+H)+ m/z calcd for C30H36F2N3O 492.2821, found: 492.2829.

### Example 38: N-((3S,10R,13S)-17-(4-methyl-1H-imidazol-1-yl)-10,13-dimethyl-2,3,4,7, 8,9,10,11,12,13,14,15-dodecahydro-1H-cyclopenta[a]phenanthren-3-yl)-2,4-difluorobenzamide hydrochloride (Compound 38)

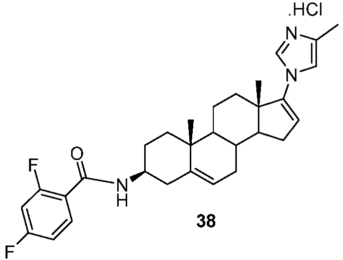

Compound 37 (30 mg, 0.061 mmol) was placed in a flask with dioxane as solvent and stirred at room temperature. A 2 M solution of hydrogen chloride in dioxane was then added. After reacting at room temperature for 2 h, the solvent was directly removed by rotary evaporation to obtain 28 mg of white solid compound 38 in 86.96% yield.

### Example 39:

### N-((3S,10R,13S)-17-(4-methyl-1H-imidazol-1-yl)-10,13-dimethyl-2,3,4,7,8,9,10,11,12,13,14,15-dodecahydro-1H-cyclopenta[a]phenanthren-3-yl)-2,4-difluorobenzamide methanesulfonate (Compound 39)

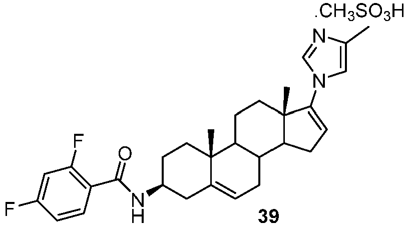

Compound 37 (30 mg, 0.061 mmol) was placed in a flask with dichloromethane as solvent and stirred at room temperature. A small amount of methanesulfonic acid was then slowly added dropwise. After stirring overnight at room temperature, a white solid precipitated. The mixture was filtered through a Buchner funnel and the filter cake was washed several times with ethyl acetate. The filter cake was dried in a vacuum drying oven to finally obtain 30 mg of white solid compound 39 in 83.3% yield.

### Example 40: N-((3S,10R,13S)-10,13-dimethyl-17-(pyridin-3-yl)-2,3,4,7,8,9,10,11,12,13,14,15-dodecahydro-1H-cyclopenta[a]phenanthren-3-yl)cyclopropane-1-sulfonamide (Compound 40)

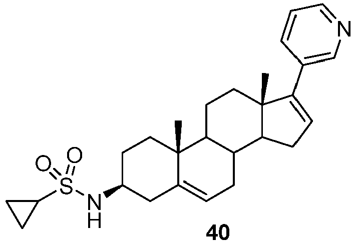

Compound 47 (60 mg, 0.172 mmol) and 4-dimethylaminopyridine (2 mg, 0.015 mmol) were placed in a flask. Anhydrous dichloromethane was added as solvent and the mixture was stirred in an ice bath. Triethylamine (60 µL, 0.42 mmol) was added and stirred for a period of time. Finally, cyclopropylsulfonyl chloride (30 µL, 0.21 mmol) was added and stirred at room temperature for 4 h. The reaction was monitored by TLC until completion. After the reaction was complete, the mixture was extracted with dichloromethane and water. The organic phase was collected, washed twice with saturated brine, dried over anhydrous sodium sulfate, filtered, and concentrated onto silica gel. Purification was performed by automated column chromatography (30-40% ethyl acetate/petroleum ether) to obtain the desired product. Finally, 51 mg of white solid compound 40 was obtained in 65.5% yield. ¹H NMR (400 MHz, DMSO) δ 8.59 (d, J = 1.8 Hz, 1H), 8.44 (dd, J = 4.7, 1.3 Hz, 1H), 7.77 (dd, J = 8.0, 1.8 Hz, 1H), 7.34 (dd, J = 7.9, 4.8 Hz, 1H), 7.10 (d, J = 8.0 Hz, 1H), 6.12 (s, 1H), 5.37 (d, J = 4.6 Hz, 1H), 3.01 (d, J = 7.0 Hz, 1H), 1.02 (s, 3H), 1.00 (s, 3H). 13C NMR (126 MHz, DMSO) δ 151.48,148.27, 147.62, 141.56, 133.76, 132.59, 129.46, 123.83, 121.22, 57.47, 53.88, 50.17, 47.11, 37.95, 36.62, 35.11, 31.75, 31.36, 31.13, 30.36, 30.25, 20.82, 19.36, 16.71, 5.48. HRMS (ESI) (M+H)+ m/z calcd for C27H37N2O₂S 453.257, found: 453.258.

### Example 41:

### N-((3S,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl)morpholine-4-sulfonamide (Compound 41)

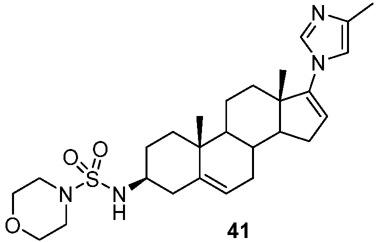

Compound 67 (50 mg, 0.142 mmol) was used to obtain 46 mg of white solid compound 41, yield: 64.7%. ¹H NMR (500 MHz, DMSO) δ 7.68 (s, 1H), 7.34 (d, J = 7.3 Hz, 1H), 7.04 (s, 1H), 5.73 (s, 1H), 5.36 (d, J = 4.2 Hz, 1H), 3.66 - 3.60 (m, 4H), 2.99 (m, 4H), 2.93 (m, 1H), 2.10 (s, 3H), 0.99 (s, 3H), 0.97 (s, 3H). 13C NMR (126 MHz, DMSO) δ 148.33, 141.53, 137.80, 135.30, 121.11, 116.73, 114.87, 65.96, 56.27, 54.26, 50.20, 46.36, 45.85, 37.89, 36.66, 34.49, 32.00, 30.88, 30.09, 29.90, 29.80, 29.60, 29.19, 20.62, 19.31, 16.09, 13.92. HRMS (ESI) (M+H)+ m/z calculated for C27H41N4O₃S 501.2894, measured: 501.2904.

### Example 42:

### N-((3R,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl)-4-fluorobenzamide (Compound 42)

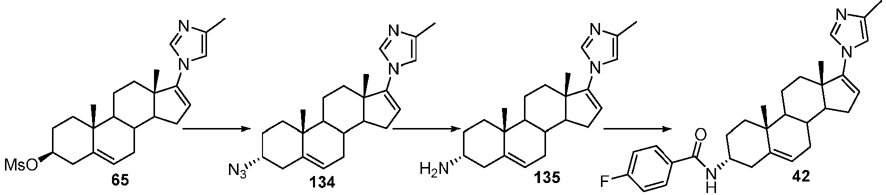

### 1-((3R,10R,13S)-3-azido-10,13-dimethyl-2,3,4,7,8,9,10,11,12,13,14,15-dodecahydro-1H-cyclopenta[a]phenanthren-17-yl)-4-methyl-1H-imidazole (134)

Compound 65 (1.5 g, 3.48 mmol) and 15-crown-5 (77 mg, 0.35 mmol) were dissolved in anhydrous DMF, then sodium azide (0.68 g, 10.44 mmol) was added. After addition, the reaction system was slowly heated to 80°C and stirred for 18 hours at this temperature. The reaction was monitored by TLC until completion. The reaction system was then cooled to room temperature, and a certain amount of ice water was added. The mixture was extracted with dichloromethane, and the organic phase was washed twice with saturated brine. After drying over anhydrous sodium sulfate, the mixture was filtered. Silica gel was added to the organic phase, concentrated, and purified by automated column chromatography (15-30% ethyl acetate/petroleum ether) to obtain the desired product. Finally, 0.63 g of light yellow solid 134 was obtained, yield: 48.09%. ¹H NMR (400 MHz, DMSO) δ 8.06 (s, 1H), 6.84 (s, 1H), 5.92 - 5.88 (m, 1H), 5.42 (d, J = 4.9 Hz, 1H), 3.27 - 3.17 (m, 1H), 2.39 (s, 3H), 1.05 (s, 3H), 1.01 (s, 3H).

### (3R,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-amine (135)

Compound 134 (2 g, 5.3 mmol) was dissolved in 16 mL of tetrahydrofuran and 8 mL of water and stirred at room temperature. Then, pre-weighed triphenylphosphine (2.08 g, 7.95 mmol) was added. The temperature was slowly raised to 60°C and stirred overnight at this temperature. The reaction was monitored by TLC until completion. Then, part of the solvent was removed by rotary evaporation, 20 mL of dichloromethane was added to dissolve, and 2 M hydrochloric acid was slowly added to adjust the pH of the solution to about 2. The layers were separated and the aqueous phase was collected. Then 2 M sodium hydroxide solution was added to adjust the pH to weakly alkaline, at which point a large amount of white solid precipitated. The aqueous phase was extracted three times with a small amount of dichloromethane. The organic phases were combined, dried over anhydrous sodium sulfate, and the solvent was removed by rotary evaporation to obtain 664 mg of white solid 135, yield 36.08%. ¹H NMR (400 MHz, CDCl₃) δ 7.53 (s, 1H), 6.78 (s, 1H), 5.63 (dd, J = 3.0, 1.7 Hz, 1H), 5.37 (d, J = 5.2 Hz, 1H), 2.25 (s, 3H), 1.05 (s, 3H), 1.01 (s, 3H).

### N-((3R,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl)-4-fluorobenzamide (Compound 42)

Compound 135 (100 mg, 0.284 mmol) was used to obtain 86 mg of white solid compound 42, yield: 63.7%. ¹H NMR (400 MHz, DMSO) δ 8.98 (d, J = 1.7 Hz, 1H), 8.67 (dd, J = 4.8, 1.5 Hz, 1H), 8.49 (d, J = 7.9 Hz, 1H), 8.18 - 8.14 (m, 1H), 7.73 (s, 1H), 7.48 (dd, J = 7.9, 4.9 Hz, 1H), 7.05 (s, 1H), 5.73 (s, 1H), 5.36 (d, J = 4.0 Hz, 1H), 3.77 - 3.65 (m, 1H), 2.09 (s, 3H), 1.03 (s, 3H), 0.96 (s, 3H). 13C NMR (126 MHz, DMSO) δ 163.90, 151.73, 148.40, 141.15, 137.05, 134.95, 134.73, 123.31, 120.51, 116.43, 114.56, 55.84, 49.81, 49.50, 45.37, 38.36, 37.53, 36.40, 34.09, 30.38, 29.68, 29.17, 28.01, 20.13, 18.95, 15.64, 13.35. HRMS (ESI) (M+H)+ m/z calculated for C30H37FN3O 474.2915, measured: 474.2916.

### Example 43:

### N-((3R,10R,13S)-10,13-dimethyl-17-(4-methyl-1H-imidazol-1-yl)-2,3,4,7,8,9,10,11,12,13,14,15-dodecahydro-1H-cyclopenta[a]phenanthren-3-yl)-2,4-difluorobenzamide (Compound 43)

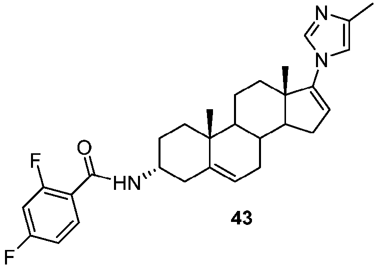

Compound 135 (100 mg, 0.028 mmol) was used to obtain 94 mg of pale yellow solid compound 43, with a yield of 67.14%. ¹H NMR (500 MHz, DMSO) δ 8.20 (d, J = 7.9 Hz, 1H), 7.67 (d, J = 1.1 Hz, 1H), 7.63 (td, J = 8.5, 6.8 Hz, 1H), 7.34 - 7.28 (m, 1H), 7.14 (td, J = 8.4, 2.1 Hz, 1H), 7.03 (s, 1H), 5.73 - 5.71 (m, 1H), 5.37 (d, J = 4.8 Hz, 1H), 3.70 - 3.61 (m, 1H), 2.09 (d, J = 0.4 Hz, 3H), 1.01 (s, 3H), 0.96 (s, 3H). 13C NMR (126 MHz, DMSO) δ 162.42, 148.46, 141.53, 137.82, 135.16, 132.06, 120.97, 116.59, 114.89, 112.20, 111.96, 104.98, 104.93, 104.72, 56.29, 50.27, 50.03, 45.85, 38.74, 37.93, 36.85, 34.61, 30.93, 30.05, 29.51, 28.49, 20.65, 19.39, 16.11, 13.94. HRMS (ESI) (M+H)+ m/z calculated for C30H36F2N3O 492.2821, measured: 492.2837.

### Biological Activity Tests

A series of in vitro and in vivo activity tests were conducted to demonstrate that the steroidal compounds of the present invention are suitable for treating diseases mediated by the AR signaling pathway. The in vitro activity tests include: wild-type AR antagonist activity test, mutant AR antagonist activity test, AR degradation activity test, and cell proliferation inhibition activity test. The in vivo activity test is the androgen-dependent organ development (Hershberger) test, as well as other druggability tests such as metabolism.

### 1. Wild-type AR Antagonist Activity Test

HEK293 cells were cultured in DMEM containing 10% fetal bovine serum (FBS) at 37°C with 5% CO₂. The cells were then digested with trypsin and counted. A 10 µL/well transfection solution was prepared in optiMEM containing 5 ng androgen receptor clone, 100 ng pGL4.36 vector, and 315 nL fugene. The cell suspension was diluted with phenol red-free DMEM supplemented with 10% dialyzed FBS and 1% GlutaMax, and mixed with the transfection solution to achieve 444,444 cells/mL. The diluted cell suspension was seeded into 96-well plates at 90 µL (40,000 cells/well), and the plates were incubated for 24 hours. Then, 10 µL of drug solution (including gradient diluted compounds or DMSO) was added, and the plates were incubated for 30 minutes. 10 nL of DHT (final concentration 1.5 nM) was added to each well of the plate, and the plates were incubated at 37°C with 5% CO₂. After 24 hours of incubation, 100 µL of Steady-Glo was added to each well of the plate, the plate was shaken at room temperature for 20 minutes, and read on an Envision. The IC50 is defined as the drug concentration that causes 50% inhibition of luciferase expression, as determined by the dose-response curve.

Using the second-generation AR antagonist enzalutamide as a positive control drug, the AR antagonist activity of representative compounds is shown in Table 1:

**Table 1. AR Antagonist Activity of Representative Compounds**

| Compound | IC₅₀/nM |
|---|---|
| **13** | 244 |
| **14** | 246.6 |
| **33** | 120.5 |
| **34** | 70.56 |
| **35** | 433.4 |
| **37** | 235 |
| Enzalutamide | 440.2 |

The test results in Table 1 show that the compounds of the present invention have excellent AR antagonist activity and can antagonize/block the AR receptor signaling pathway. The AR antagonist activity of representative compounds 34, 33, 37, 13, and 14 is 6.2, 3.6, 1.9, 1.8, and 1.8 times higher than that of the control enzalutamide, respectively.

### 2. In Vitro AR Degradation Activity Test

LNCaP cells (prostate cancer cells) were cultured in RPMI-1640 supplemented with 10% fetal bovine serum (FBS) at 37 °C with 5% CO₂. The cells were then digested with trypsin and counted using an automated cell counter. The cell suspension was diluted to the desired density in growth medium. The cell suspension was then seeded into 96-well plates at 100 µL per well, and the plates were incubated at 37 °C with 5% CO₂ for 24 hours. The compounds were then diluted 200-fold to the final concentration with DMSO or 3-fold to the final concentration with culture medium. 50 µL of compound solution was transferred to the 96-well plate and incubated at 37 °C for 24 hours. After 24 hours of incubation, the cell plate was washed 3 times with DPBS, 100 µL of 1× lysis buffer was added to each well, and placed on ice for 30 minutes. The supernatant was collected. The protein in the cell lysate samples was quantified and then diluted to the required concentration. 100 µL of cell lysate sample was added to the appropriate wells, sealed with tape and pressed firmly on top of the wells, and the plate was incubated at 37 °C for 2 hours. The plate contents were then discarded, and washed 4 times with 1× wash buffer, 200 µL per well each time. 100 µL of reconstituted detection antibody was then added, the plate was sealed with tape and incubated at 37°C for 1 hour. The aspiration/wash step was then repeated, 100 µL of reconstituted HRP-Linked secondary antibody was added to each well, the plate was sealed with tape and incubated at 37 °C for 30 minutes. The aspiration/wash step was then repeated, 100 µL of substrate solution was added to each well and incubated at 37 °C for 10 minutes, and finally 100 µL of stop solution was added to each well. The plate was gently tapped to ensure thorough mixing, and the OD450nm was recorded.

Degradation rate (%) = (OD450_max - OD450_sample) / (OD450_max - OD450_min) × 100, plotted using GraphPad Prism 5 software, and IC₅₀ is calculated using SPSS software.

Since enzalutamide only has AR antagonist activity and does not have AR degradation activity, Galeterone (CAS: 851983-85-2), which has the same steroidal structure as the compounds of the present invention, has entered clinical studies for the treatment of CRPC. Galeterone has a triple mechanism of action: CYP17A1 enzyme inhibition, AR degradation, and antagonism (J. Med. Chem. 2015, 58, 2077-2087). Therefore, using Galeterone as a positive control drug, the AR degradation activity of representative compounds of the present invention was tested, and the results are shown in Table 2.

**Table 2. AR Degradation Activity of Representative Compounds**

| Compound | DC₅₀/µM |
|---|---|
| **3** | 2.87 |
| **8** | 3.76 |
| **10** | 4.25 |
| **12** | 3.58 |
| **13** | 2.08 |
| **16** | 2.76 |
| **17** | 1.98 |
| **18** | 3.21 |
| **21** | 3.31 |
| **24** | 3.88 |
| **33** | 1.28 |
| **34** | 2.60 |
| **35** | 1.56 |
| **36** | 1.37 |
| **37** | 1.68 |
| **Galeterone** | 10.41 |

The test results in Table 2 show that the steroidal compounds of the present invention exhibit excellent in vitro AR degradation activity, and are significantly superior to the control drug Galeterone, with a 2.4-8.1 fold significant improvement. Among them, compounds 33, 36, 35, and 37 showed improvements of 8.1, 7.6, 6.7, and 6.2 times compared to Galeterone, respectively.

### 3. In Vitro LNCaP (Hormone-Sensitive Prostate Cancer) Cell Proliferation Inhibition Test

LNCaP cells were cultured in RPMI-1640 medium supplemented with 10% fetal bovine serum (FBS) at 37°C with 5% CO₂. The cells were then digested with trypsin and counted using an automated cell counter to determine cell density. The cell suspension was diluted to the desired density in growth medium. Subsequently, 100 µL of the cell suspension was seeded into 96-well plates and incubated at 37°C with 5% CO₂ for 24 hours. 10 µL of drug solution (including 3-fold serial dilutions of compounds or DMSO) was added to each well, and the plates were incubated at 37°C with 5% CO₂ for 6 days. Prior to measurement, the assay plates were equilibrated to room temperature. 40 µL of CellTiter-Glo^{®} reagent was added to each well. The plates were mixed on an orbital shaker for 2 minutes to induce cell lysis. After incubation at room temperature for 60 minutes to stabilize the luminescence signal, the plates were read on an Envision plate reader. The IC₅₀ was defined as the drug concentration causing 50% cell growth inhibition, as determined by the dose-response curve.

Using abiraterone and enzalutamide, which are currently used clinically for the treatment of advanced prostate cancer, as positive controls, the anti-proliferative activity of representative compounds of the present invention against LNCaP prostate cancer cells was determined. The results are shown in Table 3.

**Table 3. Anti-proliferative activity of representative compounds against LNCaP cells**

| Compound | IC₅₀/nM | Maximum Inhibition (%) |
|---|---|---|
| **2** | 851.1 | 93.50 |
| **8** | 763 | 104.5 |
| **9** | 765.2 | 95.87 |
| **10** | 819.7 | 99.74 |
| **12** | 932.6 | 102.9 |
| **13** | 466 | 96.97 |
| **14** | 205.1 | 99.35 |
| **15** | 752.5 | 102.6 |
| **16** | 382.5 | 92.41 |
| **17** | 317.2 | 96.99 |
| **18** | 355.7 | 98.53 |
| **19** | 427.1 | 97.68 |
| **20** | 734.5 | 94.04 |
| **22** | 818.7 | 104.1 |
| **24** | 475 | 98.02 |
| **25** | 470.9 | 89.52 |
| **33** | 477.9 | 102.9 |
| **35** | 526.7 | 96.85 |
| **36** | 642.8 | 99.38 |
| **37** | 327.2 | 95.85 |
| Abiraterone | 2803 | 84.06 |
| Enzalutamide | 460.9 | 55.16 |

The test results in Table 3 demonstrate that the compounds of the present invention exhibit excellent anti-proliferative activity (IC50) against prostate cancer cells. Their anti-tumor activity is significantly superior to abiraterone, with a 3.0-15.8 fold improvement compared to the latter. Among them, compounds 14, 17, 37, and 18 showed 15.8, 8.8, 8.6, and 7.9 fold improvements, respectively, compared to abiraterone.

The most notable characteristic of the compounds in this invention compared to enzalutamide is their significantly improved anti-tumor efficacy (maximum inhibition activity) by approximately 2-fold. This is attributed to the strong AR degradation/downregulation ability of the compounds in this invention, while enzalutamide lacks AR degradation activity. Compounds 14, 16-19, and 37 exhibit significantly superior anti-tumor activity and efficacy compared to enzalutamide, while compounds 13, 24, and 33 show comparable anti-tumor activity but significantly superior anti-tumor efficacy compared to enzalutamide.

It is evident that the steroidal compounds of the present invention possess significantly superior anti-tumor activity compared to existing AR antagonists or androgen synthesis inhibitors.

### 4. Mutant AR antagonistic activity test

First-generation AR antagonists such as flutamide and bicalutamide, and second-generation AR antagonists like enzalutamide and apalutamide, all lead to acquired/secondary resistance due to mutations in the AR ligand-binding domain after a period of clinical treatment. The compounds of the present invention, due to their unique dual function of degradation/antagonism, still exhibit strong antagonistic activity against various mutant AR receptors and will remain effective for patients with tumors resistant to enzalutamide and other drugs in clinical practice.

HEK293 cells were cultured in DMEM supplemented with 10% FBS and 1% GlutaMax at 37°C with 5% CO₂. The day before transfection, the medium was changed to DMEM containing 10% charcoal-stripped FBS and 1% GlutaMax. Androgen-responsive reporter gene constructs (pGL 4.36) encoding AR mutants AR(F876L), AR(T877A), or AR(W741L) were transfected using Lipofectamine 2000 in OptiMEM and incubated at room temperature for 15 minutes. The cell suspension was diluted with cell seeding solution, and the transfection reagent was added to achieve 500,000 cells/mL. Subsequently, 90 µL of cell suspension was seeded into each well of the assay plate. The next day, compounds were prepared: starting from 8 mM, 3-fold serial dilutions were performed in DMSO for a total of 8 points, then 500 nL was transferred to the compound plate using Echo. The compounds were then diluted with 40 µL of medium to 10x final concentration, and 10 µL was transferred to the cell plate and incubated at 37°C for 30 minutes. Subsequently, 10 nL of DHT at a final concentration of 10 nM was transferred to each well of the assay plate using TECAN. After 24 hours of incubation, 100 µL of Steady - Glo was added to the assay plate and read on an Envision reader. Data were determined and analyzed using GraphPad Prism.

For W741L and T877A mutations, the second-generation AR antagonist enzalutamide was used as a positive control. For the F876L mutation, enzalutamide exhibited agonistic activity. The test results are shown in Table 4.

**Table 4. Antagonistic activity of representative compounds against mutant AR**

| Compound | W741L IC₅₀(nM) 1st gen AR antagonist resistant mutation | T877A IC₅₀(nM) 1st gen AR antagonist resistant mutation | F876L IC₅₀(nM) 2nd gen AR antagonist resistant mutation |
|---|---|---|---|
| **14** | 490.2 | 208.8 | 268.2 |
| **33** | 371.3 | 79.75 | 389.8 |
| **35** | 258.8 | 462.5 | 326.1 |
| **37** | 413.1 | 280.9 | 273.4 |
| Enzalutamide | 1031 | 112.3 | Agonist |

The test results in Table 4 demonstrate that the compounds of the present invention exhibit excellent antagonistic activity against the major clinically occurring mutant ARs: For the W741L mutation, compound 35 showed a 4.0-fold improvement in activity compared to enzalutamide; for the T877A mutation, compound 33 showed a 1.4-fold improvement compared to enzalutamide; for the F876L mutation, which is resistant to second-generation AR antagonists enzalutamide and apalutamide, the compounds of the present invention all exhibited excellent antagonistic activity. This indicates that the compounds of the present invention can treat prostate cancer resistant to existing first- and second-generation AR antagonists.

### 5. Anti-proliferative activity against resistant cells (22RV1)

Another major form of acquired/secondary resistance of AR is the expression of the AR-V7 splice variant. Approximately 50% of patients who have received enzalutamide or abiraterone treatment express this splice variant. The ligand-binding domain of this AR splice variant is completely absent, rendering all current AR antagonists unable to bind to AR and exert their effects, leading to secondary resistance or natural resistance inactivation. Due to the excellent AR degradation activity of the steroidal compounds of the present invention, they can also degrade AR splice variants, potentially solving the problem of resistance caused by splice variants.

The antiproliferative activity of the compounds of the present invention against 22RV1 prostate cancer cells expressing the AR-V7 splice variant was tested, using all marketed second-generation AR antagonists enzalutamide, apalutamide and darolutamide as controls. The experimental procedure was essentially the same as that for LNCaP cells. The results are shown in Table 5.

**Table 5. Antiproliferative activity of representative compounds against 22RV1 cells**

| Compound | IC₅₀/nM |
|---|---|
| **14** | 590.6 |
| **33** | 623.8 |
| **35** | 650.7 |
| **37** | 451.9 |
| Enzalutamide | >10000 |
| Apalutamide | >10000 |
| Darolutamide | >10000 |

The test results in Table 5 show that, as expected, enzalutamide, apalutamide and darolutamide have no activity against tumor cells expressing AR splice variants, while the steroidal compounds of the present invention exhibit excellent antiproliferative activity against 22RV1 prostate cancer cells and can be used for the treatment of patients resistant to AR splice variants.

Taken together, the results of experiments 1-5 above demonstrate that the compounds of the present invention have strong antitumor activity against prostate cancers that are either sensitive or resistant to existing AR antagonists (first and second generation) and abiraterone, and can be used for the treatment of prostate cancer at all stages, including early hormone-sensitive prostate cancer, CRPC and resistant CRPC. This will provide an unprecedented powerful therapy for prostate cancer and other diseases related to the AR signaling pathway.

### 6. In vivo metabolism of compounds of the present invention

Male SD rats were randomly divided into 6 groups, 3 rats per group. The rats were fasted but not water-deprived for 12-14 h one day before dosing. The test samples were then dissolved in a solvent of 5% DMSO + 30% PEG400 + 65% (0.5% MC) and administered by gavage at 10 mg/kg. Food was provided 4 h after dosing. Blood samples (0.10 mL) were collected via the orbital sinus at 0, 15, 30 min, 1, 2, 4, 6, 8, and 24 h, with EDTA-K2 as anticoagulant. Blood samples were placed on ice after collection and centrifuged to separate plasma within 30 minutes (centrifugation conditions: 5000 rpm, 10 minutes, 4°C). 50.0 µL of plasma sample (taken from -80°C freezer, thawed naturally at room temperature and vortexed for 30 seconds) was added to a 1.5 mL centrifuge tube, followed by addition of 500 µL internal standard solution (methanol (TEDIA, HPLC grade), acetonitrile (TEDIA, HPLC grade), formic acid (FA, ACROS ORGANICS, HPLC grade), water was laboratory-made ultrapure water (100.0 ng·mL-1)). The mixture was vortexed for 60 seconds and centrifuged for 3 minutes (centrifugal force 12000 rpm). 100 µL of the supernatant was transferred to a 96-well injection plate containing an equal volume of water, mixed by shaking, and analyzed by LC-MS/MS with an injection volume of 10 µL. The data acquisition and control system software was Analyst 1.5.1 software (Applied Biosystem). The chromatographic sample peaks were integrated automatically. The ratio of sample peak area to internal standard peak area was used as an indicator for regression with sample concentration.

The pharmacokinetic parameters of representative compounds of the present invention were tested in male SD rats after oral gavage administration at 10 mg/kg according to the above conventional method to examine the pharmacokinetic characteristics of the compounds of the present invention in rats. The test results are shown in Table 6:

**Table 6. In vivo pharmacokinetic activity test results in rats (PO: 10 mg/kg)**

| PK parameters | | **ABT** | **16** | **14** | **17** | **33** | **37** |
|---|---|---|---|---|---|---|---|
| Dose | mg·kg⁻¹ | 10.0 | | | | | |
| Kₑₗ | h⁻¹ | 0.298 | 0.296 | 0.252 | 0.112 | 0.214 | 0.124 |
| T_{1/2} | h | 2.37 | 2.36 | 2.80 | 10.0 | 3.28 | 6.02 |
| tₘₐₓ | h | 1.83 | 4.00 | 2.17 | 2.00 | 4.00 | 6.00 |
| Cₘₐₓ | ng·mL⁻¹ | 48.4 | 3241 | 2670 | 4504 | 6431 | 1552 |
| AUC₀₋ₜ | h·ng·mL⁻¹ | 163 | 37419 | 24800 | 28824 | 68872 | 22542 |
| AUC_{0-inf} | h·ng·mL⁻¹ | 198 | 37489 | 24847 | 65578 | 69484 | 24470 |
| MRT_{PO} | h | 4.60 | 6.25 | 5.12 | 15.5 | 6.03 | 10.0 |

The in vivo pharmacokinetic experiments in rats show that compared to the steroidal compound abiraterone, these compounds exhibit excellent pharmacokinetic properties and druggability. Compared to abiraterone (ABT), Cmax increased by 32-133 fold, and drug exposure increased by more than 138 fold. The metabolic properties are significantly better than abiraterone, with very rational druggability.

### 7. In vivo anti-androgenic activity

The Hershberger assay was used to detect the in vivo anti-androgenic signal transduction activity of the compounds of the present invention. In this assay, compounds of the present invention (14, 33 and 37) were administered to prepubertal castrated male Sprague-Dawley rats in the presence of 0.4 mg/kg testosterone propionate (TP), and the weights of androgen-dependent organs were measured. Dosing continued for 10 days, and measurements were made 24 hours after the last dose. The degree of AR antagonism and subsequent organ growth inhibition was evaluated by comparison with castrated controls. The test compounds were orally administered at 20 mg/kg QD and the endpoint evaluation was performed by measuring the weight changes of 2 androgen-sensitive organs (ASO): seminal vesicles with coagulating glands (SVCG) and ventral prostate (VP). Enzalutamide (ENZ) was used as a positive control. The test results are shown in Figure 2.

The in vivo Hershberger assay showed that compounds 14, 33 and 37 significantly inhibited the growth of androgen-dependent organs compared to the castration group, and all exhibited activity comparable to enzalutamide. This demonstrates that the compounds of the present invention can still effectively antagonize the androgen receptor signaling pathway in vivo for the treatment of prostate cancer.

### 8. Activity of compounds of the present invention in inhibiting the expression of transmembrane serine protease 2 (TMPRSS2) and angiotensin-converting enzyme 2 (ACE2)

To verify whether AR antagonists or AR degraders can effectively downregulate the expression of TMPRSS2 and ACE2 proteins for the treatment of COVID-19, A549 lung cancer cells were used. Cells in logarithmic growth phase were collected, counted, and resuspended in complete culture medium to adjust the cell concentration to 3×105 cells/well. 1 mL of cell culture medium was added to each well of a 12-well plate. The cells were cultured in a 37°C, 100% relative humidity, 5% CO₂ incubator for 24 hours. The culture medium in the culture dish containing A549 cells was then discarded and washed once with PBS. The compounds of this patent and the positive drug proxalutamide (concentration 10 µM), 1 nM DHT, were mixed with culture medium and added to the culture dish, then incubated in a 37°C, 100% relative humidity, 5% CO₂ incubator for 48 h. After 48 h, cells were collected and RNA was extracted from A549 cells according to the RNA extraction kit. cDNA was obtained by reverse transcription according to the instructions of the reverse transcription kit. The effects of different candidate compounds on the mRNA expression levels of ACE2 protein and TMPRSS2 protein in A549 cells were detected by qPCR method. The test results are shown in Table 7.

**Table 7. mRNA expression levels of ACE2 protein and TMPRSS2 protein in A549 cells treated with compounds**

| Compound | TMPRSS2 mRNA level | ACE2 mRNA level |
|---|---|---|
| **Blank** | 1 | 1 |
| **21** | 0.196 | 0.234 |
| **35** | 0.394 | 0.102 |
| **Proxalutamide** | 0.608 | 0.463 |

Table 7 test results show that in A549 cells, compounds 21 and 35 can significantly downregulate the expression of ACE2 and TMPRSS2 proteins, and are superior to the control drug proxalutamide. In vitro protein expression experiments confirm that the compounds of the present invention can effectively inhibit the AR-TMPRSS2/ACE2 signaling pathway and can be used for the treatment of COVID-19 patients.

All of the above test results indicate that the compounds of the present invention will be a new generation of anti-prostate cancer drugs with both AR antagonist and degradation dual mechanisms of action, exhibiting stronger anti-tumor activity. Compared to single-function AR antagonists or androgen synthesis enzyme inhibitors, they demonstrate better efficacy and will have improved therapeutic effects for clinical CRPC patients and prostate cancer patients at all stages. In the future, they can be better used for the treatment of diseases related to the AR signaling pathway.

## Claims

1. A compound of formula (I), or an enantiomer, diastereoisomer, stereoisomer, prodrug, deuterated compound, hydrate, solvate, racemate or pharmaceutically acceptable salt thereof, 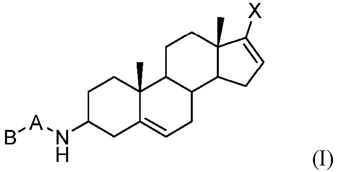
wherein A is 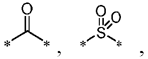 -SO- or C1-C3 alkylene;
B is hydrogen, substituted or unsubstituted C₁₋₆ alkyl, substituted or unsubstituted C₃₋₈ cycloalkyl, substituted or unsubstituted 4- to 8-membered heterocycloalkyl, substituted or unsubstituted C₆₋₁₀ aryl, substituted or unsubstituted 5- to 10-membered heteroaryl;
X is substituted or unsubstituted 5- to 10-membered heteroaryl, or substituted or unsubstituted 4- to 8-membered heterocycloalkyl;
each of the substitutions independently refers to being substituted by one or more groups selected from the group consisting of deuterium, hydroxyl, carboxyl, amino, mercapto, C₁₋₆ alkyl, C₁₋₆ alkoxy, sulfonyl, halogen, cyano, NO₂, C₁₋₆ haloalkyl, -SO-C₁₋₆ alkyl, -SO₂-C₁₋₆ alkyl, -CONH-C₁₋₆ alkyl, -NHCO-C₁₋₆ alkyl, and C₃₋₆ cycloalkyl;
the heteroatom of the heterocycloalkyl and heteroaryl is O, N or S, and the number of heteroatoms is 1, 2, 3 or 4.

2. The compound of claim 1, wherein A is 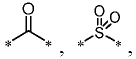 -SO-, -CH₂-, -CH₂CH₂- or - CH₂CH₂CH₂-.

3. The compound of claim 1, wherein B is substituted or unsubstituted C₁₋₄ alkyl, substituted or unsubstituted C₃₋₆ cycloalkyl, substituted or unsubstituted 5- to 6-membered heterocycloalkyl, substituted or unsubstituted phenyl, substituted or unsubstituted 5- to 7-membered heteroaryl; said substitution refers to being substituted by 1, 2 or 3 groups selected from the group consisting of deuterium, hydroxyl, carboxyl, amino, mercapto, C₁₋₄ alkyl, C₁₋₄ alkoxy, sulfonyl, F, Cl, Br, CN, NO₂, C₁₋₄ haloalkyl, -SO-C₁₋₄ alkyl, -SO₂-C₁₋₄ alkyl, - CONH-C₁₋₄ alkyl, -NHCO-C₁₋₄ alkyl, and C₃₋₆ cycloalkyl; the heteroatom of the heterocycloalkyl and heteroaryl is O, N or S, and the number of heteroatoms is 1, 2 or 3.

4. The compound of claim 1, wherein X is selected from: substituted or unsubstituted 5- to 9-membered heteroaryl, substituted or unsubstituted 4- to 6-membered heterocycloalkyl; the substituents are 1, 2 or 3 groups selected from the following group: deuterium, hydroxyl, carboxyl, amino, mercapto, C₁₋₄ alkyl, C₁₋₄ alkoxy, sulfonyl, F, Cl, Br, CN, NO₂, C₁₋₄ haloalkyl, -SO-C₁₋₄ alkyl, -SO₂-C₁₋₄ alkyl, -CONH-C₁₋₄ alkyl, -NHCO-C₁₋₄ alkyl, C₃₋₆ cycloalkyl; the heteroatom of the heterocycloalkyl and heteroaryl is O, N or S, and the number of heteroatoms is 1, 2, 3 or 4.

5. The compound of claim 1, wherein the compound or a pharmaceutically acceptable salt thereof is selected from the group consisting of

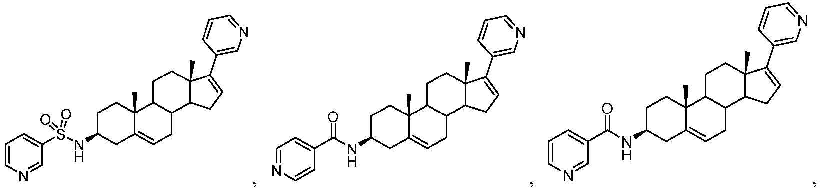

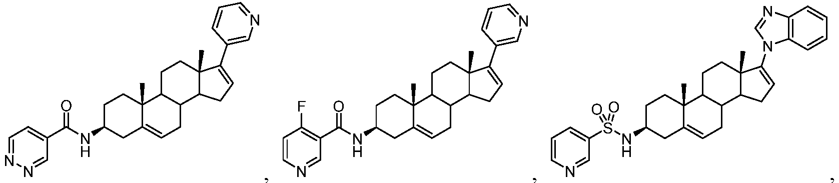

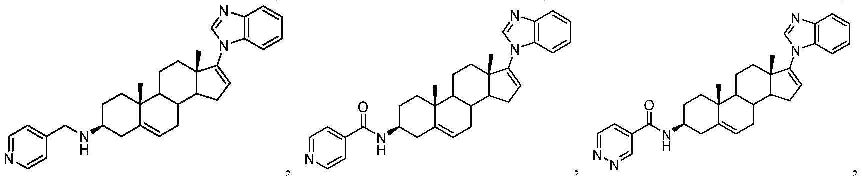

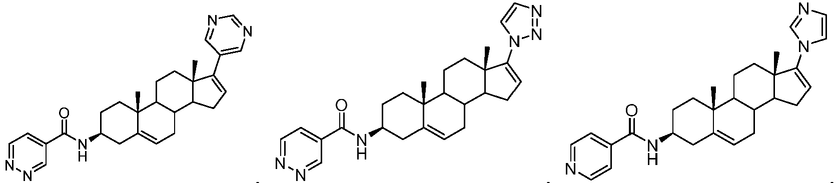

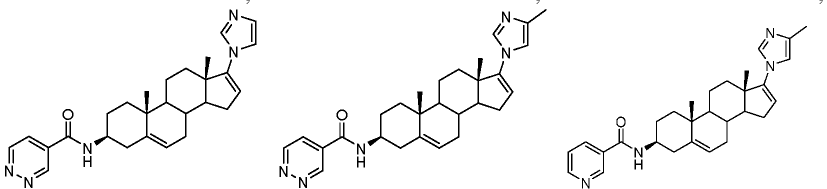

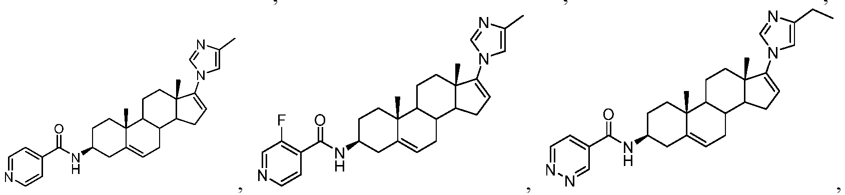

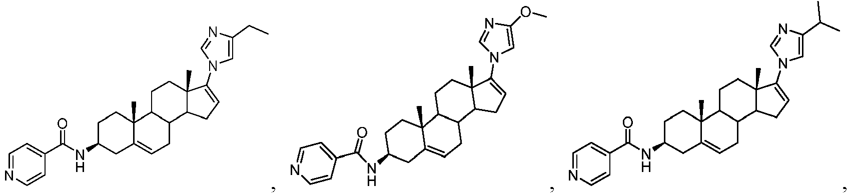

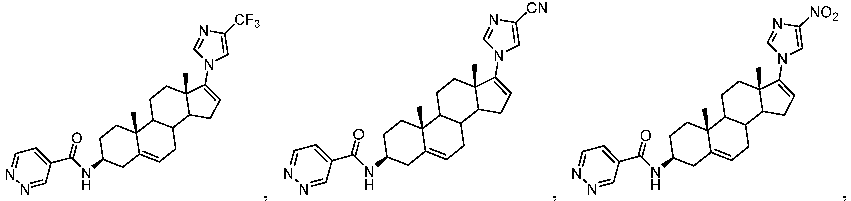

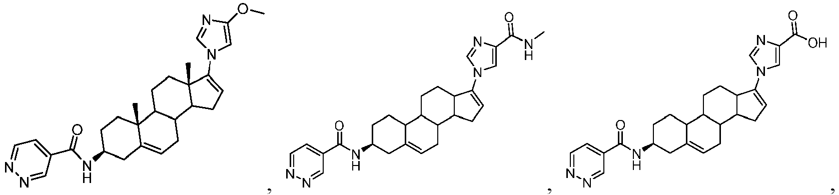

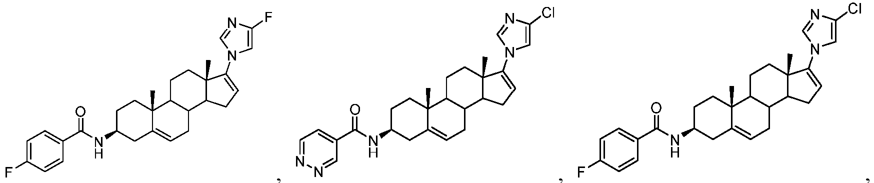

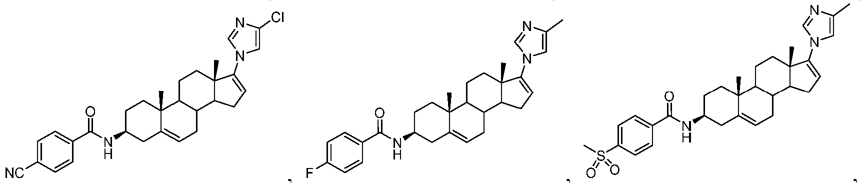

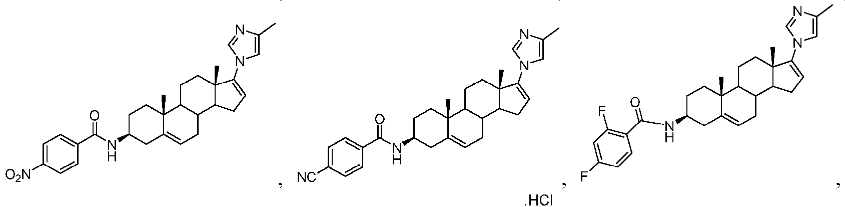

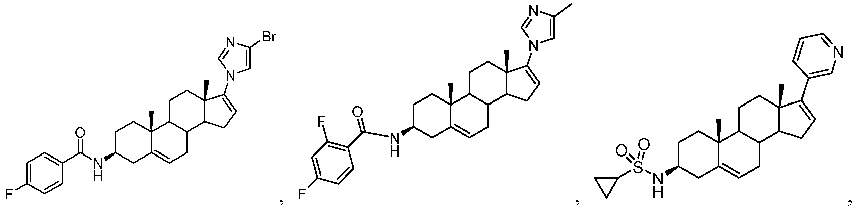

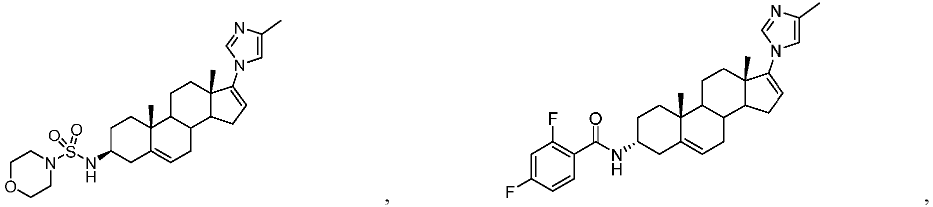

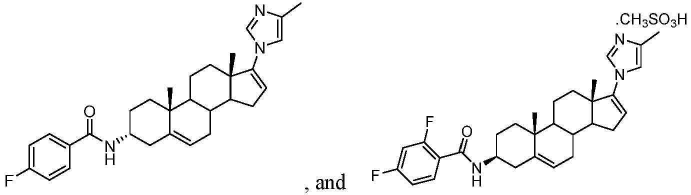

6. A preparation method of the compound of claim 1, comprising the following steps: 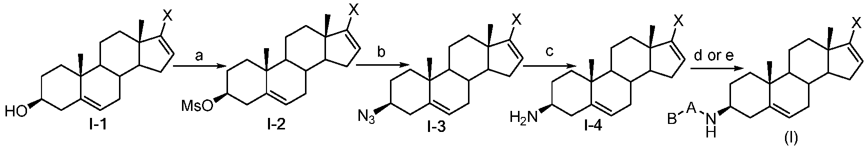
a) reacting intermediate I-1 with methanesulfonyl chloride to obtain intermediate I-2;
b) reacting intermediate I-2 with azidotrimethyl silane and boron trifluoride etherate to obtain intermediate I-3;
c) reacting intermediate I-3 with lithium aluminum hydride to obtain intermediate I-4;
d) reacting intermediate I-4 with an acyl chloride to obtain target compound I; or
e) reacting intermediate I-4 with a carboxylic acid to obtain target compound I via a condensation reaction,
wherein X, A and B are as defined above.

7. A preparation method of the compound of claim 1, wherein intermediate I-1 is obtained by a Suzuki coupling reaction of compound II-1 with a boronic acid or boronic ester, 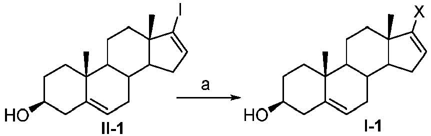 or the preparation method of intermediate I-1 comprises the following steps: 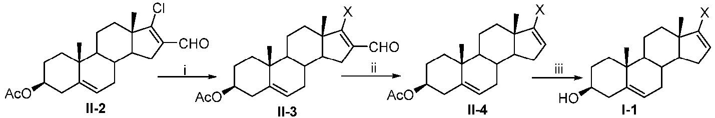
i) subjecting compound II-2 to an addition-elimination reaction to obtain compound II-3;
ii) removing the aldehyde group from compound II-3 to obtain compound II-4;
iii) hydrolyzing compound II-4 to obtain intermediate I-1,
wherein X is as defined above.

8. A pharmaceutical composition, comprising:
the compound of formula (I) of claim 1, or the enantiomer, diastereoisomer, stereoisomer, prodrug, deuterated compound, hydrate, solvate, racemate or pharmaceutically acceptable salt thereof; and
a pharmaceutically acceptable carrier.

9. Use of the compound of formula (I) of claim 1, or the enantiomer, diastereoisomer, stereoisomer, prodrug, deuterated compound, hydrate, solvate, racemate or pharmaceutically acceptable salt thereof or the pharmaceutical composition of claim 8, for preparing a medicament for preventing and/or treating a disease related to the AR signaling pathway.

10. The use of claim 9, wherein the disease related to the AR signaling pathway is selected from the group consisting of prostate cancer, castration-resistant prostate cancer, breast cancer, SARS-CoV-2 infectious disease, osteoporosis, and digestive system disease.
